# EUROPEAN PATENT APPLICATION

(11) **EP 2 034 014 A1**
(43) Date of publication of application: **11.03.2009**
(21) Application number: 07744468.5
(22) Date of filing: 31.05.2007
(51) Int. Cl.: C12N 15/00, A61K 35/76, A61K 48/00, A61P 25/28, C12N 15/09

(54) **THERAPEUTIC AGENT FOR ALZHEIMER'S DISEASE**

(30) Priority: 31.05.2006 JP 2006151617; 19.04.2007 JP 2007110623
(71) Applicant: Dnavec Corporation, Tsukuba-shi, Ibaraki 305-0856 (JP)
(72) Inventor: INOUE, Makoto, Tsukuba-shi Ibaraki 3050856 (JP); TOKUSUMI, Yumiko, Tsukuba-shi Ibaraki 3050856 (JP); IWASAKI, Hitoshi, Tsukuba-shi Ibaraki 3050856 (JP); HARA, Hiroto, Tsukuba-shi Ibaraki 3050856 (JP); TABATA, Toshiaki, Tsukuba-shi Ibaraki 3050856 (JP); HASEGAWA, Mamoru, Tsukuba-shi Ibaraki 3050856 (JP)
(74) Representative: Vossius & Partner
(86) International application number: PCT/JP2007/061058
(87) International publication number: WO 2007/139178

(57) **Abstract**

The present invention provides novel therapeutic methods and agents for treating Alzheimer's disease. Specifically, the present invention relates to anti-inflammatory cytokines, anti-inflammatory cytokine genes, negative-strand RNA viral vectors carrying an anti-inflammatory cytokine gene, which are used for treating Alzheimer's disease or developing therapeutic agents for Alzheimer's disease. The present invention also provides pharmaceutical compositions for treating or preventing Alzheimer's disease, which comprise the cytokines or vectors. The present invention further provides methods for treating Alzheimer's disease, which comprise the step of administering an anti-inflammatory cytokine, or a vector such as a negative-strand RNA viral vector carrying an anti-inflammatory cytokine gene. The present invention enables novel gene therapies for Alzheimer's disease.

## Description

### Technical Field

The present invention relates to the treatment ofAlzheimer's disease. Specifically, the present invention relates to the treatment of Alzheimer's disease using anti-inflammatory cytokines or vectors expressing anti-inflammatory cytokines, such as negative-strand RNA viral vectors carrying an anti-inflammatory cytokine gene.

### Background Art

It has been reported that about 10% of the people aged 65 years or older suffer from senile dementia in Japan's rapidly aging society. Alzheimer's disease is one of the two major causes of dementia, and accounts for about 50% of the dementia. Alzheimer's disease is becoming a serious social problem including the problem of nursing care.

Agents currently used for the therapy of Alzheimer's disease include acetylcholine modulators such as activators of the acetylcholine system, and acetylcholine esterase inhibitors; β-amyloid modulators such as β-secretase (BACE) inhibitors, which inhibit generation of amyloid peptides, and inhibitors of amyloid peptide aggregation; neuroprotection and neurotrophic therapeutic agents such as neuropeptides and nerve growth factors; chelators; antioxidants; and anti-inflammatory agents.

In terms of therapeutic effect, therapeutic agents for Alzheimer's disease can be categorized into the following three types. First-generation drugs can hardly suppress the progression of dementia itself, although they can improve the intellectual function to some extent when used at earlier stages of Alzheimer's disease; second-generation drugs have the effect of improving intellectual function, and more than that, they are expected to have an effect on suppression of the progression of Alzheimer's disease; and third-generation drugs are radical preventive/therapeutic drugs.

Most of the drugs currently under evaluation are thought to be first-generation drugs. The "amyloid cascade hypothesis", which ascribes senile plaque formation via aggregation and deposition of amyloid peptides as the cause of the disease, is widely accepted as the mechanism of onset and progression in Alzheimer's disease. Some of the drugs that target amyloid peptides are expected to be further developed into second- or third-generation drugs. Thus, currently, there are strong demands for second- and third-generation drugs as radical therapeutic drugs for Alzheimer's disease, as well as truly effective first-generation drugs.

On the other hand, the "inflammation hypothesis", which indicates that enhanced activity of inflammatory microglia induces neuronal cell death in the brain with Alzheimer's disease, has been proposed as the mechanism of onset in Alzheimer's disease. In fact, the microglial activity is enhanced and microglia are accumulated particularly around senile plaques in the brains of patients with Alzheimer's disease. It has also been demonstrated that the lymphocytes infiltrate the brains of patients with Alzheimer's disease, and that substances which are activated upon inflammation, such as complements, are accumulated in the brains. From the analytical results of epidemiological investigation, it was expected that anti-inflammatory drugs, in particular, non-steroidal anti-inflammatory drugs (NSAIDs) suppress the progression of Alzheimer's disease. Furthermore, indomethacin was reported to significantly suppress the progression of Alzheimer's disease in pilot clinical trials (Rogers J et al., Neurology. 1993 Aug; 43(8):1609-11). Thus, large-scale clinical trials were conducted mainly for Cox-2-specfic inhibitors which are less likely to cause gastrointestinal disorders. However, it has been reported in a one-year study of patients with mild to moderate Alzheimer's disease, that first-generation NSAIDs and new-generation NSAIDs could not be demonstrated to have effect of suppressing the progression in Alzheimer's disease (Aisen PS et al., JAMA. 2003 Jun 4; 289(21):2819-26; Imbimbo BP. Expert Opin Investig Drugs. 2004 Nov; 13(11):1469-81; Townsend KP et al., FASEB J. 2005 Oct;19(12):1592-601). It has also been reported that oral administration of a compound called MW01-5-188WH, which is a selective inhibitor of inflammation-induced cytokine production in glial cells, to mice suppresses the amyloid β1-42-induced up-regulation of interleukin-1β, tumor necrosis factor-α, and S 100B in the hippocampus, recovers synaptic failures in the hippocampus, and improves hippocampus-dependent Y-maze behavior (Ralay Ranaivo H et al., J Neurosci. 2006 Jan 11; 26(2):662-70).
[Non-Patent Document 1]
   Rogers J et al., Neurology. 1993 Aug; 43(8):1609-11
[Non-Patent Document 2]
   Aisen PS et al., JAMA. 2003 Jun 4; 289(21):2819-26
[Non-Patent Document 3]
   Imbimbo BP. Expert Opin Investig Drugs. 2004 Nov; 13(11):1469-81
[Non-Patent Document 4]
   Townsend KP et al., FASEB J. 2005 Oct; 19(12):1592-601
[Non-Patent Document 5]
   Ralay Ranaivo H et al., J Neurosci. 2006 Jan 11; 26(2):662-70

### Disclosure of the Invention

### [Problems to be Solved by the Invention]

The present invention was achieved in view of the circumstances described above. An objective of the present invention is to provide novel treatment methods and pharmaceutical agents for the therapy of Alzheimer's disease.

### [Means for Solving the Problems]

To achieve the above-described objective, the present inventors conducted dedicated studies to develop novel methods that are effective for treating Alzheimer's disease. Interleukin-10 (IL-10), which is one of the anti-inflammatory cytokines, regulates the inflammatory response by acting competitively against the activity of pro-inflammatory cytokines. It has been pointed out that in Alzheimer's disease, polymorphisms present in the promoter region of IL-10 are associated with the progression of the disease (Lio D et al., Genes Immun. 2003 Apr; 4(3):234-8.; Scassellati C et al., Neurosci Lett. 2004 Feb 12; 356(2):119-22.; Arosio B et al., Neurobiol Aging. 2004 Sep; 25(8):1009-15.; Ma SL et al., Neurobiol Aging. 2005 Jul; 26(7):1005-10). However, there are no cases that examined such anti-inflammatory cytokines for the purpose of treating Alzheimer's disease. The present invention provides novel methods for treating Alzheimer's disease using anti-inflammatory cytokines or vectors expressing anti-inflammatory cytokine genes, such as negative-strand RNA viral vectors. The present invention provides novel gene therapy methods and such for treating and preventing Alzheimer's disease.

Specifically, the present invention relates to negative-strand RNA viral vectors carrying an anti-inflammatory cytokine gene for treating Alzheimer's disease or developing therapeutic agents for Alzheimer's disease; pharmaceutical compositions comprising the negative-strand RNA viral vectors for Alzheimer's disease; and methods for treating and preventing Alzheimer's disease using the negative-strand RNA viral vectors. More specifically, the present invention includes the following:
[1] pharmaceutical composition for treating or preventing Alzheimer's disease, wherein the composition comprises
   a negative-strand RNA viral vector carrying a gene encoding an anti-inflammatory cytokine or a partial peptide thereof, or
   an anti-inflammatory cytokine or a partial peptide thereof;
[2] the composition of [1], wherein the composition comprises a negative-strand RNA viral vector carrying a gene encoding an anti-inflammatory cytokine or a partial peptide thereof;
[3] the composition of [1], wherein the composition comprises an anti-inflammatory cytokine or a partial peptide thereof;
[4] the composition of [1] or [2], wherein the negative-strand RNA viral vector is a paramyxovirus vector;
[5] the composition of [1] or [2], wherein the negative-strand RNA viral vector is a Sendai virus vector;
[6] the composition of any one of [1] to [5], wherein the anti-inflammatory cytokine is selected from the group consisting of interleukin-4, interleukin-10, interleukin-13, and partial peptides thereof;
[7] the composition of any one of [1] to [6], wherein the composition is used for nasal administration;
[8] a negative-strand RNA viral vector carrying a gene for an anti-inflammatory cytokine or a partial peptide thereof, wherein the vector is used for treating Alzheimer's disease or developing a therapeutic agent for Alzheimer's disease;
[9] an anti-inflammatory cytokine protein, wherein the protein is used for treating Alzheimer's disease or developing a therapeutic agent for Alzheimer's disease;
[10] the vector of [8], wherein the negative-strand RNA viral vector is a paramyxovirus vector;
[11] the vector of [8], wherein the negative-strand RNA viral vector is a Sendai virus vector;
[12] the vector of any one of [8], [10], and [11], wherein the anti-inflammatory cytokine is selected from the group consisting of interleukin-4, interleukin-10, interleukin-13, and partial peptides thereof;
[13] a method for treating or preventing Alzheimer's disease, wherein the method comprises the step of administering
   a negative-strand RNA viral vector carrying a gene encoding an anti-inflammatory cytokine or a partial peptide thereof, or
   an anti-inflammatory cytokine or a partial peptide thereof;
[14] the method of [13], wherein the administration is nasal administration.

The present invention also includes the following:
(1) a negative-strand RNA viral vector carrying a gene for an anti-inflammatory cytokine or a partial peptide thereof, wherein the vector is used for treating Alzheimer's disease or developing a therapeutic agent for Alzheimer's disease;
(2) the vector of (1), wherein the negative-strand RNA viral vector is a paramyxovirus vector;
(3) the vector of (1), wherein the negative-strand RNA viral vector is a Sendai virus vector;
(4) the vector of any one of (1) to (3), wherein the anti-inflammatory cytokine is selected from the group consisting of interleukin-4, interleukin-10, interleukin-13, and partial peptides thereof;
(5) a pharmaceutical composition for treating or preventing Alzheimer's disease, which comprises the vector of any one of (1) to (4); and
(6) the pharmaceutical composition of (5), which is used for nasal administration.

The present invention also relates to methods for treating or preventing Alzheimer's disease, which comprise the step of administering an anti-inflammatory cytokine or a vector encoding it. In particular, the present invention relates to methods for treating or preventing Alzheimer's disease, which comprise the step of administering a vector capable of expressing an anti-inflammatory cytokine, such as a negative-strand RNA viral vector carrying an anti-inflammatory cytokine gene. The negative-strand RNA viral vector is preferably a paramyxovirus vector, more preferably a Sendai virus vector. The anti-inflammatory cytokine is preferably IL-10. The administration is preferably nasal administration.

The present invention also relates to the use of an anti-inflammatory cytokine or a vector encoding it in the production of pharmaceutical agents for treating or preventing Alzheimer's disease. In particular, the present invention provides the use of an anti-inflammatory cytokine, and a vector carrying an anti-inflammatory cytokine gene, specifically, a negative-strand RNA viral vector carrying an anti-inflammatory cytokine gene in the production of pharmaceutical agents for treating or preventing Alzheimer's disease. The negative-strand RNA viral vector is preferably a paramyxovirus vector, more preferably a Sendai virus vector. The anti-inflammatory cytokine is preferably IL-10. The pharmaceutical agents comprising a negative-strand RNA viral vector are formulated into dosage forms suitable for nasal administration.

### [Effects of the Invention]

The present invention provides novel therapeutic agents and methods for Alzheimer's disease using anti-inflammatory cytokines. In particular, the present invention provides therapeutic agents for Alzheimer's disease, which comprise negative-strand RNA viral vectors encoding an anti-inflammatory cytokine, and gene therapy methods for Alzheimer's disease using the vectors. The methods of the present invention can be novel therapeutic means that can be employed to substitute for or in combination with other therapeutic methods for Alzheimer's disease.

### Brief Description of the Drawings

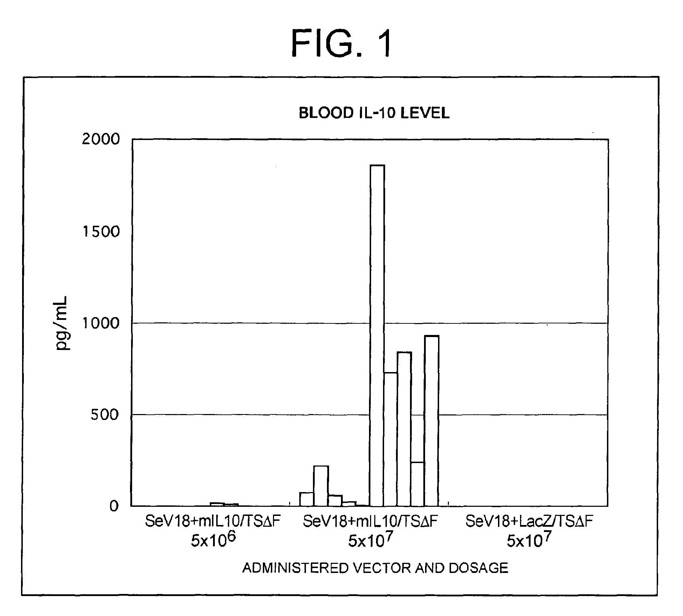

Fig. 1 depicts the result of measurement of the blood IL-10 level after administration of an SeV vector expressing IL-10. An SeV vector expressing LacZ was used as a control. Blood IL-10 was detected in a manner specific to the IL-10-expressing SeV vector and dependent on the dosage.

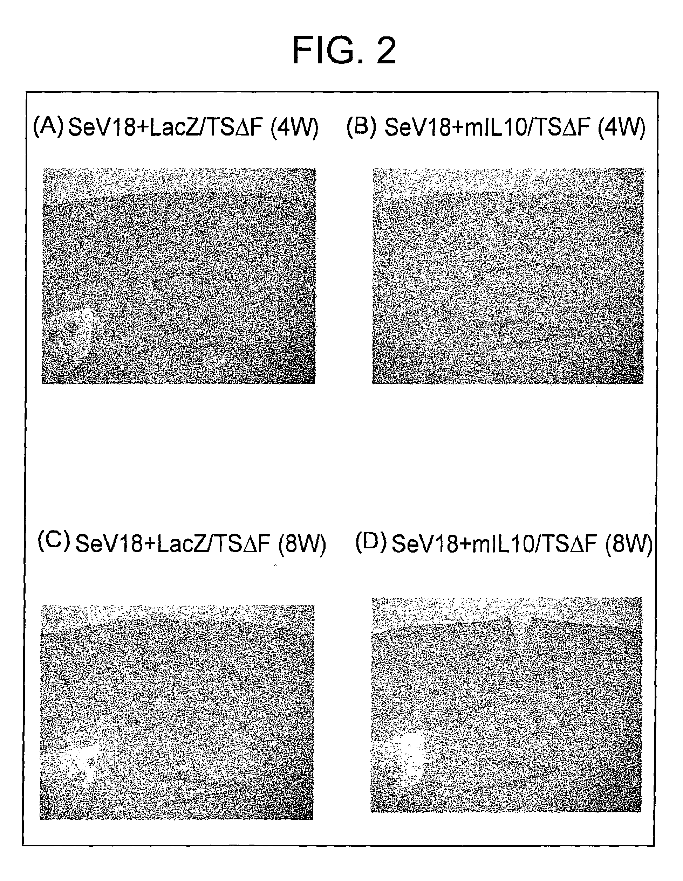

Fig. 2 depicts senile plaques in the parietal lobe of cerebral neocortex and the hippocampus (anti-Aβ antibody staining). The number of senile plaques (reddish brown spots) in the cerebral neocortex is evidently smaller in the SeV18+mIL10/TSΔF group (right) than that in the SeV18+LacZ/TSΔF group (left), both four weeks (upper panels) and eight weeks (lower panels) after the vector administration.

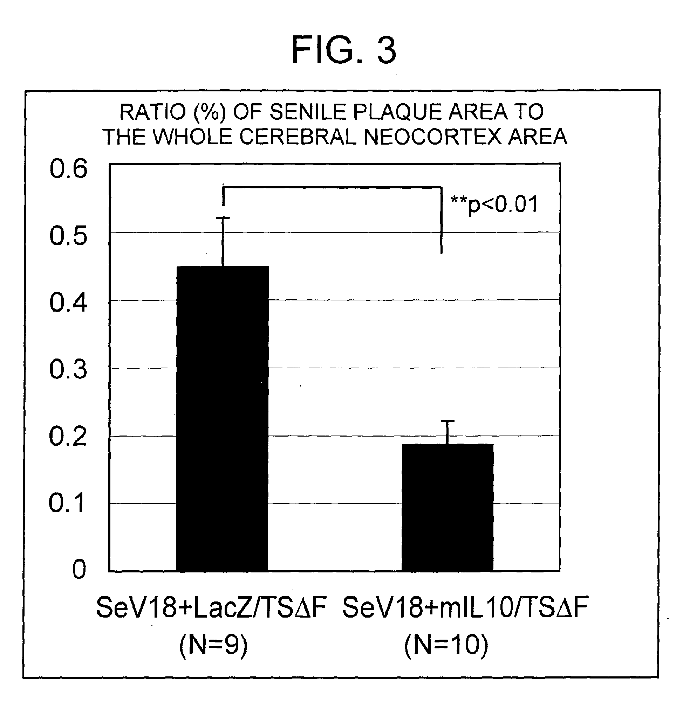

Fig. 3 depicts the ratio (%) of the senile plaque area to the entire cerebral neocortex area (mean ± SE). The ratio (%) of the area of senile plaques to that of the entire cerebral neocortex eight weeks after the administration was determined using an image analysis software. The ratio was significantly lower in the SeV18+mIL10/TSΔF group than in the control group (the SeV18+LacZ/TSΔF group) (p<0.01, Student t test).

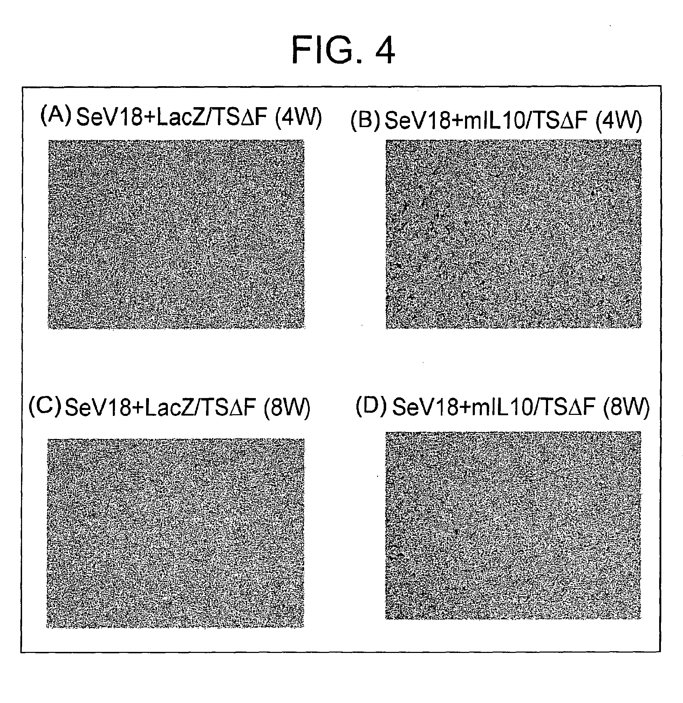

Fig. 4 depicts the activation of microglia in the olfactory bulb (Iba-1 staining). The number of activated microglia (reddish brown amoeboid cells) in the olfactory bulb is evidently increased in the SeV 18+mIL10/TSΔF group (right) as compared to the SeV18+LacZ/TSΔF group (left), both four weeks (upper panels) and eight weeks (lower panels) after the administration.

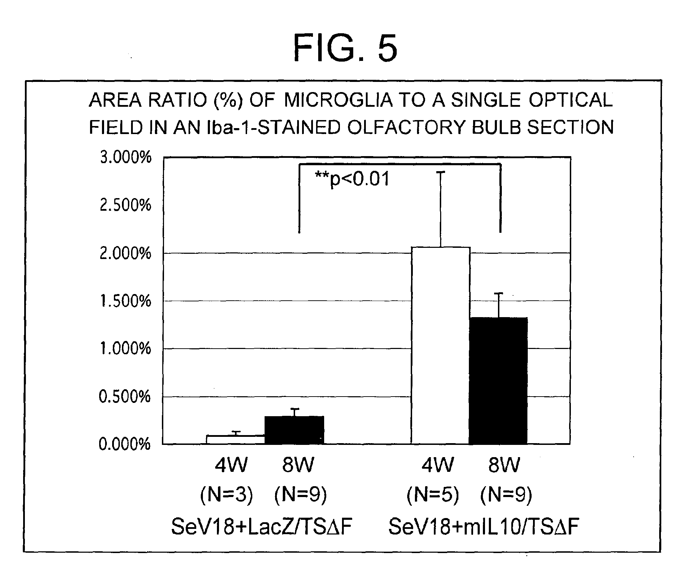

Fig. 5 depicts the ratio (%) of the area of microglia to that of a single optical field in an olfactory bulb section stained with Iba-1 (mean ± SE). The ratio (%) of the area of Iba-1-positive microglia to that of the single optical field in the olfactory bulb eight weeks after the administration was determined using an image analysis software. The ratio was significantly higher in the SeV 18+mIL10/TSΔF group than in the control group (the SeV18+LacZ/TSΔF group) (p<0.01, Student t test).

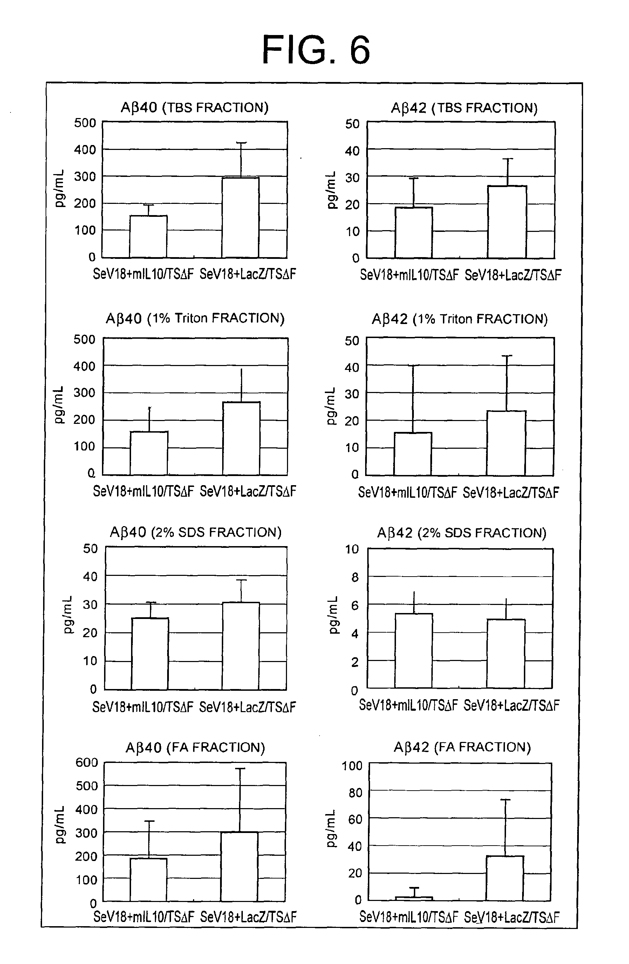

Fig. 6 depicts the result of measurement of the Aβ level in the brain tissue after administration of an IL-10-expressingSeV vector. An SeV vector expressing LacZ was used as a control. In the group to which the SeV vector expressing IL-10 was administered, Aβ was shown to be decreased in most of the fractions. In particular, soluble Aβ40 (in TBS fraction and 1 % Triton fraction) and insoluble Aβ42 (in formic acid fraction) were shown to be significantly decreased.

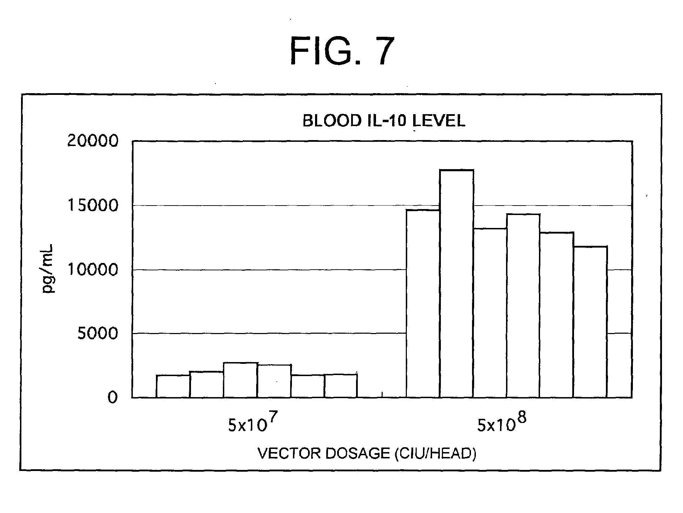

Fig. 7 depicts the result of measurement of the blood IL-10 levels in normal mice after administration of an SeV vector expressing IL-10. Blood IL-10 was detected in a vector dosage-dependent manner.

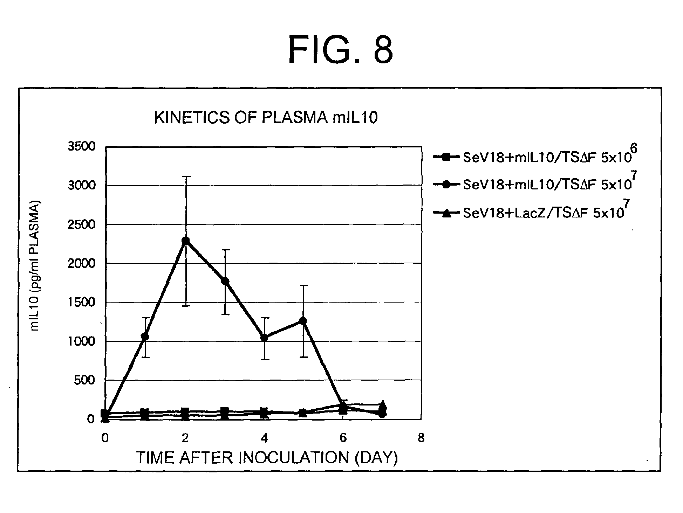

Fig. 8 depicts the kinetics of blood IL-10 level after administration of an SeV vector expressing IL-10. A single dose of nasal drop of SeV18+mIL10/TSΔF (5 x 10⁷ CIU/head) resulted in an AUC of 176,000 pg·h/ml.

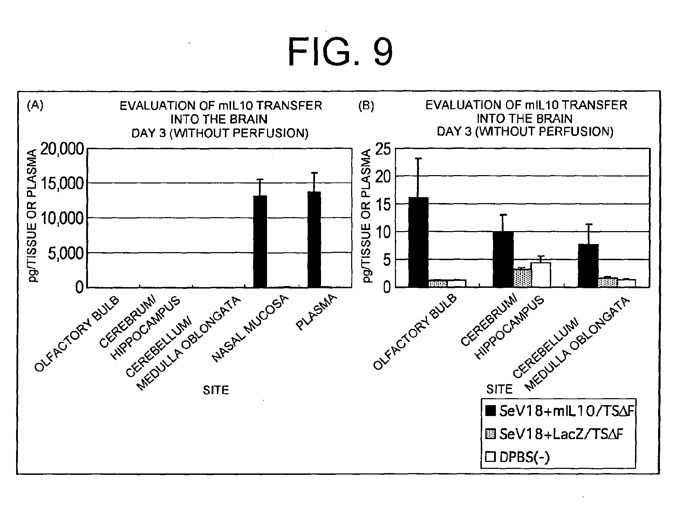

Fig. 9 depicts IL-10 transfer into the brain after nasal administration of an SeV vector expressing IL-10. SeV18+mIL10/TSΔF or SeV18+LacZ/TSΔF was nasally administered to normal C57BL/6N mice (N=5) at 5 x 10⁸ CIU/head/53 µl. The same volume of DPBS(-) was administered as a control. Brain (divided into the following three parts: the olfactory bulb, cerebrum/hippocampus, and cerebellum/medulla oblongata), nasal mucosa, trachea/lung, and plasma were collected three days after the administration. mIL-10 in each tissue was quantified by ELISA. The expression levels of mIL-10 in olfactory bulb, cerebrum/hippocampus, and cerebellum/medulla oblongata shown in panel (A) are also shown in panel (B) which has a magnified scale of vertical axis.

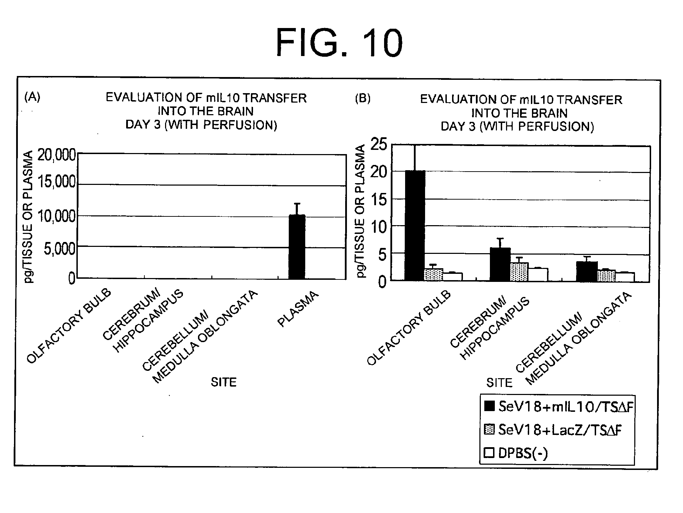

Fig. 10 depicts IL-10 transfer into the brain after nasal administration of an SeV vector expressing IL-10. SeV18+mIL10/TSΔF or SeV18+LacZ/TSΔF was nasally administered to normal C57BL/6N mice (N=5) at 5 x 10⁸ CIU/head/53 µl. The same volume of DPBS(-) was administered as a control. Perfusion was performed, and the brains were collected three days after the administration. mIL-10 in the brain (divided into the following three parts: the olfactory bulb, cerebrum/hippocampus, and cerebellum/medulla oblongata), nasal mucosa, trachea/lung, and plasma was quantified by ELISA. The expression levels of mIL-10 in olfactory bulb, cerebrum/hippocampus, and cerebellum/medulla oblongata shown in panel (A) are also shown in panel (B) which has a magnified scale of vertical axis.

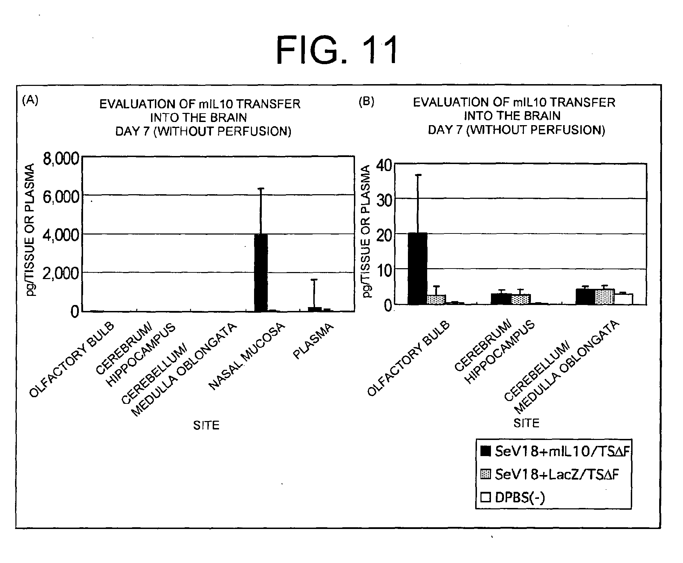

Fig. 11 depicts IL-10 transfer into the brain after nasal administration of an SeV vector expressing IL-10. SeV18+mIL10/TSΔF or SeV18+LacZ/TSΔF was nasally administered to normal C57BL/6N mice (N=5) at 5 x 10⁸ CIU/head/53 µl. The same volume of DPBS(-) was administered as a control. Brain (divided into the following three parts: the olfactory bulb, cerebrum/hippocampus, and cerebellum/medulla oblongata), nasal mucosa, trachea/lung, and plasma were collected after seven days from the administration. mIL-10 in each tissue was quantified by ELISA. The expression levels of mIL-10 in olfactory bulb, cerebnun/hippocampus, and cerebellum/medulla oblongata shown in panel (A) are also shown in panel (B) which has a magnified scale of vertical axis.

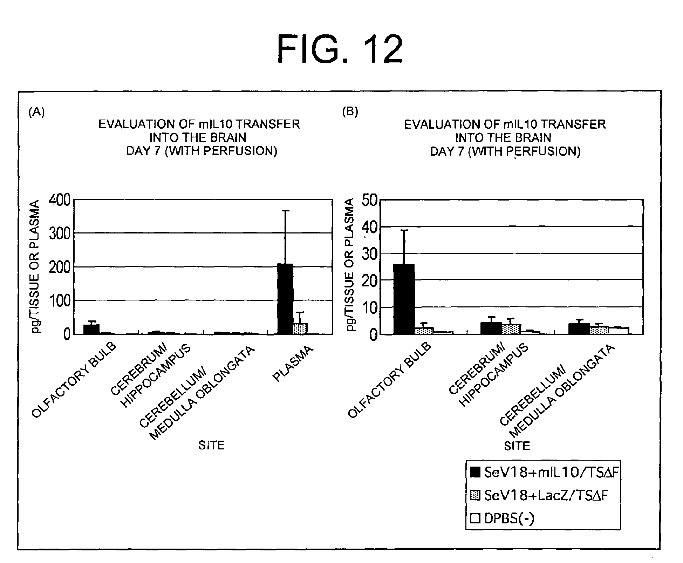

Fig. 12 depicts IL-10 transfer into the brain after nasal administration of an SeV vector expressing IL-10. SeV18+mIL10/TSΔF or SeV18+LacZ/TSΔF was nasally administered to normal C57BL/6N mice (N=5) at 5 x 10⁸ CIU/head/53 µl. The same volume of DPBS(-) was administered as a control. Perfusion was performed, and the brains were collected seven days after the administration. mIL-10 in the brain (divided into the following three parts: the olfactory bulb, cerebrum/hippocampus, and cerebellum/medulla oblongata), nasal mucosa, trachea/lung, and plasma was quantified by ELISA. The expression levels of mIL-10 in olfactory bulb, cerebrum/hippocampus, and cerebellum/medulla oblongata shown in panel (A) are also shown in panel (B) which has a magnified scale of vertical axis.

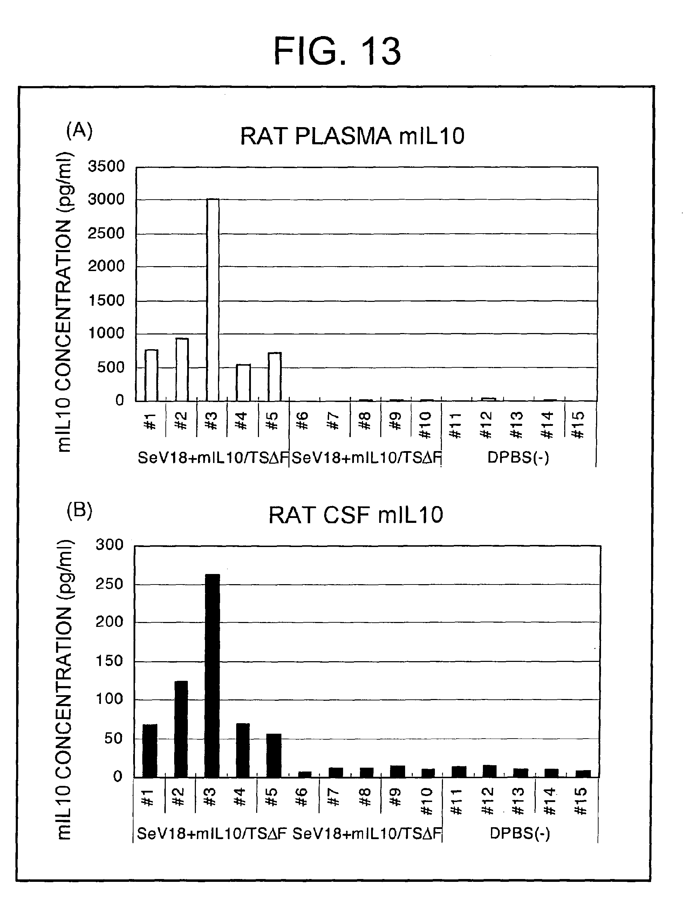

Fig. 13 depicts IL-10 transfer into the brain (concentration in CSF) after nasal administration of an SeV vector expressing IL-10. SeV18+mmIL10/TSΔF (rats #1-#5) or SeV18+LacZ/TSΔF (rats #6-#10) was nasally administered to normal Wistar rats (N=5) at 1 x 10⁹ CILJ/head/106 µl. The same volume of DPBS(-) was administered as a control (rats #11-#15). The plasma and cerebrospinal fluid were collected three days after the administration, and mIL-10 was quantified by ELISA.

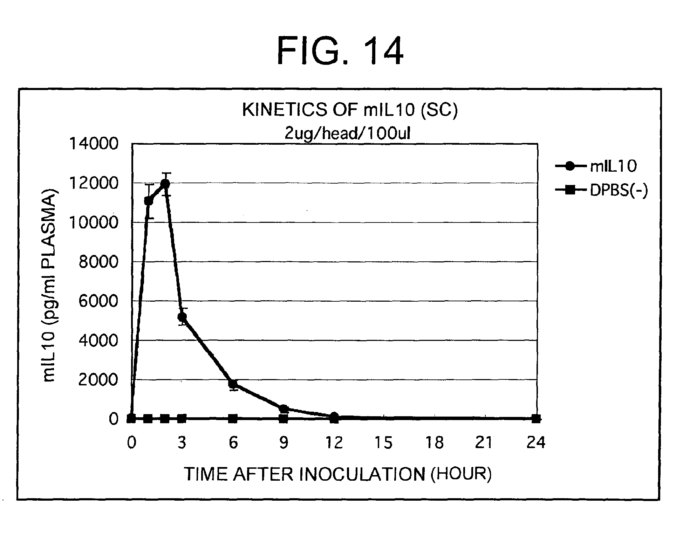

Fig. 14 depicts the kinetics of blood IL-10 level in normal mice (C57BL/6N) after subcutaneous administration of the IL-10 protein. IL-10 from recombinant mice (2.0 µg/100 µl/head) was subcutaneously administered in the back, and blood mIL-10 was quantified by ELISA. The result is as follows: AUC = 40,800 pg·h/ml; Cₘₐₓ = about 12,000 pg/ml; Tₘₐₓ = about 1.5 hr; and t_{1/2} = about 1 hr (initial value).

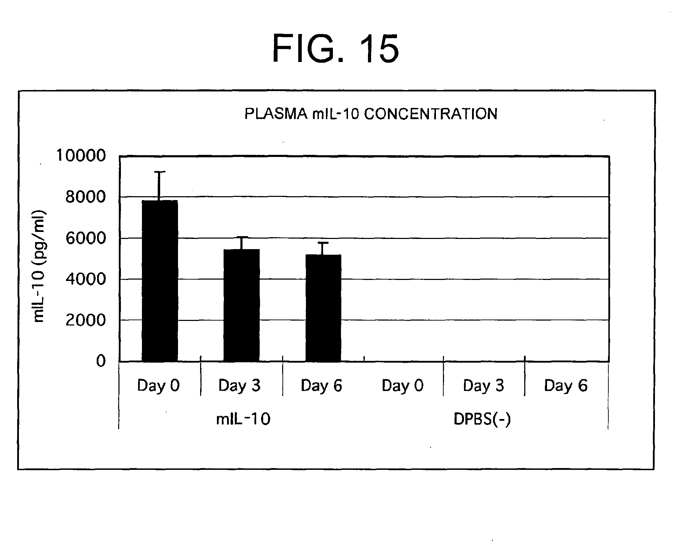

   Fig. 15 depicts the IL-10 levels in APP mice after subcutaneous administration of the IL-10 protein.

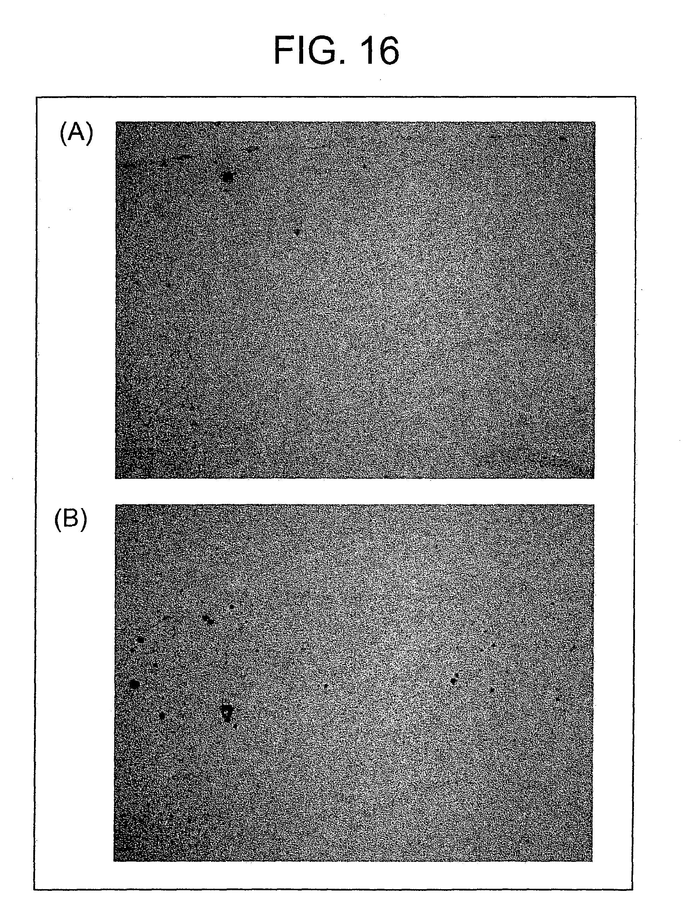

Fig. 16 shows photographs depicting the effects of continuous subcutaneous administration of IL-10 to APP model mice (Tg2576). Results of anti-Aβ antibody (4G8) immunostaining of sections of the parietal lobe of cerebral neocortex and the hippocampus are shown. The upper panel shows the result for the IL-10 administration group, in which a small number of senile plaques (brown spots) were observed in the cerebral neocortex. The lower panel shows the result for the DPBS(-) administration group, in which many senile plaques were observed in the cerebral neocortex.

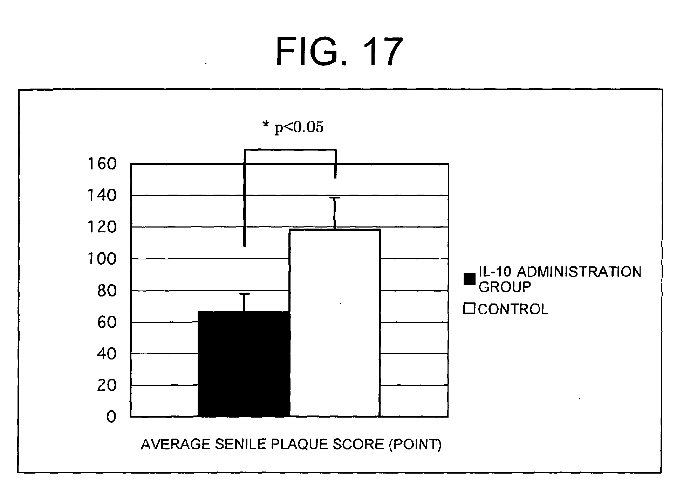

Fig. 17 depicts the result of semi-quantitative measurement of senile plaques in APP model mice (Tg2576) after continuous subcutaneous administration of IL-10. The scores for senile plaques were assigned according to the size as follows: large: nine points; middle: three points; small: one point. The total scores of senile plaques observed in the whole brain (excluding brain stem and cerebellum) were calculated. Statistical analysis between the groups was performed (Student's t test; mean ± standard deviation).

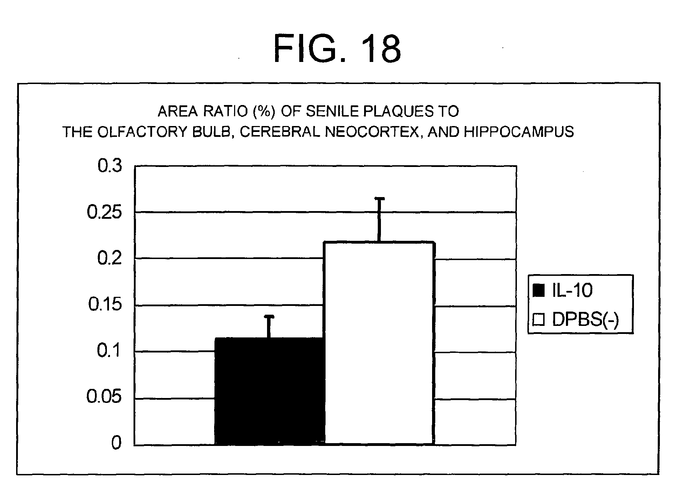

Fig. 18 depicts the result of quantification of the area of senile plaques in the olfactory bulb, cerebral neocortex, and hippocampus ofAPP model mice (Tg2576) after continuous subcutaneous administration of IL-10 (Student t test). The "IL-10" and "DPBS(-)" bars indicate the results for the IL-10 and DPBS(-) administration groups, respectively.

### Best Mode for Carrying Out the Invention

The present invention relates to therapeutic agents and preventive agents for Alzheimer's disease, which comprise anti-inflammatory cytokines or vectors expressing anti-inflammatory cytokines. Such vectors include plasmids, naked DNAs, lyposome compositions, and viral vectors. In particular, the present invention relates to negative-strand RNA viral vectors carrying an anti-inflammatory cytokine gene for treating Alzheimer's disease or developing therapeutic agents for Alzheimer's disease, and pharmaceutical compositions that comprise the vectors for treating or preventing Alzheimer's disease. "Negative-strand RNA virus" (also referred to as "minus-strand RNA virus") refers to a virus comprising a negative-strand (an antisense strand complementary to a sense strand encoding viral proteins) RNA as the genome. "Minus-strand RNA virus" is also referred to as "negative-strand RNA virus". In particular, negative-strand RNA viruses that are preferably used in the present invention are negative single-stranded RNA viruses (also referred to as non-segmented negative-strand RNA viruses). "Negative single-stranded RNA virus" refers to a virus comprising a negative single-stranded RNA, *i*.*e*., a minus-strand RNA, as the genome. Such viruses include viruses belonging to *Paramyxoviridae* (including the genera Paramyxovirus, Morbillivirus, Rubulavirus, and Pneumovirus, etc.), *Rhabdoviridae* (including the genera Vesiculovirus, Lyssavirus, and Ephemerovirus, etc.), *Filoviridae*, and such. The negative-strand RNA viral vectors used in the present invention may be transmissible vectors or non-transmissible defective vectors. "Transmissible" means that, when a host cell is infected with a viral vector, the virus is replicated in the cell to produce infectious viral particles.

Specific examples of particularly preferred negative-strand RNA viruses suitable for use in the context of the present invention include, for example, Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3 belonging to Paramyxoviridae; influenza virus belonging to Orthomyxoviridae; and vesicular stomatitis virus and rabies virus belonging to Rhabdoviridae.

More preferably, paramyxoviruses are used in the present invention. "Paramyxoviruses" refers to viruses belonging to Paramyxoviridae, or derivatives of the viruses. Preferred paramyxoviruses include viruses belonging to Paramyxovirinae (including Respirovirus, Rubulavirus, and Morbillivirus), more preferably those belonging to the genus Respirovirus (also referred to as the genus Paramyxovirus) or derivatives thereof. The derivatives include viruses that are genetically-modified or chemically-modified in a manner not to impair their gene-transferring ability. Examples of viruses of the genus Respirovirus applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). In the context of the present invention, a more preferred paramyxovirus is the Sendai virus. These viruses may be derived from natural strains, wild strains, mutant strains, laboratory-passaged strains, artificially constructed strains, or the like.

Herein, "vector" refers to a carrier for introducing nucleic acids into cells. Negative-strand RNA viruses such as Sendai virus are excellent gene transfer vectors. In their life cycle, the vectors are transcribed and replicated only in the host cytoplasm. Since the vectors do not have any DNA phase, chromosomal integration does not occur. Therefore, safety issues such as oncogenic transformation and immortalization due to chromosomal aberration do not occur. This characteristic of negative-strand RNA viruses greatly contributes to safety when they are used as vectors. Results of foreign gene expression show that few nucleotide mutations are observed even after multiple continuous passages of Sendai virus. This indicates that the viral genome is highly stable and inserted foreign genes are stably expressed over a long period of time (Yu, D. et al., Genes Cells 2, 457-466 (1997)). Furthermore, the virus has qualitative advantages such as flexibility in the size and packaging of inserted genes due to the absence of a capsid structural protein.

The negative-strand RNA viral vector of the present invention may be, for example, a complex comprising the genomic RNA of a negative-strand RNA virus and viral proteins, namely, a ribonucleoprotein (RNP). Specifically, such an RNP is a complex comprising the genomic RNA of a negative-strand RNA virus, the N protein, P protein, and L protein. When RNPs are introduced into cells, cistrons encoding viral proteins are transcribed from the genomic RNA through the action of viral proteins, and the genome itself is replicated to form daughter RNPs. Thus, sustained expression of RNPs is expected. RNPs can be introduced into cells, for example, by combining the RNPs with a desirable transfection reagent. Replication of the genomic RNA can be confirmed by detecting the increase in the copy number of the RNA using RT-PCR, Northern blot hybridization, or such.

Alternatively, the negative-strand RNA viral vector of the present invention is preferably a negative-strand RNA viral particle. "Viral particle" refers to a microparticle comprising a nucleic acid, which is released from cells through the action of viral proteins. A negative-strand RNA viral particle has a structure in which the above-described RNP comprising the genomic RNA and viral proteins is enclosed in a lipid membrane (referred to as "envelope") derived from the cell membrane. The viral particles may show infectivity. "Infectivity" refers to the ability of a negative-strand RNA viral vector, which has cell-adhesion and membrane-fusion abilities, to introduce a nucleic acid inside the vector into cells to which the vector has adhered. The negative-strand RNA viral vectors of the present invention may be transmissible vectors or defective non-transmissible vectors. "Transmissible" means that, when a host cell is infected with a viral vector, the virus is replicated in the cell to produce infectious viral particles.

The genomic RNA of a negative-strand RNA virus encodes a carried gene in the antisense direction. In general, the genome of a negative-strand RNA virus is constituted so that the viral genes are arranged in the antisense orientation between the 3' leader region and the 5' trailer region. "Transcription ending sequence (E sequence) - intervening sequence (I sequence) - transcription starting sequence (S sequence)" exists between the ORFs of individual genes, which allows the RNAs encoding the ORFs of the genes to be transcribed as separate cistrons. The genomic RNA comprised in the vector of the present invention encodes the N (nucleocapsid (also referred to as nucleoprotein (NP)), P (phospho), and L (large) proteins in the antisense direction, which are viral proteins necessary for expression of the group of genes encoded by the RNA, and for autonomous replication of the RNA itself. The RNA may encode the M (matrix) protein, which is necessary for formation of viral particles. The RNA may also encode envelope proteins, which are necessary for infection of viral particles. The envelope proteins of negative-strand RNA virus include the F (fusion) protein, which causes cell membrane fusion, and the HN (hemagglutinin-neuraminidase) (or H (hemagglutinin)) protein, which is necessary for adhesion to cells. However, for certain cell types, the HN protein is not required for infection (Markwell, M.A. et al., Proc. Natl. Acad. Sci. USA 82(4):978-982 (1985)), and infection is achieved by just the F protein. The RNA may encode viral envelope proteins other than the F protein and/or HN protein.

For example, respective genes of each virus belonging to *Paramyxovirinae* are commonly represented as follows. In general, the NP gene is also represented as "N". Furthermore, when "HN" has no neuraminidase activity, it is represented as "H (hemagglutinin)".
The genus Respirovirus: NP P/C/V M F HN - L
The genus Rubulavirus: NP P/V M F HN (SH) L
The genus Morbillivirus: NP P/C/V M F H - L

The negative-strand RNA viral vectors of the present invention may lack any of the wild-type negative-strand RNA viral genes. The viral genomic RNA can replicate and express carried genes in cells as long as it encodes viral proteins (*i*.*e*., N, L, and P) necessary for RNP reconstitution, even when it does not encode any envelope-constituting protein. Such vectors include, for example, vectors that lack at least one of the genes encoding envelope-constituting proteins such as F, H, HN, G, M, and M1, which vary depending on the type of virus (WO 00/70055 and WO00/70070; Li, H.-O. et al., J. Virol. 74(14) 6564-6569 (2000)). For example, a vector that lacks the M, F, or HN gene, or any combination thereof, can be preferably used as a paramyxovirus vector of the present invention. Such viral vectors can be reconstituted, for example, by externally supplying the missing gene products. The viral vectors prepared as described above adhere to host cells and cause cell fusion, as wild type viruses do. However, the viral vectors do not form daughter viral particles that retain the infectivity of the original vectors, since the vector genome introduced into the cells lacks some viral genes. Therefore, such vectors are useful as safe viral vectors for one-time gene introduction. Examples of genes deleted from the genome include the F gene and HN gene. In particular, vectors lacking at least the F gene are preferred in the present invention. For example, viral vectors can be reconstituted by transfecting host cells with a plasmid expressing a recombinant negative-strand RNA viral vector genome lacking the F gene, along with a vector expressing the F protein and a vector expressing the N, P, and L proteins (International Publication Numbers WO 00/70055 and WO 00/70070; Li, HO. et al., J. Virol. 74(14) 6564-6569 (2000)). Viruses can also be produced, for example, using host cells comprising F gene-integrated chromosomes. When these proteins are externally supplied, their amino acid sequences are not necessarily identical to those of virus-derived sequences. Mutations may be introduced into the proteins, and/or homologous genes from other viruses may be used as substitutes, as long as the activity of nucleic acid introduction is equivalent to or greater than that of naturally-occurring proteins.

Furthermore, amplification of the genomic RNA after introduction into cells can be prevented, when at least one of the genes encoding viral proteins (*i.e*., N, L, and P) necessary for RNP reconstitution is deleted or deficient. Such vectors can be produced by expressing the N, P, and L proteins in virus-producing cells.

The viral vectors of the present invention may be, for example, vectors that comprise, on the envelope surface thereof, proteins such as adhesion factors capable of adhering to specific cells, ligands, receptors and such, or antibodies or fragments thereof. Alternatively, the vectors may comprise chimeric proteins or such that have the above-mentioned proteins in their extracellular domain and polypeptides derived from the virus envelope in their intracellular domain. Vectors that target and infect specific tissues can thereby be produced. These proteins may be encoded by the viral genome, or may be supplied by expressing genes other than those in the viral genome (for example, genes carried by other expression vectors, or genes in the host chromosomes) at the time of viral vector reconstitution.

In the vectors of the present invention, any viral gene comprised may be altered from the wild type gene, for example, to reduce the immunogenicity of viral proteins, or to enhance the efficiency of RNA transcription or replication. Specifically, the transcriptional or replicational function of negative-strand RNA viral vector can be enhanced, for example, by altering at least one of the replication factor genes, N, P, and L. The HN protein, which is an envelope protein, has both hemagglutinin activity and neuraminidase activity. For example, the viral stability in blood can be enhanced by attenuating the hemagglutinin activity, and infectivity can be controlled by modifying the neuraminidase activity. The membrane fusion ability can be controlled by altering the F protein. Furthermore, for example, the antigen-presenting epitopes of the F protein or HN protein which may act as antigenic molecules on the cell surface can be analyzed, and this information can be used to prepare viral vectors that have a reduced antigenicity of these proteins. Furthermore, a temperature-sensitive mutation may be introduced into a viral gene to suppress release of secondarily released particles (or virus-like particles (VLPs)) (WO 2003/025570). For example, the following mutations can be introduced: G69E, T116A, and A183S for the M gene; A262T, G264, and K461 G for the HN gene; L511F for the P gene; and N1197S and K1795E for the L gene. However, temperature-sensitive mutations that can be introduced are not limited thereto (see WO 2003/025570).

In the present invention, the genomic RNA of the above-described negative-strand RNA viral vector comprises a foreign gene encoding an anti-inflammatory cytokine. Herein, "anti-inflammatory cytokine" (also referred to as "anti-inflammation cytokine") collectively refers to polypeptides that function to suppress inflammation, which include signaling molecules that promote signal transduction that leads to suppression of inflammation, and/or signaling molecules that inhibit signal transduction that leads to enhancement of inflammation (for example, pro-inflammatory cytokine inhibitors). Specifically, in the present invention, the anti-inflammatory cytokines include interleukin (IL)-4, IL-10, IL-11, IL-13, TGF-β, soluble TNF-α receptor, IL-1 receptor antagonist (IL-1ra), and other Th2 cytokines. "Th2 cytokines" refers to cytokines produced predominantly by type-2 helper T cells (Th2 cells) rather than by type-1 helper T cells (Th1 cells). Specifically, such Th2 cytokines include IL-4, IL-5, IL-6, IL-9, IL-10, and IL-13. An anti-inflammatory cytokine encoded by a vector may be a full-length natural polypeptide, or may be a partial peptide thereof (active fragment *etc*.), as long as it retains the activity. For example, deletion ofN- or C-terminal amino acid residue(s) (for example, one to 30 amino acids, more specifically, one, two, three, four, five, ten, 15, 20, or 25 amino acids) probably has no influence on the cytokine activity. Alternatively, the anti-inflammatory cytokines may be polypeptides that inhibit signal transduction of pro-inflammatory cytokines, and include a soluble fragment of pro-inflammatory cytokine receptor (comprising a ligand-binding domain), or an antibody or antibody fragment that binds to the ligand-binding domain of a pro-inflammatory cytokine receptor. For the pro-inflammatory cytokine, a desired fragment comprising a mature polypeptide that lacks signal sequence can be used, and a desired protein signal sequence can be appropriately used as the N-terminal signal sequence for its secretion to the outside of cells. The secretory signal sequences include, for example, the signal sequences of desired secretory proteins such as interleukin (IL)-2 and tissue plasminogen activator (tPA), but are not limited thereto. Alternatively, the cytokine may be expressed as a protein fused to other peptide(s).

In the present invention, the anti-inflammatory cytokine is preferably selected from the group consisting of IL-4, IL-10, IL-13, and TGF-beta, and is more preferably IL-10. The nucleotide sequence of each cytokine gene and the corresponding amino acid sequence are known (IL-4: NM_000589, NP_000580, AAH66277, AAH67515, NP_758858, NP_067258, and NP_958427; IL-10: NM_000572, NP_000563, CAG46825, NP_034678, and NP_036986; IL-13: NM_002188, NP_002179, AAB01681, NP_0323 81, and NP_446280; and TGF-beta (transforming growth factor-beta): M_60316).

Genes encoding anti-inflammatory cytokines can be obtained by hybridization using the above-exemplified anti-inflammatory cytokine genes, or such as probes. High-stringency conditions for hybridization include, for example, overnight prehybridization at 42°C followed by overnight hybridization at 42°C in a hybridization solution containing 25% formamide, 4x SSC, 50 mM Hepes (pH 7.0), 10x Denhardt's solution, and 20 µg/ml denatured salmon sperm DNA, or in a hybridization solution containing 50% formamide, 4x SSC, 50 mM Hepes (pH 7.0), 10x Denhardt's solution, and 20 µg/ml denatured salmon sperm DNA for more stringent conditions. Post-hybridization wash may be carried out under the washing and temperature conditions of "1x SSC, 0.1% SDS, 37°C" or such, "0.5x SSC, 0.1% SDS, 42°C" or such for more stringent conditions, or "0.2x SSC, 0.1% SDS, 65°C" for yet more stringent conditions. Furthermore, slight mutations causing no functional loss in protein can be introduced into natural cytokine genes by known methods. For example, site-directed mutations can be introduced by the PCR method, cassette mutagenesis method, or such. Alternatively, random mutations can be introduced by using chemical reagents, random nucleotides, or such. The amino acid sequence of an anti-inflammatory cytokine obtained by such method normally has a high homology to the amino acid sequence of the above-exemplified anti-inflammatory cytokine. "High homology" refers to sequence identity of at least 60% or more, preferably 80% or more, more preferably 90% or more, even more preferably at least 95% or more, and still more preferably at least 97% or more (for example, 98 to 99%). Such amino acid sequence identity can be determined, for example, using the BLAST algorithm by Karlin and Altschul (Proc. Natl. Acad. Sci. USA 87:2264-2268, 1990; and Proc. Natl. Acad. Sci. USA 90:5873-5877, 1993). When amino acid sequences are analyzed using BLASTX developed based on this algorithm (Altschul et al. J. Mol. Biol. 215: 403-410, 1990), the parameters are set, for example, as follows: score = 50 and wordlength = 3. When the BLAST and Gapped BLAST programs are used, the default parameters of each program are used. Specific procedures of these analytical methods are known (see the webpage of NCBI).

The activity of each anti-inflammatory cytokine can be detected by known methods. For example, methods for detecting activity by growth assay using the mouse mast cell MC/9 (ATCC CRL-8306), the human erythroleukemia cell line TF-1 (ATCC CRL-2003), or such are known (Thompson-Snipes, L. et al., 1991, J. Exp. Med. 173:507-510; Kruse N et al., EMBO J. 1993; 12:5121-5129; Oshima Y et al., J Biol Chem 2001; 276:15185-91; Oshima, Y., et al., J. Biol. Chem. 275, 14375-14380, 2000; Leland, P. et al., Oncol. Res. 7, 227-235, 1995). For example, various anti-inflammatory cytokine deletion mutants prepared using genetic recombination techniques can be assayed by the methods described above to identify active fragments. 50% effective dose (ED₅₀) is calculated. It is preferable to use partial peptides or such that have an activity of 50% or more, preferably 60% or more, 70% or more, 80% or more, 90% or more, or 95% or more when compared to the wild type.

There is no particular limitation on the origin of an anti-inflammatory cytokine encoded by the vector. The anti-inflammatory cytokine may be derived from any mammals, such as mice, rats, guinea pigs, rabbits, pigs, cattle, horses, donkeys, goats, dogs, chimpanzees, monkeys, and human. However, it is appropriate to use an anti-inflammatory cytokine derived from the same species as the subject of administration. As described above, the nucleotide sequences of the nucleic acids encoding such cytokines are available from databases. More specifically, for example, the sequence of mouse IL-10 is available under Genbank Accession Nos. AY410237 and NM_010548, and the sequence of human IL-10 is available under Genbank Accession Nos. AY029171 and NM_000572. For the site of inserting a foreign gene, a desired site can be selected, for example, within the non-protein-coding region of a virus genome. A foreign gene can be inserted, for example, between the 3' leader region of genomic RNA and the viral protein ORF closest to the 3' end, between the viral protein ORFs, and/or between the viral protein ORF closest to the 5' end and the 5' trailer region. Alternatively, in a genome in which the genes for envelope-constituting proteins, such as the M, F, or HN gene, are deleted, a foreign gene can be inserted into the deleted regions. When a foreign gene is introduced into a *paramyxoviridae* virus, the chain length of a polynucleotide fragment inserted into the genome is desirably a multiple of six (Kolakofski, D. et al., J. Virol. 1998: 72; 891-899; Calain, P. and Roux, L. J. Virol. 1993: 67; 4822-4830). An E-I-S sequence is arranged to be between the inserted foreign gene and the viral ORF. Two or more foreign genes can be inserted in tandem via E-I-S sequences.

In the present invention, "gene" refers to a genetic substance, *i*.*e*., a nucleic acid encoding a transcriptional unit. Nucleic acids include RNAs and DNAs. In the present invention, a nucleic acid encoding a polypeptide is referred to as a gene for the polypeptide. Furthermore, genes include those do not encode protein. For example, a gene may encode a functional RNA, such as a ribozyme or an antisense RNA. In general, a gene may be a naturally-occurring or artificially-designed sequence. In the present invention, "DNAs" includes both single-stranded and double-stranded DNAs. "Encoding a protein" means that a polynucleotide comprises an ORF that encodes an amino acid sequence of the protein in the sense or antisense direction, so that the protein can be expressed under appropriate conditions. In the present invention, "foreign gene" refers to a gene that is not carried by the wild-type virus from which a vector is derived.

Expression levels of a foreign gene carried by a vector can be controlled using the type of transcriptional initiation sequence added upstream (to the 3'-side of the minus strand) of the gene (WO01/18223). The expression levels can also be controlled by the position at which the foreign gene is inserted in the genome: the nearer the insertion position is to the 3'-end of the minus strand, the higher the expression level; conversely, the nearer the insertion position is to the 5'-end, the lower the expression level. Since it is generally advantageous to obtain high expression of an anti-inflammatory cytokine, it is preferable to link the anti-inflammatory cytokine-encoding gene to a highly efficient transcriptional initiation sequence, and to insert it near the 3'-end of the minus strand genome. Specifically, a foreign gene may be inserted between the 3'-leader region and the viral protein ORF closest to the 3'-end. Alternatively, a foreign gene may be inserted between the ORFs of the viral protein gene closest to the 3'-end and the second closest viral protein gene, or between the ORFs of the second and third closest viral protein genes. In wild type paramyxoviruses, the viral protein gene closest to the 3'-end of the genome is the N gene, the second closest gene is the P gene, and the third closest gene is the M gene. Alternatively, in those cases wherein a high level of expression of the antigen polypeptide is undesirable, the level of viral vector gene expression can be suppressed to obtain an appropriate effect, for example, by inserting the foreign gene at a site as close as possible to the 5'-side of the minus strand genome, or by selecting an inefficient transcriptional initiation sequence.

For example, a desired S sequence of a negative-strand RNA virus may be used as the S sequence to be attached when inserting a foreign gene-encoding nucleic acid into the genome. The consensus sequence 3'-UCCCWVUUWC-5' (W= A or C; V= A, C, or G) (SEQ ID NO: 1) can be preferably used for Sendai viruses. Particularly preferred sequences are 3'-UCCCAGUUUC-5' (SEQ ID NO: 2), 3'-UCCCACUUAC-5' (SEQ ID NO: 3), and 3'-UCCCACUUUC-5' (SEQ ID NO: 4). When shown as plus strand-encoding DNA sequences, these sequences are 5'-AGGGTCAAAG-3' (SEQ ID NO: 5), 5'-AGGGTGAATG-3' (SEQ ID NO: 6), and 5'-AGGGTGAAAG-3' (SEQ ID NO: 7). A preferred E sequence of a Sendai viral vector is, for example, 3'-AUUCUUUUU-5' (SEQ ID NO: 8) or 5'-TAAGAAAAA-3' (SEQ ID NO: 9) for the plus strand-encoding DNA. An I sequence may be, for example, any three nucleotides, specifically 3'-GAA-5' (5'-CTT-3' in the plus strand DNA).

As described above, the vector of the present invention may comprise another foreign gene at a position other than the position into which a gene encoding an anti-inflammatory cytokine is inserted. There is no limitation on such foreign genes. The foreign genes may be, for example, marker genes for monitoring vector infection, genes for cytokines, hormones, receptors, or antibodies that regulate the immune system, or fragments thereof, or other genes. The vectors of the present invention enable expression of anti-inflammatory cytokines via direct (*in vivo*) administration to a living body, or via indirect (*ex vivo*) administration which introduces a vector of the present invention into patient-derived cells or other cells and administers the cells to patients.

The negative-strand RNA viral vectors of the present invention do not encode the Aβ antigen. In other words, the negative-strand RNA viral vectors of the present invention do not comprise any nucleic acid encoding the Aβ antigen. The vectors of the present invention do not encode the Aβ antigen, and can therefore exert therapeutic effects on Alzheimer's disease. "Aβ antigen" refers to Aβ or an antigenic partial peptide thereof, and includes Aβ1-38, Aβ1-39, Aβ1-40, Aβ1-42, Aβ1-43, and Aβ1-44, and polypeptides comprising an antigenic partial fragment thereof. The negative-strand RNA viral vectors of the present invention do not encode, for example, polypeptides that comprise ten or more consecutive amino acids (preferably, nine, eight, seven, six, or five or more amino acids) from Aβ1-43 (DAEFRHDSGYEVHHQKLVFFAEDVGSNKGAIIGLMVGGVVIAT, SEQ ID NO: 10).

Recombinant negative-strand RNA viral vectors may be reconstituted using known methods. For example, such vectors can be produced by the steps of (a) transcribing DNA which encodes the genomic RNA of a negative-strand RNA virus encoding an anti-inflammatory cytokine, or the complementary strand thereof (antigenomic RNA, plus-strand), in mammalian cells or avian cells in the presence of viral proteins constituting RNP comprising the genomic RNA of the negative-strand RNA virus, and (b) collecting the produced negative-strand RNA viruses or RNP comprising the genomic RNA. The "viral proteins constituting RNP" mentioned above refers to proteins that form RNP together with the viral genomic RNA and constitute a nucleocapsid. These are a group of proteins necessary for genome replication and gene expression, and are typically N (nucleocapsid (also referred to as nucleoprotein (NP))-, P (phospho)-, and L (large)-proteins. Although these notations vary depending on viral species, corresponding proteins are known to those skilled in the art (Anjeanette Robert et al., Virology 247:1-6 (1998)). For example, "N" may be denoted as "NP".

When reconstituting viruses, a negative-strand RNA genome (*i*.*e*. antisense strand, which is the same as the viral genome) or the plus-strand RNA (antigenome, the complementary strand of the genomic RNA) may be generated as described above. However, in order to increase the efficiency of vector reconstitution, the plus-strand is preferably generated. The viral genomic RNA may be deficient in genes encoding envelope-constituting proteins, as long as it encodes viral proteins required for RNP reconstitution. For example, the genomic RNA does not need to encode viral proteins, such as F, HN, and M, as long as it encodes the N, P, and L proteins. Such defective viruses can amplify the genomic RNA in cells, but do not release infectious virions, and thus are useful as highly safe gene transfer vectors (WO00/70055, WO00/70070, and WO03/025570; Li, H.-O. et al., J. Virol. 74(14) 6564-6569 (2000)). To produce a viral vector, the above envelope-constituting proteins are expressed separately in virus-producing cells to complement particle formation. In order to express viral proteins and RNA genome in cells, a vector linked with DNA that encodes the proteins or genome downstream of an appropriate promoter is introduced into host cells. The promoter used include, for example, CMV promoters (Foecking, M. K. and Hofstetter, H. (1986) Gene 45: 101-105), retrovirus LTRs (Shinnik, T. M., Lerner, R. A. and Sutcliffe (1981) Nature, 293, 543-548), EF1 promoters, and CAG promoters (Niwa, H. et al. (1991) Gene. 108: 193-199, and Japanese Patent Application Kokai Publication No. (JP-A) H3-168087 (unexamined, published Japanese patent application)).

The terminals of genomic RNA preferably reflect the terminals of the 3'-leader sequence and 5'-trailer sequence as accurately as possible, as in the natural viral genome. For example, a self-cleaving ribozyme is added at the 5'-end of the transcript to allow the ribozyme to accurately cleave off the end of the negative-strand RNA viral genome (Inoue, K. et al. J. Virol. Methods 107, 2003, 229-236). Alternatively, in order to accurately regulate the 5'-end of the transcript, the recognition sequence of bacteriophage RNA polymerase is used as a transcription initiation site, and the RNA polymerase is expressed within a cell to induce transcription. The bacteriophage RNA polymerase used include, for example, those of E. coli T3 phage and T7 phage, and Salmonella SP6 phage (Krieg, P.A. and Melton, D.A. 1987, Methods Enzymol. 155: 397-15; Milligan, J.F. et al., 1987, Nucleic Acids Res. 15: 8783-798; Pokrovskaya, I.D. and Gurevich, V.V., 1994, Anal. Biochem. 220: 420-23). Such bacteriophage RNA polymerases can be supplied using, for example, vaccinia viruses expressing the polymerases (Fuerst, T.R. et al., Proc. Natl. Acad. Sci. USA 83, 8122-8126 (1986), or supplied from expression vectors such as plasmids. To regulate the 3'-end of the transcript, for example, a self-cleaving ribozyme is encoded at the 3'-end of the transcript, allowing accurate cleavage of the 3'-end with this ribozyme (Hasan, M. K. et al., J. Gen. Virol. 1997: 78: 2813-2820; Kato, A. et al., EMBO J. 1997, 16: 578-587; and Yu, D. et al., Genes Cells 1997, 2: 457-466). An auto-cleaving ribozyme derived from the antigenomic strand of delta hepatitis virus can be used.

In the reconstitution of viruses in which the envelope-constituting protein genes have been deleted, the infectivity of produced viruses can be complemented by expressing the deleted envelope-constituting proteins and/or other envelope proteins in virus-producing cells. For example, the viruses may also be pseudotyped with envelope proteins of negative-strand RNA viruses of a different origin from the virus from which the viral vector genome is derived. Such an envelope protein used may be, for example, the G protein of vesicular stomatitis virus (VSV) (VSV-G) (J. Virology 39: 519-528 (1981)) (Hirata, T. et al., 2002, J. Virol. Methods, 104:125-133; Inoue, M. et al., 2003, J. Virol. 77:6419-6429; Inoue M. et al., J Gene Med. 2004;6:1069-1081). Genes to be deleted from the genome include, for example, genes of spike proteins such as F, HN, H, and G, genes of envelope-lining proteins such as M, and any combinations thereof. Deletion of a spike protein gene is effective in rendering negative-strand RNA viral vectors nontransmissible, whereas deletion of the gene of an envelope-lining protein such as M protein is effective in disabling the particle formation from infected cells. For example, F gene-defective negative-strand RNA viral vectors (Li, H.-O. et al., J.Virol. 74, 6564-6569 (2000)), M gene-defective negative-strand RNA viral vectors (Inoue, M. et al., J.Virol. 77, 6419-6429 (2003)), and the like are preferably used. Moreover, greater safety would be assured with vectors defective in any combination of at least two of F, HN (or H) and M genes. For example, vectors lacking both M and F genes are nontransmissible and defective in particle formation while retaining high level infectivity and gene expression ability.

For instance, in an example of the production of F gene-defective recombinant viruses, a plasmid expressing a negative-strand RNA viral genome defective in F gene or a complementary strand thereof is transfected into host cells along with an expression vector expressing F protein and expression vectors for N, P, and L proteins. Alternatively, viruses can be more efficiently produced by using host cells in which the F gene has been incorporated into their chromosomes (WO00/70070). In this case, a sequence-specific recombinase such as Cre/loxP and FLP/FRT and a target sequence thereof are preferably used so that the F gene can be inducibly expressed (see WO00/70055, WO00/70070; Hasan, M. K. et al., 1997, J. General Virology 78: 2813-2820). Specifically, for example, the envelope protein genes are integrated into a vector having a recombinase target sequence, such as the Cre/loxP inducible expression plasmid pCALNdlw (Arai, T. et al., J. Virology 72, 1998, p1115-1121). The expression is induced by, for example, infection with the adenovirus AxCANCre at an MOI of 3 to 5 (Saito et al., Nucl. Acids Res. 23: 3816-3821 (1995); andArai, T. et al., J. Virol 72, 1115-1121 (1998)).

The negative-strand RNA viruses used in the present invention may be deficient in accessory genes. For example, by knocking out the V gene, one of the accessory genes of Sendai virus (SeV), the pathogenicity of SeV toward hosts such as mice is remarkably reduced without hindering gene expression and replication in cultured cells (Kato, A. et al., 1997, J. Virol. 71:7266-7272; Kato, A. et al., 1997, EMBO J. 16:578-587; Curran, J. et al.; WO01/04272; and EP1067179).

In addition, negative-strand RNA viruses used may include mutations in the P gene or L gene so as to enhance the persistence of infection. Specific examples of such mutations include mutation of Glu at position 86 (E86) of the SeV P protein, substitution of Leu at position 511 (L511) of the SeV P protein to another amino acid, or substitution of homologous sites in the P protein of a different negative-strand RNA virus. Specific examples include substitution of the amino acid at position 86 to Lys, and substitution of the amino acid at position 511 to Phe. Regarding the L protein, examples include substitution of Asn at position 1197 (N1197) and/or Lys at position 1795 (K1795) in the SeV L protein to other amino acids, or substitution of homologous sites in the L protein of another negative-strand RNA virus, and specific examples include substitution of the amino acid at position 1197 to Ser, and substitution of the amino acid at 1795 to Glu. Mutations of the P gene and L gene can significantly increase the effects of persistent infectivity, suppression of the release of secondary virions, and suppression of cytotoxicity.

Regarding more specific methods for the reconstitution of recombinant viruses, one can refer to, for example, the following references: WO97/16539; WO97/16538; WO00/70055; WO00/70070; WO01/18223; WO03/025570; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404; Hasan, M. K. et al., J. Gen. Virol. 78: 2813-2820, 1997; Kato, A. et al., 1997, EMBO J. 16: 578-587; Yu, D. et al., 1997, Genes Cells 2: 457-466; Tokusumi, T. et al. Virus Res. 2002: 86; 33-38; Li, H.-O. et al., J. Virol. 2000: 74; 6564-6569. Following these methods, negative-strand RNA viruses including parainfluenza virus, vesicular stomatitis virus; rabies virus, measles virus, rinderpest virus, Sendai virus, and the like can be reconstituted from DNA.

The present invention provides methods for producing therapeutic and/or preventive agents for Alzheimer's disease (pharmaceutical compositions for treating and/or preventing Alzheimer's disease), which comprise the steps of:
(a) allowing transcription of a DNA that encodes the genomic RNA of a negative-strand RNA virus encoding an anti-inflammatory cytokine, or the complementary strand thereof (antigenomic RNA), in the presence of viral proteins that constitute an RNP comprising the genomic RNA of a negative-strand RNA virus in mammalian cells; and
(b) collecting the generated negative-strand RNA virus or RNP that comprises the genomic RNA.

The present invention also relates to the use of DNAs that encode the genomic RNA of a negative-strand RNA virus encoding an anti-inflammatory cytokine, or the complementary strand thereof (antigenome RNA), in producing therapeutic and/or preventive agents for Alzheimer's disease (pharmaceutical compositions for treating and/or preventing Alzheimer's disease). Furthermore, the present invention relates to therapeutic and/or preventive agents for Alzheimer's disease (pharmaceutical compositions for treating and/or preventing Alzheimer's disease), which comprise as an active ingredient, a negative-strand RNA virus encoding an anti-inflammatory cytokine. The present invention also relates to the use of anti-inflammatory cytokines, cells producing anti-inflammatory cytokines, nucleic acids encoding an anti-inflammatory cytokine, and cells into which a nucleic acid encoding an anti-inflammatory cytokine has been exogenously introduced, in producing therapeutic and/or preventive agents for Alzheimer's disease (pharmaceutical compositions for treating and/or preventing Alzheimer's disease). In addition, the present invention relates to therapeutic and/or preventive agents for Alzheimer's disease (pharmaceutical compositions for treating and/or preventing Alzheimer's disease), which comprise as an active ingredient, an anti-inflammatory cytokine, cells producing an anti-inflammatory cytokine, a nucleic acid encoding an anti-inflammatory cytokine, or cells into which a nucleic acid encoding an anti-inflammatory cytokine has been exogenously introduced.

Desired mammalian cells and the like can be used for virus production. Specific examples of such cells include cultured cells, such as LLC-MK2 cells (ATCC CCL-7) and CV-1 cells (for example, ATCC CCL-70) derived from monkey kidney, BHK cells (for example, ATCC CCL-10) derived from hamster kidney, and cells derived from humans. In addition, to obtain a large quantity of a virus vector, a viral vector obtained from an above-described host can be used to infect embryonated hen eggs to amplify the vector. Methods for manufacturing viral vectors using hen eggs have already been developed (Nakanishi, et al., ed. (1993), "State-of-the-Art Technology Protocol in Neuroscience Research III, Molecular Neuron Physiology", Koseisha, Osaka, pp. 153-172). For example, a fertilized egg is placed in an incubator, and cultured for nine to twelve days at 37 to 38°C to grow an embryo. After the viral vector is inoculated into the allantoic cavity, the egg is then cultured for several days (for example, three days) to proliferate the viral vector. Conditions, such as the period of culture, may vary depending upon the recombinant Sendai virus being used. Then, allantoic fluids, including the vector, are recovered. Separation and purification of a Sendai virus vector from allantoic fluids can be performed according to conventional methods (Tashiro, M., "Virus Experiment Protocol," Nagai, Ishihama, ed., Medical View Co., Ltd., pp. 68-73, (1995)).

Titers of viruses recovered can be determined, for example, by measuring CIU (Cell Infectious Unit) or hemagglutination activity (HA) (WO 00/70070; Kato, A. et al., 1996, Genes Cells 1: 569-579; Yonemitsu, Y and Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Titers of vectors carrying a marker gene such as GFP (green fluorescent protein) can be quantified (for example, as GFP-CIU) by directly counting infected cells, using the marker as an index. Titers thus determined can be treated in the same way as CIU (WO 00/70070).

The viral vectors can be purified to be substantially pure. Purification can be achieved using known purification/separation methods, including filtration, centrifugation, adsorption, and column purification, or any combinations thereof. The phrase "substantially pure" means that the virus component constitutes a major proportion of a solution of the viral vector. For example, a viral vector composition can be deemed "substantially pure" based on the fact that the proportion of protein contained as the viral vector component as compared to the total protein (excluding proteins added as carriers and stabilizers) in the solution is 10% (w/w) or greater, preferably 20% or greater, more preferably 50% or greater, preferably 70% or greater, more preferably 80% or greater, and even more preferably 90% or greater. Specific purification methods for the viral vectors include, for example, methods using cellulose sulfate ester or cross-linked polysaccharide sulfate ester (Japanese Patent Application Kokoku Publication No. (JP-B) S62-30752 (examined, approved Japanese patent application published for opposition), JP-B S62-33879, and JP-B S62-30753) and methods including adsorption to fucose sulfate-containing polysaccharide and/or degradation products thereof (WO97/32010). However, the invention is not limited thereto.

The present invention also relates to compositions for treating and preventing Alzheimer's disease, and developing therapeutic and preventive agents for Alzheimer's disease, which comprise an anti-inflammatory cytokine, cells producing an anti-inflammatory cytokine, a nucleic acid encoding an anti-inflammatory cytokine, and cells into which a nucleic acid encoding an anti-inflammatory cytokine has been exogenously introduced. In particular, the present invention relates to compositions for treating and preventing Alzheimer's disease, and developing therapeutic and preventive agents for Alzheimer's disease, which comprise a negative-strand RNA viral vector carrying an anti-inflammatory cytokine gene, cells producing the viral vector, or cells into which the vector has been introduced. When producing compositions comprising a vector, the vector may be combined with a desired pharmaceutically acceptable carrier or vehicle as needed. "Pharmaceutically acceptable carrier or vehicle" includes desired solutions in which the vector or cells can be suspended, for example, phosphate-buffered saline (PBS), sodium chloride solutions, Ringer's solution, and culture media. In the case where the vector is amplified using hen eggs, or such, it may contain allantoic fluid. Furthermore, compositions comprising the vector may comprise a carrier or vehicle such as deionized water and an aqueous solution of 5% dextrose. In addition, the compositions may also contain vegetable oils, suspending agents, surfactants, stabilizers, biocidal agents, or such. Preservatives or other additives may also be added. The compositions of the present invention do not comprise an Aβ antigen or a nucleic acid encoding an Aβ antigen. The vectors of the present invention and composition comprising them are useful in treating and preventing Alzheimer's disease, and developing therapeutic and preventive agents for Alzheimer's disease. The present invention relates to methods for producing therapeutic and preventive agents for Alzheimer's disease, which comprise the step of producing a composition comprising an anti-inflammatory cytokine, cells producing an anti-inflammatory cytokine, a nucleic acid encoding an anti-inflammatory cytokine, or cells into which a nucleic acid encoding an anti-inflammatory cytokine has been exogenously introduced, and a pharmaceutically acceptable carrier or vehicle. The present invention also relates to the use of an anti-inflammatory cytokine, cells producing an anti-inflammatory cytokine, a nucleic acid encoding an anti-inflammatory cytokine, or cells into which a nucleic acid encoding an anti-inflammatory cytokine has been exogenously introduced, in producing therapeutic and preventive agents for Alzheimer's disease. In addition, the present invention relates to methods for producing therapeutic and preventive agents for Alzheimer's disease, which comprise the step of producing a composition comprising a negative-strand RNA viral vector carrying an anti-inflammatory cytokine gene, cells producing the viral vector, or cells into which the vector has been introduced, and a pharmaceutically acceptable carrier or vehicle. The present invention also relates to the use of a negative-strand RNA viral vector carrying an anti-inflammatory cytokine gene, cells producing the viral vector, or cells into which the vector has been introduced, in producing therapeutic and preventive agents for Alzheimer's disease. By using vectors of the present invention, the blood level of an anti-inflammatory cytokine can be increased very efficiently, thereby achieving a high AUC (area under the pharmacokinetic curve). This enables effective suppression of Aβ accumulation and senile plaque formation.

Compositions comprising a vector of the present invention can be combined with, as a carrier, an organic substance such as a biopolymer, or an inorganic substance such as hydroxyapatite; specifically, a collagen matrix, a polylactate polymer or copolymer, a polyethylene glycol polymer or copolymer, and a chemical derivative thereof, etc.

Furthermore, compositions of the present invention, for example, compositions comprising a negative-strand RNA viral vector carrying an anti-inflammatory cytokine gene, may further comprise an anti-inflammatory cytokine or a nucleic acid encoding an anti-inflammatory cytokine. Such anti-inflammatory cytokines include those described herein and combinations thereof. Preferably, the anti-inflammatory cytokine is selected from the group consisting of IL-4, IL-10, and IL-13.

Moreover, the compositions of the present invention may comprise an adjuvant. For example, the use of a composition comprising a Th2 adjuvant can further promote a shift in the Th1/Th2 balance toward Th2. The term "Th2 adjuvant" refers to adjuvants which activate type II helper T cells (Th2 cells) more predominantly than type I helper T cells (Th1 cells). Specifically, aluminum hydroxide (alum), cholera toxin (B subunit), *Schistosoma mansoni* egg extract proteins (such as Lacto-N-fucopentaose III), and the like may be used (Grun, J. L. and P. H. Maurer, 1989, Cellular Immunology 121: 134-145; Holmgren J et al., 1993, Vaccine 11:1179-1184; Wilson AD et al., 1993, Vaccine 11:113-118; Lindsay DS et al, 1994, Int Arch Allergy Immunol 105:281-288; Xu-Amano J et al., 1993, J Exp Med 178:1309-1320; Okano M et al., 2001, J Immunol. 167(1):442-50).

The negative-strand RNA viruses encoding an anti-inflammatory cytokine and the compositions of the present invention, such as compositions comprising the viruses, are used for treating or preventing Alzheimer's disease, or developing therapeutic or preventive agents for Alzheimer's disease. Herein, "used for treating or preventing Alzheimer's disease, or developing therapeutic or preventive agents for Alzheimer's disease" refers to exclusive use for treating or preventing Alzheimer's disease, or for development of therapeutic or preventive agents for Alzheimer's disease. "Development of therapeutic or preventive agents" means that at least one therapeutic or preventive effect on Alzheimer's disease is detected in a negative-strand RNA virus or a composition comprising the virus when its effectiveness either as a therapeutic or preventive agent is assessed. "Treatment of Alzheimer's disease" means amelioration of at least one symptom of Alzheimer's disease, including, for example, reduction of Aβ accumulation in brain tissues or blood, or decrease of senile plaques or their area. The compositions of the present invention are useful as agents for suppressing Aβ accumulation, in particular, agents for suppressing Aβ accumulation in brain tissues, blood, or such, as compared to when the compositions of the present invention are not administered. Alternatively, the compositions of the present invention are useful as agents for suppressing senile plaque, in particular, agents for reducing the number and/or the total area of senile plaques, as compared to when the compositions of the present invention are not administered. In the present invention, the treatment or prevention of Alzheimer's disease, or the development of a therapeutic or preventive agent for Alzheimer's disease does not comprise the step of administering an Aβ antigen or a nucleic acid encoding an Aβ antigen to an individual. The present invention relates to the use of an anti-inflammatory cytokine, cells producing an anti-inflammatory cytokine, a nucleic acid encoding an anti-inflammatory cytokine, or cells into which a nucleic acid encoding an anti-inflammatory cytokine has been exogenously introduced, in particular, a negative-strand RNA viral vector carrying an anti-inflammatory cytokine gene, for treating or preventing Alzheimer's disease, or for developing therapeutic or preventive agents for Alzheimer's disease. The present invention also relates to the use of an anti-inflammatory cytokine, cells producing an anti-inflammatory cytokine, a nucleic acid encoding an anti-inflammatory cytokine, or cells into which a nucleic acid encoding an anti-inflammatory cytokine has been exogenously introduced, in particular, a negative-strand RNA viral vector carrying an anti-inflammatory cytokine gene, in producing pharmaceutical compositions for treating or preventing Alzheimer's disease.

Furthermore, the present invention relates to packages and kits for preventing and/or treating Alzheimer's disease, which comprise vessels containing an anti-inflammatory cytokine, cells producing an anti-inflammatory cytokine, a nucleic acid encoding an anti-inflammatory cytokine, or cells into which a nucleic acid encoding an anti-inflammatory cytokine has been exogenously introduced. In particular, the present invention relates to packages and kits for preventing and/or treating Alzheimer's disease, which comprise vessels containing a negative-strand RNA viral vector carrying an anti-inflammatory cytokine gene. The vectors used may be those described herein. The vessels preferably have a configuration suitable to store active ingredients such as the negative-strand RNA viral vector carrying an anti-inflammatory cytokine gene in sterile conditions. Specifically, the vessels may be a glass or plastic ampule, vial, tube, bottle, syringe, or such. The packages and kits may further comprise an anti-inflammatory cytokine or another vector encoding an anti-inflammatory cytokine. The anti-inflammatory cytokine described herein and combinations thereof may be used. Preferably, the anti-inflammatory cytokine is selected from the group consisting of IL-4, IL-10, and IL-13. The packages and kits do not contain an Aβ antigen or a nucleic acid encoding an Aβ antigen. The negative-strand RNA viral vectors may be made into, for example, compositions suitable for nasal administration. The vessels, packages, and/or kits may contain descriptions or instructions on the use of active ingredients such as an anti-inflammatory cytokine, or a gene encoding the cytokine, for preventing and/or treating Alzheimer's disease. For example, kits comprising a negative-strand RNA viral vector carrying an anti-inflammatory cytokine gene may contain descriptions or instructions on the use of the vector for preventing and/or treating Alzheimer's disease. Furthermore, the vessels, packages, and/or kits may contain descriptions or instructions that neither an Aβ antigen nor a nucleic acid encoding an Aβ antigen is used in used in combination. The kits of the present invention are useful, for example, for suppression of Aβ accumulation, in particular, for suppressing Aβ accumulation in brain tissues, blood, or such, as compared to when the kits are not used. Furthermore, the kits of the present invention are useful for suppressing senile plaques, in particular, for reducing the number and/or the total area of senile plaques, as compared to when the kits are not used.

Alzheimer's disease can be treated and prevented by directly or indirectly administering an anti-inflammatory cytokine or a nucleic acid expressing an anti-inflammatory cytokine to an individual. In particular, Alzheimer's disease can be effectively treated and prevented by administering a negative-strand RNA virus carrying an anti-inflammatory cytokine gene or a composition comprising the virus to an individual. The present invention provides methods for treating and/or preventing Alzheimer's disease, which comprise the step of directly or indirectly administering an anti-inflammatory cytokine or a nucleic acid expressing an anti-inflammatory cytokine. In particular, the present invention provides methods for treating and/or preventing Alzheimer's disease, which comprise the step of administering a negative-strand RNA viral vector carrying an anti-inflammatory cytokine gene. The methods of the present invention do not comprise the step of administering an Aβ antigen or a nucleic acid encoding the antigen. The methods of the present invention enable the treatment of Alzheimer's disease without administering an exogenous Aβ antigen. The administration of an anti-inflammatory cytokine or a vector carrying an anti-inflammatory cytokine gene may be *in vivo*, or *ex vivo* via cells. When a negative-strand RNA viral vector is administered, the vector may be an infectious viral particle, a non-infectious viral particle, a viral core (an RNP complex containing a genome and genome-binding viral proteins), or such. In the present invention, "negative-strand RNA viral vector" refers to complexes that include a ribonucleoprotein (RNP) complex comprising the genomic RNA derived from the negative-strand RNA virus and viral proteins necessary for replicating the RNA and expressing the carried gene, and that replicate the genomic RNA and express the carried gene in infected cells. The RNP is, for example, a complex comprising the genomic RNA of a negative-strand RNA virus and the N, L, and P proteins. Thus, in the present invention, the negative-strand RNA viral vector includes viral infectious particles, noninfectious particles (virus-like particles; also referred to as VLP), and RNPs containing a genomic RNA and viral proteins binding to the genomic RNA, such as a nucleocapsid of the negative-strand RNA virus. RNP (viral core) that is a virion from which its envelope has been removed is, when introduced into cells, still capable of replicating the viral genomic RNA in the cells (WO97/16538; WO00/70055). RNP or VLP may be administered together with, for example, a transfection reagent (WO00/70055; WO00/70070).

To administer the negative-strand RNA viral vector via cells, the negative-strand RNA viral vector is introduced into appropriate cultured cells, cells collected from an inoculation subject animal, or the like. For infecting cells with the negative-strand RNA viruses outside the body (for example, in a test tube or dish), the infection is carried out *in vitro* (or *ex vivo*), in a desired physiological aqueous solution such as a culture solution or a physiological salt solution. Herein, MOI (multiplicity of infection; number of infectious viruses per cell) is preferably within a range of one to 1000, more preferably two to 500, yet more preferably three to 300, and even more preferably five to 100. The negative-strand RNA viruses and cells can be sufficiently contacted even for a short time. The contact may be carried out, for example, for one minute or more, and preferably three minutes or more, five minutes or more, ten minutes or more, or 20 minutes or more. The duration may be for example about one to 60 minutes, and more specifically about five to 30 minutes. Of course, the contact may be carried out for a longer duration than the above, such as several days or more.

Specific methods for introducing into cells RNPs or non-infectious viral particles (virus-like particles (VLPs)) that contain viral genomic RNAs, naked DNAs, plasmids, or such include those known to those skilled in the art, such as methods that utilize calcium phosphate (Chen, C. & Okayama, H. (1988) BioTechniques 6:632-638; Chen, C. and Okayama, H., 1987, Mol. Cell. Biol. 7: 2745), DEAE-dextran (Rosenthal, N. (1987) Methods Enzymol. 152:704-709), various liposome-based transfection reagents (Sambrook, J. et al. (1989) Molecular Cloning: A Laboratory Manual (Cold Spring Harbor Laboratory, Cold Spring Harbor, NY)), or electroporation (Ausubel, F. et al. (1994) In Current Protocols in Molecular Biology (John Wiley and Sons, NY), Vol. 1, Ch. 5 and 9). Chloroquine may be added to the transfection to suppress the degradation in endosomes (Calos, M. P., 1983, Proc. Natl. Acad. Sci. USA 80: 3015). Transfection reagents include, for example, DOTMA (Roche), Superfect Transfection Reagent (QIAGEN, Cat No. 301305), DOTAP, DOPE, DOSPER (Roche #1811169), TransIT LT1 (Mirus, Product No. MIR 2300), CalPhosTM Mammalian Transfection Kit (Clontech #K2051-1), and CLONfectinTM (Clontech #8020-1). Enveloped viruses are known to incorporate host cell-derived proteins during virion formation, and such proteins can potentially cause antigenicity and cytotoxicity when introduced into cells (J. Biol. Chem. (1997) 272, 16578-16584). It is thus advantageous to use RNPs without the envelope (WO 00/70055).

Moreover, virus RNPs can be directly produced in a cell by introducing into the cell an expression vector which expresses viral genomic RNAs and an expression vector which encodes viral proteins (the N, P, and L proteins) necessary for replicating the genomic RNAs. Cells into which a viral vector has been introduced may also be produced in such a manner.

Once cells into which a negative-strand RNA viral vector has been introduced are prepared, they are preferably cultured for about twelve hours to five days (preferably for one to three days) to express an anti-inflammatory cytokine from the vector. When a signal peptide has been added to the anti-inflammatory cytokine to be expressed from the vector, the cytokine can be secreted to the outside of the cells. The resulting cells may be administered to animals without further treatment or as a cell homogenate (lysate) containing the viral vector. To eliminate the growth potential, the cells may be treated with irradiation, ultraviolet radiation, a chemical agent, or such. A lysate of cells into which the vector has been introduced can be prepared by using methods of lysing the cell membrane with surfactants, methods in which freeze-thaw cycles are repeated, or such. The surfactants include non-ionic surfactants such as Triton X-100 and Nonidet P-40. The lysate may be administered in combination with transfection reagents.

The dosage of the composition of the present invention varies depending on the disease, patient's weight, age, sex, and symptom, the purpose of administration, the form of composition administered, the administration method, the gene to be introduced, and such. The dosage can be appropriately determined by those skilled in the art. The route of administration can be appropriately selected, which includes, for example, percutaneous, intranasal, transbronchial, intramuscular, intraperitoneal, intravenous, and subcutaneous administration, but is not limited thereto. In particular, a preferred administration includes intramuscular injection (for example, into gastrocnemius muscle), subcutaneous administration, intranasal administration (nasal drop), intracutaneous administration to the palm or sole, direct intrasplenic administration, intraperitoneal administration, and such. More preferred administration methods include intranasal administration, subcutaneous administration, and intramuscular administration. When an anti-inflammatory cytokine is administered, for example, subcutaneous administration is preferred. On the other hand, when a negative-strand RNA viral vector is administered, nasal administration (including administration using nasal drop, spray, catheter, etc.) is preferred. The number of inoculation sites may be one or more (for example, two to 15 sites). The dosage of inoculation may be appropriately adjusted depending on the subject animal to be inoculated, inoculation site, inoculation frequency, and such. The dosage of a cytokine protein may be about 8000 µg per kilogram body weight (8000 µg/kg) or less (Regul Toxicol Pharmacol. 2002 Feb; 35(1):56-71), preferably 25 µg/kg to 100 µg/kg (Pharmacol Rev. 2003 Jun; 55(2):241-69). When a negative-strand RNA viral vector is used, the vector is preferably administered at a dosage in the range of about 10⁵ CIU/ml to about 10¹¹ CIU/ml, more preferably about 10⁷ CIU/ml to about 10⁹ CIU/ml, still more preferably about 1 x 10⁸ CIU/ml to about 5 x 10⁸ CIU/ml, together with a pharmaceutically acceptable carrier. When converted into a virus titer, the single dose for human is 1 x 10⁴ CIU to 5 x 10¹¹ CIU (cell infectious unit), preferably 2 x 10⁵ CIU to 2 x 10¹⁰ CIU. The frequency of administration is once or more and within the range of clinically acceptable side effects. The same applies to the number of doses per day. Although one single administration can produce significant effects, the effects can be enhanced by performing administration twice or more. Furthermore, the anti-inflammatory cytokine itself may be administered additionally.

When the vector is inoculated via cells (*ex νiνo* administration), for example, human cells, preferably autologous cells, can be infected with a negative-strand RNA viral vector, and 1x10⁴ to 10⁹ cells, preferably 1x10⁵ to 10⁸ cells, or a lysate of the cells can be administered. For non-human animals, for example, the dosage can be converted from the above-described dosage based on the body weight ratio or the volume ratio (e.g., mean value) of the target site for administration between the animal of interest and human, and administered. The subject to which a composition comprising a vector of the present invention is administered is preferably mammals (including human and non-human mammals). Specifically, the mammals include human, non-human primates such as monkeys, rodents such as mice and rats, rabbits, goats, sheep, pigs, cattle, dogs, and all other mammals. The subject animals for administration include animals and patients having at least one factor of Alzheimer's disease or at least one symptom of Alzheimer's disease. Such animals and patients include, for example, individuals with Alzheimer's disease, individuals with an increased amount of Aβ or enhanced Aβ deposition, individuals having an Alzheimer-type mutant gene, and Alzheimer's disease model animals.

The methods of the present invention ameliorate at least one symptom of Alzheimer's disease. Symptoms of Alzheimer's disease include, for example, enhancement of microglial activity; infiltration and/or accumulation of microglia in the brain, in particular, in senile plaques; accumulation of substances activated upon inflammation, e.g., complements, in the brain; accumulation and/or deposition of Aβ in the brain tissues; and impairment of learning and/or memory.

Furthermore, the present invention also relates to methods for assessing therapeutic effect on Alzheimer's disease, which comprise the steps of: administering a composition of the present invention, for example, a vector of the present invention or a composition comprising the vector, to an individual suffering from Alzheimer's disease; and detecting at least one symptom of Alzheimer's disease in the individual. The symptoms of Alzheimer's disease may be compared with a control that the composition of the present invention, for example, vector, is not administered. As described above, the symptoms of Alzheimer's disease to be assessed include enhancement of microglial activity; infiltration and/or accumulation of microglia in the brain, in particular, in senile plaques; accumulation of substances activated upon inflammation, e.g., complements, in the brain; accumulation and/or deposition of Aβ in the brain tissues; and impairment of learning and/or memory. The present invention also relates to methods for assessing therapeutic effect on Alzheimer's disease, which comprise the steps of: administering a composition of the present invention, for example, a vector of the present invention or a composition comprising the vector, to an individual; and detecting a symptom of Alzheimer's disease in the individual. The individual for administration includes those having at least one factor of Alzheimer's disease or at least one symptom of Alzheimer's disease, for example, individuals with Alzheimer's disease, individuals with an increased amount of Aβ or enhanced Aβ deposition, individuals having an Alzheimer-type mutant gene, and Alzheimer's disease model animals. When the composition is administered to individuals before the onset of Alzheimer's disease, control individuals to which the composition is not administered are monitored until they develop at least one symptom of Alzheimer's disease, and then the individuals to which the composition is administered are compared with the control individuals to evaluate the effects. Therapeutic and preventive effects on Alzheimer's disease can be monitored by using these methods.

### Examples

Hereinbelow, the present invention is specifically described with reference to the Examples; however, it should not be construed as being limited thereto. All the publications cited herein are incorporated as a part of the present specification.

### [Example 1] Construction of an F gene-deficient SeV genomic cDNA carrying the IL-10 gene (1-1) Construction of a NotI fragment of each gene (addition of the transcriptional signal of Sendai virus)

PCR was carried out using the two primers, pmIL01-N (SEQ ID NO: 13) and pmIL10-C (SEQ ID NO: 14), and cDNA of the mouse IL-10 (mIL-10) gene (Accession Number NM_010548; SEQ ID NO: 11; the amino acid sequence is shown in SEQ ID NO: 12) as a template. The resulting product was digested with *Not*I*,* and was subcloned into pBluescript^{™} II KS (Stratagene), to construct an mIL-10 gene *Not*I fragment (SEQ ID NO: 15) to which the transcriptional signal of Sendai virus has been added.

### SEQ ID NO: 11

ATGCCTGGCTCAGCACTGCTATGCTGCCTGCTCTTACTGACTGGCATGAGGATCAGCA
GGGGCCAGTACAGCCGGGAAGACAATAACTGCACCCACTTCCCAGTCGGCCAGAGC
CACATGCTCCTAGAGCTGCGGACTGCCTTCAGCCAGGTGAAGACTTTCTTTCAAACA
AAGGACCAGCTGGACAACATACTGCTAACCGACTCCTTAATGCAGGACTTTAAGGGT
TACTTGGGTTGCCAAGCCTTATCGGAAATGATCCAGTTTTACCTGGTAGAAGTGATGC
CCCAGGCAGAGAAGCATGGCCCAGAAATCAAGGAGCATTTGAATTCCCTGGGTGAG
AAGCTGAAGACCCTCAGGATGCGGCTGAGGCGCTGTCATCGATTTCTCCCCTGTGAA
AATAAGAGCAAGGCAGTGGAGCAGGTGAAGAGTGATTTTAATAAGCTCCAAGACCA
AGGTGTCTACAAGGCCATGAATGAATTTGACATCTTCATCAACTGCATAGAAGCATAC
ATGATGATCAAAATGAAAAGCTAA

### SEQ ID NO: 13

ACTTGCGGCCGCCAAAGTTCAATGCCTGGCTCAGCACTGCTATGCTGCCTG

### SEQ ID NO: 14

ATCCGCGGCCGCGATGAACTTTCACCCTAAGTTTTTCTTACTACGGTTAGCTTTTCATT
TTGATCATCATGTATGCTTC

### SEQ ID NO: 15

gcggccgccaaagttcaATGCCTGGCTCAGCACTGCTATGCTGCCTGCTCTTACTGACTGGCAT
GAGGATCAGCAGGGGCCAGTACAGCCGGGAAGACAATAACTGCACCCACTTCCCAG
TCGGCCAGAGCCACATGCTCCTAGAGCTGCGGACTGCCTTCAGCCAGGTGAAGACTT
TCTTTCAAACAAAGGACCAGCTGGACAACATACTGCTAACCGACTCCTTAATGCAGG
ACTTTAAGGGTTACTTGGGTTGCCAAGCCTTATCGGAAATGATCCAGTTTTACCTGGT
AGAAGTGATGCCCCAGGCAGAGAAGCATGGCCCAGAAATCAAGGAGCATTTGAATTC
CCTGGGTGAGAAGCTGAAGACCCTCAGGATGCGGCTGAGGCGCTGTCATCGATTTCT
CCCCTGTGAAAATAAGAGCAAGGCAGTGGAGCAGGTGAAGAGTGATTTTAATAAGCT
CCAAGACCAAGGTGTCTACAAGGCCATGAATGAATTTGACATCTTCATCAACTGCATA
GAAGCATACATGATGATCAAAATGAAAAGCTAAccgtagtaagaaaaacttagggtgaaagttcatcgcggc
cgc

### (1-2) Construction of an F gene-deficient SeV cDNA carrying the mIL-10 gene

A cDNA (pSeV18+NotI/ΔF) of F gene-deficient SeV vector (WO 00/70070) was digested with *Not*I. The mIL-10 gene *Not*I fragment was inserted into the *Not*I site to construct an F gene-deficient SeV cDNA carrying the IL-10 gene (pSeV18+mIL10/ΔF).

### [Example 2] Reconstitution and amplification of Sendai virus vector

Virus reconstitution and amplification were carried out according to the report of Li *et al.* (Li, H.-O. et al., J. Virology 74. 6564-6569 (2000), WO 00/70070) and a modified method thereof (WO 2005/071092). Helper cells for the F protein, in which expression of the F protein can be induced by the Cre/loxP expression induction system, were used for producing vectors. This system uses the pCALNdLw plasmid (Arai, T. et al., J. Virol. 72: 1115-1121 (1988)), which has been designed in a way that expression of gene products is induced by the Cre DNA recombinase. To express the inserted gene, a transformant with the plasmid was infected with a recombinant adenovirus expressing the Cre DNA recombinase (AxCANCre) by the method of Saito *et al.* (Saito, I. et al., Nucl. Acid. Res. 23, 3816-3821 (1995), Arai, T. et al., J. Virol. 72, 1115-1121 (1998)).

An F gene-deficient SeV vector (abbreviated as SeV18+mIL10/ΔF) carrying the mouse IL-10 gene (hereinafter abbreviated as mIL-10) was prepared by the method described above. The genomic RNA (minus strand) of SeV18+mIL10/ΔF is shown in SEQ ID NO: 16, and the antigenomic RNA (plus strand) is shown in SEQ ID NO: 17. Furthermore, the genomic RNA (minus strand) sequence of an F gene-deficient Sendai virus vector expressing IL-10 (hereinafter abbreviated as SeV18+mIL10/TSΔF), which has the G69E, T116A, and A183S temperature-sensitive mutations in the M protein, the A262T, G264, and K461 G temperature-sensitive mutations in the HN protein, the L511F mutation in the P protein, and the N1197S and K1795E mutations in the L protein, is shown in SEQ ID NO: 18. The antigenomic RNA (plus strand) sequence thereof is shown in SEQ ID NO: 19. As controls, an F gene-deficient SeV vector carrying the *E*. *coli* LacZ gene (abbreviated as SeV18+lacZ/ΔF) and an F gene-deficient SeV vector of temperature-sensitive mutation type (hereinafter abbreviated as SeV18+LacZ/TSΔF) were produced by the same method as described above.

### [Example 3] Therapeutic effects of nasal drop (nasal) administration of SeV-mIL10/ΔF in Alzheimer's disease model animal

### (3-1) Animal and administration method

The therapeutic and preventive effects of SeV18+mIL10/ΔF of the present invention on Alzheimer's disease can be assessed by using Alzheimer's disease model mice (hereinafter referred to as APP mice) such as APP transgenic mice (Tg2576; Hsiao K et al., Science, 1996, 274: 99-102). Mice were divided into two groups, each containing four mice; one was the treatment group and the other was the control group. The body weights of the APP transgenic mice used in this assessment were about 20 g. 5 x 10⁶ CIU of SeV18+mIL10/ΔF or 5 x 10⁶ CIU of SeV18+lacZ/ΔF was intranasally (nasally) administered to each animal in the treatment group or the control group, respectively. An example of the experiment using SeV18+mIL10/TSΔF and SeV18+LacZ/TSΔF is as follows. 16-month-old APP mice (Tg2576) (female) were divided into three groups, each containing ten mice. For one animal in each group, 5 x 10⁶ CIU/20 µl/head of SeV18+mIL10/TSΔF, 5 x 10⁷ CIU/20 µl/head of SeV18+mIL10/TSΔF, or 5 x 10⁷ CIU/20 µl/head of SeV 18+LacZ/TSΔF was intranasally administered. Before the administration and three days after the administration, blood was collected from the mice, and plasma was prepared. The plasma IL-10 level was determined using the mouse IL10 ELISA Kit Quantikine (R&D Systems) according to the appended protocol. The mouse plasma was diluted 50-fold with the dilution buffer attached to the kit. The plasma IL-10 level before the administration was lower than or comparable to the detection limit (4 pg/ml). The plasma IL-10 level three days after the administration is shown in Fig. 1. Plasma IL-10 was detected in a dosage-dependent manner, although the level varies among animals.

### (1) Senile plaque elimination effect

A Sendai virus vector was intranasally (nasally) administered to mice. The mice were dissected eight weeks after the administration. Brain tissue sections can be prepared from regions such as the cortex of frontal lobe, parietal lobe, and hippocampus. The experiment described below can be conducted using the cryosections. To detect the Aβ protein or senile plaques in the tissues, the sections were treated with 70% formic acid, and endogenous peroxidases were inactivated with 5% H₂O₂. After reaction with a rabbit anti-pan-Aβ antibody (1000-fold dilution), a peroxidase-labeled secondary antibody was added, and then DAB staining was performed. The area of Aβ accumulation in each region was measured, and then the ratio of the area of Aβ accumulation that occupies in each measured site was calculated.

Specifically, the effect of SeV18+mIL10/TSΔF was assessed using 12-month-old female APP transgenic mice (Tg2576) (Hsiao K et al., Science, 1996, 274: 99-102). Mice were divided into two groups, each containing 15 mice; one was the treatment group and the other was the control group. 5 x 10⁶ CIU of SeV18+mIL10/TSΔF or 5 x 10⁵ CIU of SeV 18+LacZ/TSΔF was intranasally (nasally) administered to a animal in the treatment group or the control group, respectively, under light anesthesia with sevoflurane. Four weeks after the treatment, blood was collected from five mice from the SeV18+mIL10/TSΔF group and four mice from the SeV18+LacZ/TSΔF group, and then the mice were dissected. Eight weeks after the treatment, blood was collected from ten mice from the SeV18+mIL10/TSΔF group and nine mice from the SeV18+LacZ/TSΔF group, and then the mice were dissected. The brain with the olfactory bulb was vertically divided into the right and left halves. One was rapidly frozen and stored for biochemical measurement, and the other was immersed and fixed in 4% paraformaldehyde solution for histopathological examination. Paraffin-embedded histopathological sections were prepared as vertical sections containing the olfactory bulb at 1 mm from the midline. The sections were stained with hematoxylin and eosin for standard histopathological examination, and observed under a microscope. Immunostaining with an anti-Aβ antibody (4G8) was performed to detect the Aβ protein and senile plaques in the tissues. Alternatively, Iba-1 immunostaining was performed to stain microglia and macrophages.

Samples stained with the anti-Aβ antibody were divided into the following four parts: the olfactory bulb, cerebral neocortex, hippocampus, and brain stem/cerebellum. The quantity of senile plaques and blood vessels positive for anti-Aβ antibody staining that were present in each region were evaluated under a light microscope. The area of senile plaques in the cerebral neocortex was quantified by image analysis software (NIH Image, Japanese Edition) using recorded images with the same magnification.

An example of the result of staining sections is shown in Fig. 2 (the parietal lobe of cerebral neocortex and hippocampus). Meanwhile, the area ratio of Aβ deposition is shown in Fig. 3. Since only a small number of senile plaques were formed in the hippocampus of both groups, there was no significant difference between the groups. In contrast, eight weeks after the treatment, the area of senile plaques in the cerebral neocortex was clearly reduced in the SeV18+mIL10/TSΔF group. The difference was statistically significant (p<0.01).

On the other hand, in the Iba-1 immunostaining samples, a clear difference in the number of activated microglia in the olfactory bulb was observed, although there was no clear difference observed in the cerebral neocortex, hippocampus, or brain stem/cerebellum. As shown in Fig. 4, the number of activated microglia was increased in the SeV18+mIL10/TSΔF group four and eight weeks after the treatment. According to the result of the analysis of recorded images with the same magnification, as shown in Fig. 5, the area ratio of Iba-d1-positive cells was statistically significantly increased in the SeV 18+mIL10/TSΔF group eight weeks after the treatment (p<0.01, Student t test). This suggests that, in the SeV18+mIL10/TSΔF group, activated microglia or macrophages actively eliminate foreign materials (the majority of them are dead olfactory cells infected with SeV) from the olfactory bulb. This also suggests that the mIL-10 protein expressed in nasal mucosa cells is transported along axons of olfactory cells in the opposite direction to the olfactory bulb, and the microglial activation is promoted in the olfactory bulb. Since the number of images analyzed for each group was small and different, statistical analysis was not performed. However, the number of microglia in the SeV18+mIL10/TSΔF group was clearly larger than that of the control group (SeV18+LacZ/TSΔF group) even just four weeks after the administration.

### (2) Assay of Aβ in brain tissues

Mouse cerebrum and cerebellum were cut along the fissura mediana. A hemisphere was rapidly frozen and stored at -80°C. The brain hemisphere was homogenized in 1 ml of TBS solution. The homogenate was centrifuged at 100,000 g in a bench-top ultracentrifuge for one hour. The soluble fraction (TBS fraction) was stored, while the insoluble fraction was dissolved in 2% SDS, homogenized, and then centrifuged at 100,000 g for one hour. The soluble fraction (2% SDS fraction) was stored, while the insoluble fraction was dissolved in 70% formic acid, homogenized, and then centrifuged at 100,000 g for one hour. The soluble fraction (formic acid fraction) was stored. An ELISA kit from Biosource was used to assay Aβ40 and 42 in brain tissues. The TBS fraction was diluted four-fold; the 2% SDS fraction was diluted 400- to 2000-fold; and the formic acid fraction was diluted 1000-fold in 1 M Tris solution. Then, the diluted fractions were further diluted (2- to 10-fold) with ELISA dilution buffer and assayed.

Specifically, 10-month-old APP mice (Tg2576) were divided into two groups, each containing ten mice. SeV18+mIL10/TSΔF or SeV18+LacZ/TSΔF was intranasally (nasally) administered to each group at 5 x 10⁶ CIU/20 µl/head per animal. Eight weeks after the administration, the brains were excised and the right hemispheres were used. Using Teflon^{®} homogenizer, each mouse brain was homogenized in five volumes of TBS (50 mM Tris-HCI (pH 7.6), 150 mM NaCl) containing a protease inhibitor cocktail (CALBIOCHEM). The homogenate was centrifuged at 100,000 g for one hour at 4°C. The supernatant was collected and stored at -80°C (TBS fraction). The precipitate was homogenized using a hand homogenizer in three volumes of 1% Triton X-100/TBS (containing protease inhibitors) to one volume of the mouse brain. The homogenate was incubated at 37°C for 15 minutes, and then centrifuged at 100,000 g for one hour at 4°C. The supernatant was collected and stored at -80°C (1% Triton fraction). Furthermore, the precipitate was homogenized using a hand homogenizer in three volumes of 2% SDS/TBS (containing protease inhibitors) to one volume of the mouse brain. The homogenate was incubated at 37°C for 15 minutes, and then centrifuged at 100,000 g for one hour at 25°C. The supernatant was collected and stored at -80°C (2% SDS fraction). Finally, using a hand homogenizer, the precipitate was homogenized in the same volume of 70% formic acid as the mouse brain. The homogenate was centrifuged at 100,000 g for one hour at 4°C. The supernatant was collected, and its pH was adjusted by adding ten volumes of 1 M Tris. The supernatant was then stored at -80°C (FA fraction).

Aβ in the brain tissues was assayed using the Human/Rat β Amyloid ELISA Kit WAKO (Wako Pure Chemical Industries). The result of the assay is shown in Fig. 6. Soluble Aβ40 (TBS fraction and 1% Triton fraction) and insoluble Aβ42 (FA fraction) were decreased in the SeV18+mIL10/TSΔF administration group as compared to the SeV18+LacZ/TSΔF administration group.

### [Example 4] Determination of IL-10 protein levels after administration (nasal drop (nasal) administration) of SeV18+mIL10/TSΔF

Blood IL-10 levels in normal mice after nasal administration of SeV18+mIL10/TSΔF were determined as described below. Eight-week-old C57BL/6N mice were divided into two groups, each containing six mice. SeV18+mIL10/TSΔF was intranasally (nasally) administered to each group at 5 x 10⁷ or 5 x 10⁸ CIU /20 µl/head per animal. Before the administration and three days after the administration, blood was collected from the mice, and plasma was prepared. The plasma IL-10 level was determined using the mouse IL10 ELISA Kit Quantikine (R&D Systems) according to the appended protocol. The mouse plasma was diluted 50-fold using the dilution buffer attached to the kit.

The plasma IL-10 level before administration was lower than or comparable to the detection limit (4 pg/ml). The plasma IL-10 level three days after the administration was increased in a dosage-dependent manner (Fig. 7).

### [Example 5] IL-10 protein distribution after administration (nasal drop (nasal) administration) of SeV18+mIL10/TSΔF

### (1) Kinetic measurement of blood IL-10 level

20 µl of the SeV18+mIL10/TSΔF vector suspended in DPBS(-) at 5 x 10⁷ CIU/20 µl or 5 x 10⁶ Civil/20 µl was nasally administered to six mice in each group of normal mice (C57BL/6N, female). Then, blood was collected from the eye pit using heparin-containing hematocrit tubes at 24-hour intervals up to day 7. After blood collection, plasma was separated using a hematocrit centrifuge and stored at -80°C.

As a control, 20 µl of SeV18+LacZ/TSΔF was nasally administered at 5 x 10⁷ CIU/20 µl to six mice. The mouse plasma IL-10 levels were determined by ELISA (R&D Systems, Catalog No. M1000), according to the manual instructions.

The result shows that AUC (area under the pharmacokinetic curve) was 176,000 pg•h/ml after a single intranasal administration of SeV18+mIL10/TSΔF (5 x 10⁷ CIU/head) (Fig. 8).

### (2) Transfer into the brain (concentration in brain tissues)

53 µl of Sev18+mIL10/TSΔF suspended in DPBS(-) at 5 x 10⁸ CIU/53 µl or the same concentration of SeV18+LacZ/TSΔF vector (as a control) was nasally administered to ten animals in each group of normal mice (C57BL/6N, female). As a non-administration control, 53 µl of DPBS(-) was nasally administered to ten mice. The mice were sacrificed three or seven days after administration of the above-described vectors. Hereinafter, samples derived from the tissues of the mice sacrificed on day three and day seven are referred to as "day-3 sample" and "day-7 sample", respectively. Five mice from each group were anesthetized with ether and underwent thoracotomy. After cutting the right auricle of heart, perfusion was performed for about eight minutes by injecting 30 ml of physiological saline into the left atrium using a 24G-needle syringe. After perfusion, the first cervical vertebra was cut, and then craniotomy was performed to collect the brain. After dividing the brain into two hemispheres, they were further divided into the following three parts: the olfactory bulb, cerebrum, and cerebellum/medulla oblongata, which were rapidly frozen in liquid nitrogen and then stored at -80°C. The five non-perfused mice were anesthetized with Sevofrane and underwent laparotomy. After exsanguination by cutting the caudal vena cava and ventral aorta, craniotomy was performed to collect and store the brains, as described above. From the non-perfused mice, the sites of administration: nasal septum, ethmoid bone, and nasal turbinates including nasal mucosa, were also collected and stored: Each sample was homogenized in the extraction buffer (1% Triton X-100, 50 mM Tris-HCl (pH 7.5), and Complete Protease Inhibitor Cocktail (Roche Diagnostics)) using the glass-Teflon^{™} homogenizer (750 rpm, 10 strokes) and then the Dremel homogenizer (30,000 rpm, 30 sec). Then, the homogenate was ultra-centrifuged at 100,000 g for one hour. The centrifuged supernatant was collected and used as extract. The extract was stored at -80°C. The mouse IL-10 level in the extract was determined by ELISA (R&D Systems, Catalog No. M1000), according to the manual instructions.

After administration of the above-described vectors, mIL-10 was detected in each tissue of the day-3 sample of the non-perfused group. The mIL-10 level detected in the brain extract of the administered mice was about 0.1 % of the expression level in the plasma and nasal mucosa (Fig. 9(B)). Furthermore, in the group where blood was removed by perfusion, IL-10 was also detected in the tissues including the brain (Fig. 10(B)). In the olfactory bulb, the detected mIL-10 level of the day-7 sample of the group where blood was removed by perfusion (Fig. 12(B)) was comparable with that of the day-7 sample of the non-perfused group (Fig. 11(B)).

Furthermore, after administration of the above-described vectors, the detected mIL-10 level in the olfactory bulb of the day-7 sample of the non-perfused group (Fig. 11 (B)) was comparable with that of the day-3 sample of the non-perfused group (Fig. 9(B)). In addition, the detected mIL-10 level in the olfactory bulb of the day-7 sample of the group where blood was removed by perfusion (Fig. 12(B)) was comparable with that of the day-3 sample of the group where blood was removed by perfusion (Fig. 10(B)).

Thus, it was demonstrated that mIL-10 expressed from the nasally-administered SeV vector was transferred into tissues of the central nervous system (Figs. 10(B) and 12(B)).

In particular, the expression level of mIL-10 in the olfactory bulb was confirmed to be constant in the presence or absence of perfusion (Figs. 10(B) and 12(B)). This suggests that mIL-10 expressed from the intranasally administered SeV vector is surely transferred into the olfactory bulb.

### (3) Transfer into the brain (concentration in CSF)

106 µl of SeV18+mIL10/TSΔF suspended in DPBS(-) at 1 x 10⁹ CIU/106 µl or the same concentration of the SeV18+LacZ/TSΔF vector (as a control) was nasally administered to five animals in each group of normal rats (Wistar, female). As a non-administration control, 106 µl of DPBS(-) was nasally administered to five rats. The rats were sacrificed three days after the administration. Under anesthesia, the skin and the muscles of the lateral cervical region were removed to expose the dura mater. A 200-µl pipette tip was inserted into the magna sterna to collect the cerebrospinal fluid (CSF). The CSF was rapidly frozen in liquid nitrogen, and stored at -80°C. The mouse IL-10 level in the CSF was determined by ELISA (R&D Systems, Catalog No. M1000), according to the manual instructions.

As a result, the mIL-10 concentration detected in the CSF of the administered rats was about one-tenth of that in the plasma (Fig. 13). There was a positive correlation between the mIL-10 concentration in the plasma and that in the rat CSF. This suggests that mIL-10 expressed from the nasally administered SeV vector is centrally transferred.

### [Example 6] Assessment of the efficacy of the IL-10 protein (subcutaneous administration)

### (1) Kinetic measurement of blood IL-10 level

100 µl of recombinant mouse IL-10 (Wako Pure Chemical Industries, Catalog No. M1000091-04691) suspended in DPBS(-) at 2.0 µg/100 µl, or 100 µl of DPBS(-) as a control was subcutaneously administered in the back to three animals in each group of normal mice (C57BL/6N). Blood was collected from the eye pit using heparin-containing hematocrit tubes one, two, three, six, nine, twelve, and 24 hours after the administration. After blood collection, plasma was separated using a hematocrit centrifuge and stored at -80°C. The mouse plasma IL-10 levels were determined by ELISA (R&D Systems, Catalog No. M1000), according to the manual instructions (Fig. 14). The result is as follows: Cₘₐₓ = about 12,000 pg/ml; Tₘₐₓ = about 1.5 hr; t_{1/2} = about 1 hr (initial value). AUC was 40,800 pg•h/ml.
The AUC ratio relative to that of the SeV18+mIL10/TSΔF administration (Fig. 8) was 4.3 (176,000/40,800). This suggests that repeating subcutaneous administration for about four times can result in an AUC equivalent to that achieved by nasal administration of SeV18+mIL10/TSΔF.

### (2) Determination of blood IL-10 levels in APP mice

IL-10 from recombinant mice (Wako Pure Chemical Industries, Code No. 091-04691; http://www.wako-chem.co.jp/siyaku/info/bai/article/cytokine.htm) was suspended in DPBS(-) at 2.0 µg/100 µl. 100 µl of the suspension (hereinafter referred to as "IL-10 suspension") or 100 µl of DPBS(-) as a control was subcutaneously administered in the back to eight animals in each group ofAPP mice (Tg2576, female, 13-month-old) every twelve hours over seven days (a total of 14 times). The body weights of the APP transgenic mice used in this assessment were about 20 to 30 g. Blood was collected from the eye pit using heparin-containing hematocrit tubes one hour after administering for the first (day 0), seventh (day 3), and thirteenth (day 6) time. After blood collection, plasma was separated using a hematocrit centrifuge and stored at -80°C. The mouse plasma IL-10 levels were determined by ELISA (R&D Systems, Catalog No. M1000), according to the manual instructions.

Subcutaneous injection of recombinant mouse IL-10 in the back resulted in a plasma IL-10 concentration of 5000 pg/ml to 8,000 pg/ml one hour after the administration (Fig. 15).

### (3) Senile plaque-eliminating effect in APP mice to which the IL-10 protein was administered

The IL-10 protein was subcutaneously administered to mice twice a day for seven consecutive days. The mice were dissected four or eight weeks after the administration. Brain tissue sections can be prepared from regions such as the cortex of frontal lobe, parietal lobe, and hippocampus. The experiment described below can be conducted using the cryosections. To detect the Aβ protein or senile plaques in the tissues, the sections were treated with 70% formic acid, and the endogenous peroxidases were inactivated with 5% H₂O₂. After reaction with a rabbit anti-pan-Aβ antibody (1000-fold dilution), a peroxidase-labeled secondary antibody was added and then DAB staining was performed. To evaluate the degree of senile plaques (Aβ-accumulated portions) in each region, the area of senile plaques was determined semi-quantitatively or by viewing tissue section images under a light microscope. The area ratio of senile plaques in each site tested was calculated.

Specifically, the effect of IL-10 suspension was assessed using 13-month-old female APP transgenic mice (Tg2576). The body weights of the APP transgenic mice used in this assessment were about 20 to 30 g. The mice were divided into two groups, each containing 15 mice; one was the treatment group and the other was the control group. 2 µg/100 µl of mouse IL-10 suspension, or 100 µl of DPBS(-) was subcutaneously administered to an animal in the treatment group or the control group, respectively, twice a day at twelve-hour intervals. Four weeks after the treatment was teriminated, blood was collected from eight mice from the IL-10 suspension-administered group and seven mice from the DPBS(-)-administered group, and then the mice were dissected. The brain with the olfactory bulb was vertically divided into the right and left halves. One was rapidly frozen and stored for biochemical measurement, and the other was embedded in an OTC compound and frozen in dry ice for histopathological examination. Histopathological samples were prepared as vertical sections containing the olfactory bulb at 1 mm from its midline. The cryosections were prepared, fixed with formalin for a short period, stained with hematoxylin and eosin for standard histopathological examination, and then observed under a microscope. Immunostaining with an anti-Aβ antibody (4G8) was performed to detect the Aβ protein and senile plaques in the tissues. Alternatively, Iba-1 immunostaining was performed to stain microglia and macrophages.

Samples stained with the anti-Aβ antibody were divided into the following four parts: the olfactory bulb, cerebral neocortex, hippocampus, and brain stem/cerebellum. The quantity of senile plaques and blood vessels positive for anti-Aβ antibody staining present in each region were evaluated under a light microscope. In general, senile plaque deposition is not observed in the brain stem/cerebellum region. Thus, senile plaques in the olfactory bulb, cerebral neocortex, and hippocampus observed under a light microscope were classified into three categories based on the size (large, middle, and small), and a score was assigned to each category (large: nine points; middle: three points; small: one point). The degree of senile plaques was semi-quantitatively assessed from the total score. Furthermore, the effect of IL-10 was statistically analyzed using the total score for each individual. The areas of senile plaques in the olfactory bulb, cerebral neocortex, and hippocampus were quantified by image analysis software (NIH Image, Japanese Edition) using recorded images with the same magnification.

An example of the stained sections is shown in Fig. 16 (parietal lobe of cerebral neocortex and hippocampus). The result of semi-quantitative determination of senile plaques is shown in Fig. 17, and the result of quantifying the area of senile plaques is shown in Fig. 18. In both groups, senile plaque formation was clearly observed in the olfactory bulb, cerebral neocortex, and hippocampus. The number of senile plaques in the olfactory bulb, cerebral neocortex, and hippocampus four weeks after the treatment is clearly reduced (about 50%) in the IL-10 suspension-administered group, as compared to the DPBS(-)-administered control group (Fig. 16). As a result of semi-quantitative determination, the difference was statistically significant (p<0.05) (Fig. 17). Furthermore, the result of quantifying the area of senile plaques demonstrated that the degree of senile plaques tended to decrease (about 50%) in the mIL-10 administration group as compared to the PBS control group (p=0.06) (Fig. 18). This suggests that the elimination of senile plaques or Aβ deposits in the brain by activated microglia or macrophages was enhanced in the IL-10 suspension-administered group.

SeV18+mILI0/TSΔF and SeV18+LacZ/TSΔF were used in the above Examples; however, the vectors of the present invention are not limited thereto. For example, effects equivalent to those described in the Examples can be expected when non-F gene-deficient SeV vectors, F gene-deficient but non-temperature-sensitive SeV vectors, or such, that carry an anti-inflammatory cytokine gene such as IL-10, are used.

### Industrial Applicability

The present invention provides therapeutic agents for Alzheimer's disease, which comprise an anti-inflammatory cytokine, or a vector expressing an anti-inflammatory cytokine, such as a negative-strand RNA viral vector encoding an anti-inflammatory cytokine, and gene therapy methods for Alzheimer's disease using the cytokine or vector. The methods of the present invention are novel therapeutic means that can be used to substitute for or in combination with other therapeutic methods for Alzheimer's disease.

## Claims

1. A pharmaceutical composition for treating or preventing Alzheimer's disease, wherein the composition comprises
a negative-strand RNA viral vector carrying a gene encoding an anti-inflammatory cytokine or a partial peptide thereof, or
an anti-inflammatory cytokine or a partial peptide thereof.

2. The composition of claim 1, wherein the composition comprises a negative-strand RNA viral vector carrying a gene encoding an anti-inflammatory cytokine or a partial peptide thereof.

3. The composition of claim 1, wherein the composition comprises an anti-inflammatory cytokine or a partial peptide thereof.

4. The composition of claim 1 or 2, wherein the negative-strand RNA viral vector is a paramyxovirus vector.

5. The composition of claim 1 or 2, wherein the negative-strand RNA viral vector is a Sendai virus vector.

6. The composition of any one of claims 1 to 5, wherein the anti-inflammatory cytokine is selected from the group consisting of interleukin-4, interleukin-10, interleukin-13, and partial peptides thereof.

7. The composition of any one of claims 1 to 6, wherein the composition is used for nasal administration.

8. A negative-strand RNA viral vector carrying a gene for an anti-inflammatory cytokine or a partial peptide thereof, wherein the vector is used for treating Alzheimer's disease or developing a therapeutic agent for Alzheimer's disease.

9. An anti-inflammatory cytokine protein, wherein the protein is used for treating Alzheimer's disease or developing a therapeutic agent for Alzheimer's disease.

10. The vector of claim 8, wherein the negative-strand RNA viral vector is a paramyxovirus vector.

11. The vector of claim 8, wherein the negative-strand RNA viral vector is a Sendai virus vector.

12. The vector of any one of claims 8, 10, and 11, wherein the anti-inflammatory cytokine is selected from the group consisting of interleukin-4, interleukin-10, interleukin-13, and partial peptides thereof.

13. A method for treating or preventing Alzheimer's disease, wherein the method comprises the step of administering
a negative-strand RNA viral vector carrying a gene encoding an anti-inflammatory cytokine or a partial peptide thereof, or
an anti-inflammatory cytokine or a partial peptide thereof.

14. The method of claim 13, wherein the administration is nasal administration.


SEQUENCE LISTING

<110>  DNAVEC CORPORATION

<120>  Therapeutic agents for Alzheimer's disease

<130>  D4-A0601Y1P

<140>  PCT/JP2007/061058
<141>  2007-05-31

<150>  JP 2006-151617
<151>  2006-05-31

<150>  JP 2007-110623
<151>  2007-04-19

<160>  19

<170>

<210>  1
<211>  10
<212>  RNA
<213>  Artificial

<220>
<223>  gene start (S) sequence

<400>  1
cwuuvwcccu                                                                10

<210>  2
<211>  10
<212>  RNA
<213>  Artificial

<220>
<223>  gene start (S) sequence

<400>  2
cuuugacccu                                                                10

<210>  3
<211>  10
<212>  RNA
<213>  Artificial

<220>
<223>  gene start (S) sequence

<400>  3
cauucacccu                                                                10

<210>  4
<211>  10
<212>  RNA
<213>  Artificial

<220>
<223>  gene start (S) sequence

<400>  4
cuuucacccu                                                                10

<210>  5
<211>  10
<212>  DNA
<213>  Artificial

<220>
<223>  gene start (S) sequence

<400>  5
agggtcaaag                                                                10

<210>  6
<211>  10
<212>  DNA
<213>  Artificial

<220>
<223>  gene start (S) sequence

<400>  6
agggtgaatg                                                                10

<210>  7
<211>  10
<212>  DNA
<213>  Artificial

<220>
<223>  gene start (S) sequence

<400>  7
agggtgaaag                                                                10

<210>  8

<211>  9
<212>  RNA
<213>  Artificial

<220>
<223>  gene end (E) sequence

<400>  8
uuuuucuua                                                                    9


<210>  9
<211>  9
<212>  DNA
<213>  Artificial

<220>
<223>  gene end (E) sequence

<400>  9
taagaaaaa                                                                    9


<210>  10
<211>  43
<212>  PRT
<213>  Homo sapiens

<400>  10
Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30


Gly Leu Met Val Gly Gly Val Val Ile Ala Thr
        35                  40


<210>  11
<211>  537
<212>  DNA
<213>  Mus musculus


<220>
<221>  CDS
<222>  (1)..(534)

<400>  11
atg cct ggc tca gca ctg cta tgc tgc ctg ctc tta ctg act ggc atg          48
Met Pro Gly Ser Ala Leu Leu Cys Cys Leu Leu Leu Leu Thr Gly Met

1                 5                   10                  15

agg atc agc agg ggc cag tac agc cgg gaa gac aat aac tgc acc cac          96
Arg Ile Ser Arg Gly Gln Tyr Ser Arg Glu Asp Asn Asn Cys Thr His
            20                  25                  30

ttc cca gtc ggc cag agc cac atg ctc cta gag ctg cgg act gcc ttc          144
Phe Pro Val Gly Gln Ser His Met Leu Leu Glu Leu Arg Thr Ala Phe
        35                  40                  45

agc cag gtg aag act ttc ttt caa aca aag gac cag ctg gac aac ata          192
Ser Gln Val Lys Thr Phe Phe Gln Thr Lys Asp Gln Leu Asp Asn Ile
    50                  55                  60

ctg cta acc gac tcc tta atg cag gac ttt aag ggt tac ttg ggt tgc          240
Leu Leu Thr Asp Ser Leu Met Gln Asp Phe Lys Gly Tyr Leu Gly Cys
65                  70                  75                  80

caa gcc tta tcg gaa atg atc cag ttt tac ctg gta gaa gtg atg ccc          288
Gln Ala Leu Ser Glu Met Ile Gln Phe Tyr Leu Val Glu Val Met Pro
                85                  90                  95

cag gca gag aag cat ggc cca gaa atc aag gag cat ttg aat tcc ctg          336
Gln Ala Glu Lys His Gly Pro Glu Ile Lys Glu His Leu Asn Ser Leu
            100                 105                 110

ggt gag aag ctg aag acc ctc agg atg cgg ctg agg cgc tgt cat cga          384
Gly Glu Lys Leu Lys Thr Leu Arg Met Arg Leu Arg Arg Cys His Arg
        115                 120                 125

ttt ctc ccc tgt gaa aat aag agc aag gca gtg gag cag gtg aag agt          432
Phe Leu Pro Cys Glu Asn Lys Ser Lys Ala Val Glu Gln Val Lys Ser
    130                 135                 140

gat ttt aat aag ctc caa gac caa ggt gtc tac aag gcc atg aat gaa          480
Asp Phe Asn Lys Leu Gln Asp Gln Gly Val Tyr Lys Ala Met Asn Glu
145                 150                 155                 160

ttt gac atc ttc atc aac tgc ata gaa gca tac atg atg atc aaa atg          528
Phe Asp Ile Phe Ile Asn Cys Ile Glu Ala Tyr Met Met Ile Lys Met
                165                 170                 175

aaa agc taa                                                              537
Lys Ser

<210>  12
<211>  178
<212>  PRT
<213>  Mus musculus

<400>  12

Met Pro Gly Ser Ala Leu Leu Cys Cys Leu Leu Leu Leu Thr Gly Met
1               5                   10                  15

Arg Ile Ser Arg Gly Gln Tyr Ser Arg Glu Asp Asn Asn Cys Thr His
            20                  25                  30

Phe Pro Val Gly Gln Ser His Met Leu Leu Glu Leu Arg Thr Ala Phe
        35                  40                  45

Ser Gln Val Lys Thr Phe Phe Gln Thr Lys Asp Gln Leu Asp Asn Ile
    50                  55                  60

Leu Leu Thr Asp Ser Leu Met Gln Asp Phe Lys Gly Tyr Leu Gly Cys
65                  70                  75                  80

Gln Ala Leu Ser Glu Met Ile Gln Phe Tyr Leu Val Glu Val Met Pro
                85                  90                  95

Gln Ala Glu Lys His Gly Pro Glu Ile Lys Glu His Leu Asn Ser Leu
            100                 105                 110

Gly Glu Lys Leu Lys Thr Leu Arg Met Arg Leu Arg Arg Cys His Arg
        115                 120                 125

Phe Leu Pro Cys Glu Asn Lys Ser Lys Ala Val Glu Gln Val Lys Ser
    130                 135                 140

Asp Phe Asn Lys Leu Gln Asp Gln Gly Val Tyr Lys Ala Met Asn Glu
145                 150                 155                 160

Phe Asp Ile Phe Ile Asn Cys Ile Glu Ala Tyr Met Met Ile Lys Met
                165                 170                 175

Lys Ser


<210>  13
<211>  51
<212>  DNA
<213>  Artificial

<220>
<223>  a PCR primer

<400>  13
acttgcggcc gccaaagttc aatgcctggc tcagcactgc tatgctgcct g                51


<210>  14
<211>  79
<212>  DNA
<213>  Artificial

<220>
<223>  a PCR primer

<400>  14
atccgcggcc gcgatgaact ttcaccctaa gtttttctta ctacggttag cttttcattt      60

tgatcatcat gtatgcttc                                                   79


<210>  15
<211>  596
<212>  DNA
<213>  Artificial

<220>
<223>  a Not I fragment encoding mouse IL-10

<400>  15
gcggccgcca aagttcaatg cctggctcag cactgctatg ctgcctgctc ttactgactg      60

gcatgaggat cagcaggggc cagtacagcc gggaagacaa taactgcacc cacttcccag     120

tcggccagag ccacatgctc ctagagctgc ggactgcctt cagccaggtg aagactttct     180

ttcaaacaaa ggaccagctg gacaacatac tgctaaccga ctccttaatg caggacttta     240

agggttactt gggttgccaa gccttatcgg aaatgatcca gttttacctg gtagaagtga     300

tgccccaggc agagaagcat ggcccagaaa tcaaggagca tttgaattcc ctgggtgaga     360

agctgaagac cctcaggatg cggctgaggc gctgtcatcg atttctcccc tgtgaaaata     420

agagcaaggc agtggagcag gtgaagagtg attttaataa gctccaagac caaggtgtct     480

acaaggccat gaatgaattt gacatcttca tcaactgcat agaagcatac atgatgatca     540

aaatgaaaag ctaaccgtag taagaaaaac ttagggtgaa agttcatcgc ggccgc         596


<210>  16
<211>  14172
<212>  RNA
<213>  Artificial

<220>
<223>  SeV18+mIL10/dF (genomic RNA)


<220>
<221>  CDS
<222>  (146)..(6829)

<220>
<221> CDS
<222> (6968)..(8692)

<220>
<221> CDS
<222> (8855)..(9898)

<220>
<221> CDS
<222> (10020)..(11723)

<220>
<221> CDS
<222> (11876)..(13447)

<220>
<221> CDS
<222> (13504)..(14037)

<400> 16
accagacaag aguuuaagag auauguaucc uuuuaaauuu ucuugucuuc uuguaaguuu 60

uucuuacuau ugucauaugg acaaguccaa gacuuccagg uaccgcggag cuucgaucgu 120

ucugcacgau agggacuaau uauuacgagc ugucauaugg cucgauauca ccuagugauc 180

caucaucaau cacggucgug uauucauuuu gccuggcccc gaacaucuug acugccccua 240

aaaucuucau caaaaucuuu auuucuuugg ugaggaaucu auacguuaua cuauguauaa 300

uauccucaaa ccugucuaau aaaguuuuug ugauaacccu cagguuccug auuucacggg 360

augauaauga aacuauuccc aauugaaguc uugcuucaaa cuucugguca gggaaugacc 420

caguuaccaa ucuuguggac auagauaaag auauccaaga uagcacaagu cuucuagaaa 480

uugucuucaa cuugccugaa ucucucacag gauacagguc auacuuacca guuaguuuaa 540

gccuuuuauc ugacucaguu auucuagccc auucgaagau uguauccuuc aaaacccugu 600

ugaaagcuau caugcaguug uguguaucag cuauguucau gguggacaag aaaucucugc 660

ucaauuuaua caaguuuggu ucaaagccaa acgucugcag ugcuugaugg uaaggucuaa 720

gcaucccuga ugaagagcuu ccuucucuca auucccgagu aacccauucg ugagccuuug 780

ccuucucuau uaagauccac uuuucuaucu ugaugcuauc uucuuuugac aaagggagga 840

gacuagcuaa cacugucuug cuguccucua uaaugucaga uuuggggugc cucgauagga 900

gauacaucuc uguggaagca ggguuagaug uuuuaagcac uauuagguua accucguccc 960

aguaucucag auauaggcug agcugccugg uccaauccgu gcccagccug ggagcaaucu 1020
ugcuuauaag cacaacgucu cgauccccca ccagauacgc gauccggauu acacuguaau 1080
gcucaugcag uacaacuuga ucauccuuau gaucuccucc cuccauguca caguggacua 1140
ggccuaucga gcuauucugu aauucauucc aaaucaaagc cucacacuca ucauucccaa 1200
uccaugucga gccaggauuc ccguugaaua acacuuuaac ucuuugaccc agacuaguaa 1260
cauuguuuaa uuucuuuccc acuagugcca ccucagcagg auauauauuu aacucucucu 1320
gcccauugac aucacaagag uauaccccug aguuauaaua guugaugcau gggccaagag 1380
uagcgucaua acaggaaagc auggccccag cuccuucccc uaaauauagc cuaucuuuau 1440
ccuugucaac uaaggggcuc aauagguagg uaaguucaag ugcuuucaag cagcuaguac 1500
uguugaugcc aaagagccuc agcuggugag auagauaucu uccaucgaau gguaacguca 1560
gaccaacacc ugaucugccu aucucaccuc uugugaucuc cuuguacccc cggagacgca 1620
guaccuguga cuuggauacu cucaaccccc aaaaaggggc ucuagucuga ugucccaaag 1680
gaauauucug uuguauuucu gccgcgauac caucuaacag ugcauuaucu gccucgggau 1740
cccaaucuuc uaagacuuca gggacuccua uaccucuugu ccuuaacacu uuuauagaug 1800
acuuccggau auaggucaaa guagauggga auacuuugau gacuaggcca gucaacugac 1860
uguaccucag uaccggguca ucaagaaaug ugaguuccag guaacucuuu agugacucga 1920
gccucucuag agaguucauu gauucuaauc uuggcccauc ccuagauauc ucugccaagc 1980
ugcauaggua ugcaagaugu cuugccaaga aagaggaccu ccucaugucg gccacaucug 2040
ggucauuguc acagauaaag aucucaagcg guacaccccc uugccaaucg ugcaugaaua 2100
gauccacaga auauucacag acagagaggg ccaagaguau cuuauccuga uuugagaggu 2160
uaggcccaua cacagguucc acgacaccug cauuccagaa ucguuugaag auuuuuggau 2220
gagauagagc auuagauaag acuuuuaaaa cugcguggga gguaucuuua agaauccgga 2280
cuacaugucc ccauauuucu ucccuuccuc ugauguuuaa gccguagagu gaguaugcaa 2340
acugauugac uagaauaccc ccgaacguug agcaaaauaa aggaacauca aucaccauaa 2400
acucaguaau caagcuguug acaucaucgu cauuuacuag ugcgaucauc ucuuuuaagu 2460
ugucucuauc uaauugagac auuguaucag cuaucgucau ugcaguacag auacugguug 2520
cucugauaac uucaucaucu gaccaauaag ucaugucaac uguguguaca acaucucuga 2580

ccuugcuaaa uagcucaagg uccacauccu ugaguggauc aggaucauag aucaauuuau 2640
uguucucuug uguaaucucu aaaucuaaug uggaccuugg ggggauauuc gccuccugug 2700
gggacuccau uauacagcac ccguuauuaa gauguaagug caauaucagu ggcuucccua 2760
aggaaccuuu cuuauaucuc auauugaacu cgaacaagcu uagcccaguu agcauaaucu 2820
gcugauacac gagauuagua uccuucgacu ccccugcuuc uuugagugcc auguuaucau 2880
uugauauugu uaugaaccga cuugcacgga cuaguguugc acuagagaac uucaucuggg 2940
uugccguauc uuucaaccua ugagauagau uaguggaggu ugaaacagga gucagcagcu 3000
uuagauucuc uaagcucaga uuagcucuug uuugggcuau aagagcggcu uccauccacg 3060
auaucucauc agucccguag gcccacguau acaccauagc uauccggaug gccgccuuug 3120
cggguuugcu uagauuucuu acauacccga guugggcuuc cgaccuuuca ucaguggcug 3180
auccaaaaua ggggauucuu auagccggac auccguuugu aagcgugucc aggucuauau 3240
ugucaggaag auagaaccau guauagaugg ggucugcucc uucagaccug caaagcuugc 3300
acaccucuga accuucgaua aauauucccc ucaagagcuc uaaagggucu gguguuucua 3360
gcccauguau gggucucccg uaagucaggu ggauccacau uuucugccuu agaccgacag 3420
cuagcucaac ugaacacaua uacucauauu cgauguuguc uuucaccggu uuccugagag 3480
uucuagucag ugucucguac ugcaauagau cauaauugac aagccuccuc aauaucccau 3540
augauaaucc uccuuuccua acgcuggcuc ucacuagaga cuuggucgua ucaagcaucc 3600
cugcaaucgc cucccuaacu ccaguuaagg aauuacccag gaucucauga gccacucucg 3660
gcaggaugac uuuccggucc auaaggaacg aggccagguu gagauccucu ucuccacuag 3720
ucucggugaa gagaccagac aguagaggau ucggggauuc cugcagcaca gaucuagcag 3780
ugauauucuu uauaaucgua guuauacucu gagaaugcgg gagguuacac gaauaagggu 3840
cugaagccca aucuagaaaa cuagagucac cggguucuug auucaugacc cuguauaaua 3900
ccugcuuguc uaacagaucc gcucugauga aucuuuugag aucagcuagg gcugcuacug 3960
cggggucucc aauauuucua acaaagcauc uagauguaga cauguaguug aauccuccaa 4020
cauuugcugg aaucaacacu gcacaucuca gccaauucuu acccuuaaag uauugaucuc 4080
uuacggucgg gcugauaguu ggauuuauag ucaucccuag ugauaugcac accugcugac 4140

aggucuuaua caacgcaaug caguagccua guauaggaga auacccauuu ucgauagcuu 4200
uugcuaugga uguugagaug uucgaacaag cagaucuguu uucaucuacc agugucucgg 4260
accagaauac acacuugguc aaggcuuuca ggcacugugg uaaaaucuuc ccaucauagu 4320
auauccuuuu acuauagaca aacaucuugc uacuaaugau ggucucguuc aauuuuagcu 4380
cgugcccuac aucaaacaug acgugucuua gagcaccgaa auauuuggug aucuccucau 4440
agacaugauu uuucuucugc uuguaagucu gagcuacagg uacucuugau gucacggcua 4500
uagcuugauu gucacccuga accauugcag agacccugac acccacucuc acagcugcua 4560
gguggauugc acugauugag auuaagaucc acagcuucug gcaguaaccu ucuaugcccc 4620
cccuaggauu auguaugaaa augccagagu cugcaugauc cuggaguugu cgaugcaucc 4680
ggucggcgac uggacaguaa ggaucuccaa cauauauugu acaccuuuca aggacuggau 4740
gcauccaguu aaagaagguc uugaagccaa auaucucguu gcaucucuga ccaaacaaug 4800
caguacucuc aaaucuccag uuuaagcagu auuucuugag gucuguugug aggaagcaac 4860
uuaacguuuc auagccgucu guugaugaau cuguugccuu gaauucaugu cuggaccucu 4920
ucuuuucguc ccaguacccc ccagaguucu uauuuuccau gccuucguuu cucuucucug 4980
augauuuaga guuauuguac acugaaucag uccuggggac gccugagaca gaaagaguag 5040
ucaaucuuuu aaguaggucu aucucuccuu uaaccauccc auuuucgcug aauagcucuc 5100
cuauuccuuu agccaguagu gucucugcca gcaccuguac ggcucgcauc uuauaaguca 5160
uuuuugcgaa uagacgaccc ucuugcuuga ucucuuucuc uuugagacug uacgagaugu 5220
ugaacuccuc gucuuucaac caaucuccug acuccacaua auugauaauu ucuucugggu 5280
ugaaauucuc aucauuuaug aacacuucaa uaagccgccg ggucucuuca gacucugggg 5340
cuuuauagua cagauuacua uccggguaua cagaguccca ugccuccuuc cugggggaua 5400
gugcuuuguc uuucauauau auugugagau cuucaucuag uugugguucu auaaacuucc 5460
gaaacuugaa gccuaugaaa cuuguauagu ugucuacagc acauucauaa gagauugccg 5520
uauuggaccc uugagcguuc cuuaguucua gacacacgug aucagggaag ucacaggggg 5580
gccacugucc gccaugccuc ucucuauacc cauuuaugau gauagugcaa aaaacugcau 5640
gacacucgua uagggucuua agcuuuauug ccuuuugugc auacauaugg gcccuuaccu 5700
ugucggcggc agugacagcc ucuaagcugg gguggccaaa uguccuaaag aaggaaaaga 5760

ucucugcuuu cucaucaaua gagguuccau ggaaaauggc gaguaacgac uccacaauag 5820
ugucugcuuc agcaucugug uacacgucuc uacuuguuaa aacagucugu agcucuguca 5880
acacaugccu cauaaaugcc ccacguagag guauaacagg aucauuuagu uguaugagag 5940
caagugauag gggcuccaau agugcgauga cauuguauau uuccucucca agacuugaga 6000
agagggaauc cacuaguucc cauaauuccu caccuuugcu uguuauccca auggacuucu 6060
uaucuagaug cccugcagca gacauauucc accuuccuuc uacaacauca caauacauca 6120
agaccagcuc agggguuagg auauacccug ucaaugucaa cuuguucagu aucaugacaa 6180
gaucuccgua uguuacuaga guguaugauu ugcauucuag gagguuaugu gaauuagagg 6240
uaucgagggg uccccccggu cuggucuucu gcauccaccg caugucauau uugaugcuga 6300
accaaguuag gaauggccua uaccaucuau uccugcugua cuuaucaguu aucuccggga 6360
uggugccgau auccugcaac ggaucguacc ccucucuucc uucuauauug ccuaguugcu 6420
ugaagauauu caaccaaaga uccuggaacc cacuagauaa cucccugguc agccgauccg 6480
agaccgcgaa gacggaucgg auuuugucac auaucucugg uauaucaagc cuaaguaauu 6540
ccugagagua gguuggguac gguucaaacg uguaucgguc uaaauccuuu auugugcguu 6600
gaagagccuu acccagagac cugaucuuaa uuugacgggg ggacaauccu ccguuccuaa 6660
uuuugugcuu uguaauauuu auuaugcugu cguccuucag ucuguagggc ugguucacau 6720
cuaacaagac gugcaacugu gcuaucuucc cccugacuau gggagaguuc agguggcauu 6780
cuggauagag uaugucagaa ggguuuuggg aggacuccug cccauccaug accuauggca 6840
agcuucccau ucacccuggg uuuuucuuaa uacugugaga gacguaagag acugagagau 6900
auuguggaug uucggagaug acucuagugu cagcaaggcc gacaagccug cuagucaguu 6960
aaauuuaaga cucggccuug cauaauuuag ggaugcuagu cuuaaagagc aucggcugua 7020
agguauucag gcucuucuga uugaucucga ugaugugaaa gcaguagccu uuaccaaaau 7080
gcgugauaca cgaugucgug guauaugcag ccucuaauug aacauccuuu auccuuaaca 7140
uauuuauaau guuaguagug uuagaauaca ugauuguugg guugacacgc gauguauugg 7200
cauauagcgu gacgguagcg acguuagcug caucagggga caauggauaa gcaucagugu 7260
auacgccuga uaugcauucc uucggacacu uauuguacca auugcacucu uuauuuccug 7320

gucuagacaa ggcuucauga gguguccagu ugauagucaa aggguggcug acaucaagua 7380
cuccuaucug caguugagag ugccagccug augaucuugu auagauguac acccgaucac 7440
ccaauuuuaa uaaucuaccu uccgccccga gauaguuuug agugauugga augguuguga 7500
cucuuaucuu uggccucucu gagagauagu cauugaccug gaugaucacg cugaccaccu 7560
guuucccucc uagccaugua auuuucagag ccucauugca ugugucuugc gacaccuguu 7620
ggcauccuug gguccuacau uuuguaucac ccugcagagg ggugguuagu ccaccauacc 7680
caagaaauau caaugagccu ucuguugcaa ugccguugcc uacacugggg uauagugcag 7740
agaacgggug aucaagaucu accucgcugu ugcgauaccg gugagacuua guucucccuu 7800
ugagauccag gacaucaagg accagauccu caauaccauc acuagaguag ucgguucuuu 7860
cgucuacagu cggcauggag caaagcugau aaccccuagu cccgguugcc accacagagc 7920
augauuuccg auugucguug augucauaag ugugggacac uacgggguua agaucaggga 7980
acauaucuga auugagugau auguacccua gcugcaggac cugauaugau uucccuaugu 8040
cagcacaacc uuguguaaug agauuugaug aauaggcaua gauugccucg ccaauugaga 8100
gugaagggag ccuaacacau ccagagaucg uuguagaacc agauaacaag cucggaccag 8160
gcagcaauga gauuucagga ucugagcuaa gauacgguuc uccgacaggg caucuccaga 8220
aacuaugugg cucaaguggg gcaauuccau cggcauggug gacugcgauc guacucucac 8280
agugcugagu gagcucuugu cugcugcacg acuuaucaau caucuggaug acaucccugc 8340
uguuuuuguu caacaagacu gggauuccgg uuugcacaga gcucugaaug uugacagccc 8400
uugcuauaac cucuugccuu auuagacugg uaagugacuc uuucaccucc cugcugcuca 8460
uguucaaugc cucuacaguc auugaguacu cuuucauacu auacccuugu cuagcagaaa 8520
uuaugauaca gaugaucacu guggcaauug acaaagccca cugggugaau gagagaauca 8580
gcaaccaugu gucggcuuua cuugaccucu cccaaccuga ugcugguuuu guggugcuac 8640
cacuaggaga aguagaccag uacgagucac guuugccccu aucaccaucc augaucugug 8700
cuuguaccag guggagaguu uugcaaccaa gcacucacaa gggacuuuau cccuaaguuu 8760
uucuuauuua agacaaggag ugacccggug gcuccgacug ccagguggug ucugcuuggg 8820
gcauugucgc aggucucuga ugggugcaca uuuacagcuu ucugauccuu ccgauguucu 8880
uagccacaac auuaggguag uagcggaaau cacgagggau ggccgguugg aacaccgcau 8940

cgacgccugu gauuucuaca gaugccgccc aaaucaccau guucauaugg ggauucacau 9000
ccauuaaugg gaagcagacu gcccucuucc augcgagcug acucaugaau gucuuagaua 9060
gugucccauu aaccugaaca uggaagcuua uaccgccgau uaacccaagu gagaaaauca 9120
gccgcauucu cucaaucuug cucuugcagu acucaacaga guauaucuuc ccgaccuuuc 9180
uccugaucaa cccgaggugc accauaaaau ugagcuuuuu cuccccuuga ucaucaagua 9240
cugggaguac ccccuuuugu ucuguggaga ucccggucuu gagugucacc aguaaauuaa 9300
cagauauaga guugggcaau gcaaggucug caagggucuu ugggaucuug gcuaugguga 9360
uugccccuag agauguccca uugacaaaca ccacucugag ucuuaugucc uuguccacag 9420
ggaggcauug gggagcuagu gcgaccuugu uugcauuaaa uaucauuccc ugucucagcc 9480
ugccugacca ugguaggagu ggagcaccaa ucgaauccac cauguauacg aucaucucuc 9540
cugcucgaac aguccuccuc accguaauuc ugagaucggu gcaggccuuu aagaguuccu 9600
gaucaguccc guaguauuug gccacaccua uggguaacga cccggagccg cauauugagu 9660
agcuggucgg cucugucaag ucagauacgc ucccuagauu gguuguuugu uucggugucu 9720
caaagaaacc caagagcaau aaaucuaggu aucucacucc auguuuagga gggucuccua 9780
ccuugacaau ccugaugugg gggauggcuu ucuuauccgg accaguucuc agaggcaggg 9840
gcuccacagu accguuaucc ucauaugaga acuuagggaa ucuauagaua ucugccauug 9900
cgccguguua ggugaaauuu cuuucacccu aaguuuuucu uaaucuuuac uggcugaugc 9960
ugaugguaga uuggaucucu cuaugacuga ggaugguagg augccucacc cgggaucuag 10020
uuggucagug acucuauguc cucuucuacg aguuccauga cugccuuaac cucuuggucu 10080
gucuugcacu uggauaauga uuucacauau gcuacuuucu cagcucugcu uaggggacug 10140
cucucuauga cgagccugag agagugcauu gugggcuucu cuuuggaggg gaggagacgu 10200
gaugcguuug aggcccuggg uucugugucc cucucuuggu acaccggguu gcggaucuca 10260
ucucuaaauu cauccucucg gauuaggucc gguuuguacu ucauaucuuc uaggucuccc 10320
auagaugggu caaaccuggu agccuuaguc uuguucucuu uugauuuugc aaaaacggag 10380
ggggaccuug uaagggaguc uguguuguca gucuugccac cucuaucugu gaugauauga 10440
aguguagaua gguuggacau cagcaaugag uucuguucuu ucugauacuc agagaaucuc 10500

uuguaaauau cccggaauga uuccacgcuc ucuuggaucu guuugagcag uuguuuguuc 10560
ucaucuaccu uacgagcgga agauuucucg gcagaaagga ucaggccgca uacauugaau 10620
gucaucucug cauaguuugc agacuuuagg gcacgucuug caaacacaua acucgcgucu 10680
cgggaugauu cgaauucuug agcagacugg auuacaccaa gacucgucaa caauguagcc 10740
aucucuuuca uagaugaugu guucucuccu augcccuuuu uuguugaguc ggucucuaga 10800
cccgggugga uugggcggcc guuggcugga caaucugaga cagagcgggu cccuauuggu 10860
gguuuccggu cuuugacccu gacggcgggg guguccccag aguugaugug cucauccugu 10920
guagaugggg guuuuccugg uggugacccu gugcuguugu aacgauucag cgguggggac 10980
cgggugccag gcacgguugc uggaguaaga gguuuggacc cacuguuggu aggucuucuu 11040
uuguuccucc guagcacagc cucuucaagu ucggggcuag gaaucaccag gaccccaguu 11100
acucuugcac uaugugagcu gccaggcucc augcuucuuc cauuauuacu ugcuccaccu 11160
ucuccuucau cagguaggga uguacuuccu cguaccucuu cuggaagucc uucagcuugg 11220
ucuucucccc ucuuaucagg gugcgcagcc aucucucugu uuucaucuuc aauaccugau 11280
cucggauauc cucucucauu uggaggauuu ucaaggauuc cggagucucc uccaucgccc 11340
agauccugag auacagaguu uguaccaguu cuucccccaa aggcccggug uauauuuugu 11400
uuaucaaggu uuccagcaug ugcuucugcc ucuggcuugc uuguucuccc agagacucua 11460
cucuccucac cugaucgauu aucuuggguc gacgguguug agacuucucc uucgcccuca 11520
cuuuuggcuc uaugagcaga gccugguccu uggggagugu ugaugguguu guggagccag 11580
cuucuguccc cuccgauguc aguugguuca cucgacagga cagcaucgag gaauccgaua 11640
acauccgaga gcgacucucg uccuccuggc gccucccucu caacuucaga aucuucuuua 11700
agaaugaagg caucuugauc caugcgguaa guguagccga agccguggcu gucucgacug 11760
cugggugugg ucgguggggu uggguguggc cuugccugag ccgaucggug gaugaacuuu 11820
cacccuaagu uuuucuuacu acggaucaag uaccuugaag ccucguauga uccuagauuc 11880
cucccuaucc agcuacugcu gcggcaucgu caucuucguc augaucgaca ccguuauugc 11940
ggccuucauc uccauggguu gcagaauccu cuugccgucu cucugcgagu cucauggcua 12000
uucuucucuc uaugucugau acauccucau cauugguuuc cuccucuaac cguucagccc 12060
cauguagugu gacaaagugg ccaccacuca ccugacgugc ccaucuuuca ccacuaucuc 12120

caccccaacc ccuagcgucc ugguccgcau gggcuuuugu uucuaggucg augucggcau 12180
ugucuagagc uaccucaauu gcaccaccgc cuguugguuu gugguaagca ccauccccac 12240
cggacaaguu ugccagauga ugucugagcc uccccuuggc uguauccguc acuccuaacu 12300
caucuuccaa ggcacugcug aucuucgauu cagcauccuu ugccacggcu uguccuagua 12360
agaacauuuc cauaucaagg uauguccucc cugugacgua cugcugcauu gccuuguucu 12420
guacgacggc gacucccaug gcguaacucc auagugcagg auaauugccu ggagcaaauu 12480
caccaugaac aggguccuug aggauacaga uaaagggagc ucuggggccu uuugacaggu 12540
aggugucuau gaggcuucua agcuuauuaa uaucgggccu cagguuugac aacguuagag 12600
cugccaucuu uguuuccacc ccauauuuaa uaguguucau gaaggaagcc agcccugcau 12660
cucggaugua guucccaacg aucuggaugu ucuucucuaa uguggugaga ucagaucuug 12720
caguauucau agucacaagg gucucaacca ugagagauac aaggcuuugc ugagaucuca 12780
uaaccgagcc uauccccuca acugucuccc cagugaaaac uaaggcaccu uucacggugc 12840
cgucuugucu gaacgccucu aaccuguuga agaacccuuu ccuuaagccg gcgcugcuug 12900
ugauggccuu caccagcaca auccagacuu ggacaauuau ugcuccuagg caugcaggau 12960
acccauagau uugaaggagu gugucagggu cugcagcauc ccguugaccc uggaagagug 13020
ggcucuuguu gaccauaggu ccaaacagcc auucuguggu ccucucauau uccauaucuc 13080
ucgucuucac aaugaauccg ucugucuucg uccucuuagg gucuuucucu auguuguaga 13140
ucacauauuu gacaucggcg uuuacuccgu uuguugucaa guacaauucu ggacuacugu 13200
aagccauggc aagcagagag acgaggaacc ccccucucug agagugcugc uuaucugugu 13260
ccaaugagug agcuaggaag guaguugcaa ugaauaacuu gucugcauca ucagucacac 13320
uugggccuag uacgaacacu gagacugugc uccucuggcc ggggauaaca gcaccuccuc 13380
ccgacuuauu aauacuuucg cuccuccuag agcuaaaugu aucgaaggug cucaacaacc 13440
cggccaucgu gaagaucugc ggccgcgaug aacuuucacc cuaaguuuuu cuuacuacgg 13500
uuagcuuuuc auuuugauca ucauguaugc uucuaugcag uugaugaaga ugucaaauuc 13560
auucauggcc uuguagacac cuuggucuug gagcuuauua aaaucacucu ucaccugcuc 13620
cacugccuug cucuuauuuu cacaggggag aaaucgauga cagcgccuca gccgcauccu 13680

gagggucuuc agcuucucac ccagggaauu caaaugcucc uugauuucug ggccaugcuu 13740

cucugccugg ggcaucacuu cuaccaggua aaacuggauc auuuccgaua aggcuuggca 13800

acccaaguaa cccuuaaagu ccugcauuaa ggagucgguu agcaguaugu uguccagcug 13860

guccuuuguu ugaaagaaag ucuucaccug gcugaaggca guccgcagcu cuaggagcau 13920

guggcucugg ccgacuggga agugggugca guuauugucu ucccggcugu acuggccccu 13980

gcugauccuc augccaguca guaagagcag gcagcauagc agugcugagc caggcauuga 14040

acuuuggcgg ccgcgugaac uuuggcagca aagcaaaggg ucuggaaccu gcuccucagg 14100

guggauacuu ugacccuaaa auccuguaua acuucauuac auaucccaua cauguuuuuu 14160

cucuuguuug gu 14172

<210> 17
<211> 14172
<212> RNA
<213> Artificial

<220>
<223> SeV18+mIL10/dF (antigenomic RNA)

<220>
<221> CDS
<222> (136)..(669)

<220>
<221> CDS
<222> (726)..(2297)

<220>
<221> CDS
<222> (2450)..(4153)

<220>
<221> CDS
<222> (4275)..(5318)

<220>
<221> CDS
<222> (5481)..(7205)

<220>
<221> CDS
<222> (7344)..(14027)

<400> 17

accaaacaag agaaaaaaca uguaugggau auguaaugaa guuauacagg auuuuagggu 60
caaaguaucc acccugagga gcagguucca gacccuuugc uuugcugcca aaguucacgc 120
ggccgccaaa guucaaugcc uggcucagca cugcuaugcu gccugcucuu acugacuggc 180
augaggauca gcaggggcca guacagccgg gaagacaaua acugcaccca cuucccaguc 240
ggccagagcc acaugcuccu agagcugcgg acugccuuca gccaggugaa gacuuucuuu 300
caaacaaagg accagcugga caacauacug cuaaccgacu ccuuaaugca ggacuuuaag 360
gguuacuugg guugccaagc cuuaucggaa augauccagu uuuaccuggu agaagugaug 420
ccccaggcag agaagcaugg cccagaaauc aaggagcauu ugaauucccu gggugagaag 480
cugaagaccc ucaggaugcg gcugaggcgc ugucaucgau uucuccccug ugaaaauaag 540
agcaaggcag uggagcaggu gaagagugau uuuaauaagc uccaagacca aggugucuac 600
aaggccauga augaauuuga caucuucauc aacugcauag aagcauacau gaugaucaaa 660
augaaaagcu aaccguagua agaaaaacuu agggugaaag uucaucgcgg ccgcagaucu 720
ucacgauggc cggguuguug agcaccuucg auacauuuag cucuaggagg agcgaaagua 780
uuaauaaguc gggaggaggu gcuguuaucc ccggccagag gagcacaguc ucaguguucg 840
uacuaggccc aagugugacu gaugaugcag acaaguuauu cauugcaacu accuuccuag 900
cucacucauu ggacacagau aagcagcacu cucagagagg gggguuccuc gucucucugc 960
uugccauggc uuacaguagu ccagaauugu acuugacaac aaacggagua aacgccgaug 1020
ucaaauaugu gaucuacaac auagagaaag acccuaagag gacgaagaca gacggauuca 1080
uugugaagac gagagauaug gaauaugaga ggaccacaga auggcuguuu ggaccuaugg 1140
ucaacaagag cccacucuuc cagggucaac gggaugcugc agacccugac acacuccuuc 1200
aaaucuaugg guauccugca ugccuaggag caauaauugu ccaagucugg auugugcugg 1260
ugaaggccau cacaagcagc gccggcuuaa ggaaaggguu cuucaacagg uuagaggcgu 1320
ucagacaaga cggcaccgug aaaggugccu uaguuuucac uggggagaca guugagggga 1380
uaggcucggu uaugagaucu cagcaaagcc uuguaucucu caugguugag acccuuguga 1440
cuaugaauac ugcaagaucu gaucucacca cauuagagaa gaacauccag aucguuggga 1500
acuacauccg agaugcaggg cuggcuuccu ucaugaacac uauuaaauau gggguggaaa 1560
caaagauggc agcucuaacg uugucaaacc ugaggcccga uauuaauaag cuuagaagcc 1620

ucauagacac cuaccuguca aaaggcccca gagcucccuu uaucuguauc cucaaggacc 1680
cuguucaugg ugaauuugcu ccaggcaauu auccugcacu auggaguuac gccaugggag 1740
ucgccgucgu acagaacaag gcaaugcagc aguacgucac agggaggaca uaccuugaua 1800
uggaaauguu cuuacuagga caagccgugg caaaggaugc ugaaucgaag aucagcagug 1860
ccuuggaaga ugaguuagga gugacggaua cagccaaggg gaggcucaga caucaucugg 1920
caaacuuguc cgguggggau ggugcuuacc acaaaccaac aggcgguggu gcaauugagg 1980
uagcucuaga caaugccgac aucgaccuag aaacaaaagc ccaugcggac caggacgcua 2040
gggguugggg uggagauagu ggugaaagau gggcacguca ggugaguggu ggccacuuug 2100
ucacacuaca uggggcugaa cgguuagagg aggaaaccaa ugaugaggau guaucagaca 2160
uagagagaag aauagccaug agacucgcag agagacggca agaggauucu gcaacccaug 2220
gagaugaagg ccgcaauaac ggugucgauc augacgaaga ugacgaugcc gcagcaguag 2280
cugggauagg aggaaucuag gaucauacga ggcuucaagg uacuugaucc guaguaagaa 2340
aaacuuaggg ugaaaguuca uccaccgauc ggcucaggca aggccacacc caaccccacc 2400
gaccacaccc agcagucgag acagccacgg cuucggcuac acuuaccgca uggaucaaga 2460
ugccuucauu cuuaaagaag auucugaagu ugagagggag gcgccaggag gacgagaguc 2520
gcucucggau guuaucggau uccucgaugc uguccugucg agugaaccaa cugacaucgg 2580
aggggacaga agcuggcucc acaacaccau caacacuccc caaggaccag gcucugcuca 2640
uagagccaaa agugagggcg aaggagaagu cucaacaccg ucgacccaag auaaucgauc 2700
aggugaggag aguagagucu cugggagaac aagcaagcca gaggcagaag cacaugcugg 2760
aaaccuugau aaacaaaaua uacaccgggc cuuuggggga agaacuggua caaacucugu 2820
aucucaggau cugggcgaug gaggagacuc cggaauccuu gaaaauccuc caaaugagag 2880
aggauauccg agaucaggua uugaagauga aaacagagag auggcugcgc acccugauaa 2940
gaggggagaa gaccaagcug aaggacuucc agaagaggua cgaggaagua caucccuacc 3000
ugaugaagga gaagguggag caaguaauaa uggaagaagc auggagccug gcagcucaca 3060
uagugcaaga guaacugggg uccuggugau uccuagcccc gaacuugaag aggcugugcu 3120
acggaggaac aaaagaagac cuaccaacag uggguccaaa ccucuuacuc cagcaaccgu 3180

gccuggcacc cgguccccac cgcugaaucg uuacaacagc acagggucac caccaggaaa 3240
acccccaucu acacaggaug agcacaucaa cucuggggac acccccgccg ucagggucaa 3300
agaccggaaa ccaccaauag ggacccgcuc ugucucagau uguccagcca acggccgccc 3360
aauccacccg ggucuagaga ccgacucaac aaaaaagggc auaggagaga acacaucauc 3420
uaugaaagag auggcuacau uguugacgag ucuuggugua auccagucug cucaagaauu 3480
cgaaucaucc cgagacgcga guuauguguu ugcaagacgu gcccuaaagu cugcaaacua 3540
ugcagagaug acauucaaug uaugcggccu gauccuuucu gccgagaaau cuuccgcucg 3600
uaagguagau gagaacaaac aacugcucaa acagauccaa gagagcgugg aaucauuccg 3660
ggauauuuac aagagauucu cugaguauca gaaagaacag aacucauugc ugauguccaa 3720
ccuaucuaca cuucauauca ucacagauag agguggcaag acugacaaca cagacucccu 3780
uacaaggucc cccuccguuu uugcaaaauc aaaagagaac aagacuaagg cuaccagguu 3840
ugacccaucu auggagaccc uagaagauau gaaguacaaa ccggaccuaa uccgagagga 3900
ugaauuuaga gaugagaucc gcaacccggu guaccaagag agggacacag aacccagggc 3960
cucaaacgca ucacgucucc uccccuccaa agagaagccc acaaugcacu cucucaggcu 4020
cgucauagag agcagucccc uaagcagagc ugagaaagua gcauauguga aaucauuauc 4080
caagugcaag acagaccaag agguuaaggc agucauggaa cucguagaag aggacauaga 4140
gucacugacc aacuagaucc cgggugaggc auccuaccau ccucagucau agagagaucc 4200
aaucuaccau cagcaucagc caguaaagau uaagaaaaac uuaggugaaa agaaauuuca 4260
ccuaacacgg cgcaauggca gauaucuaua gauucccuaa guucucauau gaggauaacg 4320
guacugugga gccccugccu cugagaacug guccggauaa gaaagccauc ccccacauca 4380
ggauugucaa gguaggagac ccuccuaaac auggagugag auaccuagau uuauugcucu 4440
uggguuucuu ugagacaccg aaacaaacaa ccaaucuagg gagcguaucu gacuugacag 4500
agccgaccag cuacucaaua ugcggcuccg ggucguuacc cauaggugug gccaaauacu 4560
acgggacuga ucaggaacuc uuaaaggccu gcaccgaucu cagaauuacg gugaggagga 4620
cuguucgagc aggagagaug aucguauaca ugguggauuc gauuggugcu ccacuccuac 4680
cauggucagg caggcugaga cagggaauga uauuuaaugc aaacaagguc gcacuagcuc 4740
cccaaugccu cccuguggac aaggacauaa gacucagagu gguguuuguc aaugggacau 4800

cucuaggggc aaucaccaua gccaagaucc caaagacccu ugcagaccuu gcauugccca 4860
acucuauauc uguuaauuua cuggugacac ucaagaccgg gaucuccaca gaacaaaagg 4920
ggguacuccc aguacuugau gaucaagggg agaaaaagcu caauuuuaug gugcaccucg 4980
gguugaucag gagaaagguc gggaagauau acucuguuga guacugcaag agcaagauug 5040
agagaaugcg gcugauuuuc ucacuugggu uaaucggcgg uauaagcuuc cauguucagg 5100
uuaaugggac acuaucuaag acauucauga gucagcucgc auggaagagg gcagucugcu 5160
ucccauuaau ggaugugaau ccccauauga acauggugau uugggcggca ucuguagaaa 5220
ucacaggcgu cgaugcggug uuccaaccgg ccaucccucg ugauuuccgc uacuacccua 5280
auguuguggc uaagaacauc ggaaggauca gaaagcugua aaugugcacc caucagagac 5340
cugcgacaau gccccaagca gacaccaccu ggcagucgga gccaccgggu cacuccuugu 5400
cuuaaauaag aaaaacuuag ggauaaaguc ccuugugagu gcuugguugc aaaacucucc 5460
accugguaca agcacagauc auggauggug auaggggcaa acgugacucg uacuggucua 5520
cuucuccuag ugguagcacc acaaaaccag caucagguug ggagagguca aguaaagccg 5580
acacaugguu gcugauucuc ucauucaccc agugggcuuu gucaauugcc acagugauca 5640
ucuguaucau aauuucugcu agacaagggu auaguaugaa agaguacuca augacuguag 5700
aggcauugaa caugagcagc agggagguga aagagucacu uaccagucua auaaggcaag 5760
agguuauagc aagggcuguc aacauucaga gcucugugca aaccggaauc ccagucuugu 5820
ugaacaaaaa cagcagggau gucauccaga ugauugauaa gucgugcagc agacaagagc 5880
ucacucagca cugugagagu acgaucgcag uccaccaugc cgauggaauu gccccacuug 5940
agccacauag uuucuggaga ugcccugucg gagaaccgua ucuuagcuca gauccugaaa 6000
ucucauugcu gccugguccg agcuuguuau cugguucuac aacgaucucu ggauguguua 6060
ggcucccuuc acucucaauu ggcgaggcaa ucuaugccua uucaucaaau cucauuacac 6120
aagguugugc ugacauaggg aaaucauauc agguccugca gcuaggguac auaucacuca 6180
auucagauau guucccugau cuuaaccccg uaguguccca cacuuaugac aucaacgaca 6240
aucggaaauc augcucugug guggcaaccg ggacuagggg uuaucagcuu ugcuccaugc 6300
cgacuguaga cgaaagaacc gacuacucua gugaugguau ugaggaucug guccuugaug 6360

uccuggaucu caaagggaga acuaagucuc accgguaucg caacagcgag guagaucuug 6420
aucacccguu cucugcacua uaccccagug uaggcaacgg cauugcaaca gaaggcucau 6480
ugauauuucu uggguauggu ggacuaacca ccccucugca gggugauaca aaauguagga 6540
cccaaggaug ccaacaggug ucgcaagaca caugcaauga ggcucugaaa auuacauggc 6600
uaggagggaa acaggugguc agcgugauca uccaggucaa ugacuaucuc ucagagaggc 6660
caaagauaag agucacaacc auuccaauca cucaaaacua ucucggggcg gaagguagau 6720
uauuaaaauu gggugaucgg guguacaucu auacaagauc aucaggcugg cacucucaac 6780
ugcagauagg aguacuugau gucagccacc cuuugacuau caacuggaca ccucaugaag 6840
ccuugucuag accaggaaau aaagagugca auugguacaa uaaguguccg aaggaaugca 6900
uaucaggcgu auacacugau gcuuauccau uguccccuga ugcagcuaac gucgcuaccg 6960
ucacgcuaua ugccaauaca ucgcguguca acccaacaau cauguauucu aacacuacua 7020
acauuauaaa uauguuaagg auaaaggaug uucaauuaga ggcugcauau accacgacau 7080
cguguaucac gcauuuuggu aaaggcuacu gcuuucacau caucgagauc aaucagaaga 7140
gccugaauac cuuacagccg augcucuuua agacuagcau cccuaaauua ugcaaggccg 7200
agucuuaaau uuaacugacu agcaggcuug ucggccuugc ugacacuaga gucaucuccg 7260
aacauccaca auaucucuca gucucuuacg ucucucacag uauuaagaaa aacccagggu 7320
gaaugggaag cuugccauag gucauggaug ggcaggaguc cucccaaaac ccuucugaca 7380
uacucuaucc agaaugccac cugaacucuc ccauagucag gggaagauag cacaguugc 7440
acgucuuguu agaugugaac cagcccuaca gacugaagga cgacagcaua auaaauauua 7500
caaagcacaa aauuaggaac ggaggauugu ccccccguca aauuaagauc aggucucugg 7560
guaaggcucu ucaacgcaca auaaaggauu uagaccgaua cacguuugaa ccguacccaa 7620
ccuacucuca ggaauuacuu aggcuugaua uaccagagau augugacaaa auccgauccg 7680
ucuucgcggu cucggaucgg cugaccaggg aguuaucuag uggguuccag gaucuuuggu 7740
ugaauaucuu caagcaacua ggcaauauag aaggaagaga gggguacgau ccguugcagg 7800
auaucggcac caucccggag auaacugaua aguacagcag gaauagaugg uauaggccau 7860
uccuaacuug guucagcauc aaauaugaca ugcgguggau gcagaagacc agaccggggg 7920
gaccccucga uaccucuaau ucacauaacc uccuagaaug caaaucauac acucuaguaa 7980

cauacggaga ucuugucaug auacugaaca aguugacauu gacaggguau auccuaaccc 8040
cugagcuggu cuugauguau ugugauguug uagaaggaag guggaauaug ucugcugcag 8100
ggcaucuaga uaagaagucc auugggauaa caagcaaagg ugaggaauua ugggaacuag 8160
uggauucccu cuucucaagu cuuggagagg aaauauacaa ugucaucgca cuauuggagc 8220
cccuaucacu ugcucucaua caacuaaaug auccuguuau accucuacgu ggggcauuua 8280
ugaggcaugu guugacagag cuacagacug uuuuaacaag uagagacgug uacacagaug 8340
cugaagcaga cacuauugug gagucguuac ucgccauuuu ccauggaacc ucuauugaug 8400
agaaagcaga gaucuuuucc uucuuuagga cauuuggcca ccccagcuua gaggcuguca 8460
cugccgccga caagguaagg gcccauaugu augcacaaaa ggcaauaaag cuuaagaccc 8520
uauacgagug ucaugcaguu uuuugcacua ucaucauaaa uggguauaga gagaggcaug 8580
gcggacagug gccccccugu gacuucccug aucacgugug ucuagaacua aggaacgcuc 8640
aaggguccaa uacggcaauc ucuuaugaau gugcuguaga caacuauaca aguuucauag 8700
gcuucaaguu ucggaaguuu auagaaccac aacuagauga agaucucaca auauauauga 8760
aagacaaagc acuauccccc aggaaggagg caugggacuc uguauacccg gauaguaauc 8820
uguacuauaa agccccagag ucugaagaga cccggcggcu uauugaagug uucauaaaug 8880
augagaauuu caacccagaa gaaauuauca auuaugugga gucaggagau ugguugaaag 8940
acgaggaguu caacaucucg uacagucuca aagagaaaga gaucaagcaa gagggucguc 9000
uauucgcaaa aaugacuuau aagaugcgag ccguacaggu gcuggcagag acacuacugg 9060
cuaaaggaau aggagagcua uucagcgaaa augggauggu uaaaggagag auagaccuac 9120
uuaaaagauu gacuacucuu ucugucucag gcguccccag gacugauuca guguacaaua 9180
acucuaaauc aucagagaag agaaacgaag gcauggaaaa uaagaacucu gggggguacu 9240
gggacgaaaa gaagaggucc agacaugaau ucaaggcaac agauucauca acagacggcu 9300
augaaacguu aaguugcuuc cucacaacag accucaagaa auacugcuua aacuggagau 9360
uugagaguac ugcauuguuu ggucagagau gcaacgagau auuuggcuuc aagaccuucu 9420
uuaacuggau gcauccaguc cuugaaaggu guacaauaua uguuggagau ccuuacuguc 9480
cagucgccga ccggaugcau cgacaacucc aggaucaugc agacucuggc auuuucauac 9540

auaauccuag ggggggcaua gaagguuacu gccagaagcu guggaccuua aucucaauca 9600
gugcaaucca ccuagcagcu gugagagugg gugucagggu cucugcaaug guucagggug 9660
acaaucaagc uauagccgug acaucaagag uaccuguagc ucagacuuac aagcagaaga 9720
aaaaucaugu cuaugaggag aucaccaaau auuucggugc ucuaagacac gucauguuug 9780
auguagggca cgagcuaaaa uugaacgaga ccaucauuag uagcaagaug uuugucuaua 9840
guaaaaggau auacuaugau gggaagauuu uaccacagug ccugaaagcc uugaccaagu 9900
guguauucug guccgagaca cugguagaug aaaacagauc ugcuuguucg aacaucucaa 9960
cauccauagc aaaagcuauc gaaaaugggu auucuccuau acuaggcuac ugcauugcgu 10020
uguauaagac cugucagcag gugugcauau cacuagggau gacuauaaau ccaacuauca 10080
gcccgaccgu aagagaucaa uacuuuaagg guaagaauug gcugagaugu gcaguguuga 10140
uuccagcaaa uguuggagga uucaacuaca ugucuacauc uagaugcuuu guuagaaaua 10200
uuggagaccc cgcaguagca gcccuagcug aucucaaaag auucaucaga gcggaucugu 10260
uagacaagca gguauuauac agggucauga aucaagaacc cggugacucu aguuuucuag 10320
auugggcuuc agacccuuau ucguguaacc ucccgcauuc ucagaguaua acuacgauua 10380
uaaagaauau cacugcuaga ucugugcugc aggaaucccc gaauccucua cugucugguc 10440
ucuucaccga gacuagugga gaagaggauc ucaaccuggc cucguuccuu auggaccgga 10500
aagucauccu gccgagagug gcucaugaga uccuggguaa uuccuuaacu ggaguuaggg 10560
aggcgauugc agggaugcuu gauacgacca agucucuagu gagagccagc guuaggaaag 10620
gaggauuauc auaugggaua uugaggaggc uugucaauua ugaucuauug caguacgaga 10680
cacugacuag aacucucagg aaaccgguga aagacaacau cgaauaugag uauauguguu 10740
caguugagcu agcugucggu cuaaggcaga aaauguggau ccaccugacu uacgggagac 10800
ccauacaugg gcuagaaaca ccagacccuu uagagcucuu gaggggaaua uuuaucgaag 10860
guucagaggu gugcaagcuu ugcaggucug aaggagcaga ccccaucuau acaugguucu 10920
aucuuccuga caauauagac cuggacacgc uuacaaacgg auguccggcu auaagaaucc 10980
ccuauuuugg aucagccacu gaugaaaggu cggaagccca acucggguau guaagaaauc 11040
uaagcaaacc cgcaaaggcg gccauccgga uagcuauggu guauacgugg gccuacggga 11100
cugaugagau aucguggaug gaagccgcuc uuauagccca aacaagagcu aaucugagcu 11160

uagagaaucu aaagcugcug acuccuguuu caaccuccac uaaucuaucu cauagguuga 11220
aagauacggc aacccagaug aaguucucua gugcaacacu aguccgugca agucgguuca 11280
uaacaauauc aaaugauaac auggcacuca aagaagcagg ggagucgaag gauacuaauc 11340
ucguguauca gcagauuaug cuaacugggc uaagcuuguu cgaguucaau augagauaua 11400
agaaagguuc cuuagggaag ccacugauau ugcacuuaca ucuuaauaac gggugcugua 11460
uaauggaguc cccacaggag gcgaauaucc ccccaagguc cacauuagau uuagagauua 11520
cacaagagaa caauaaauug aucuaugauc cugauccacu caaggaugug gaccuugagc 11580
uauuuagcaa ggucagagau guuguacaca caguugacau gacuuauugg ucagaugaug 11640
aaguuaucag agcaaccagu aucuguacug caaugacgau agcugauaca augucucaau 11700
uagauagaga caacuuaaaa gagaugaucg cacuaguaaa ugacgaugau gucaacagcu 11760
ugauuacuga guuuauggug auugauguuc cuuuauuuug cucaacguuc ggggguauuc 11820
uagucaauca guuugcauac ucacucuacg gcuuaaacau cagaggaagg gaagaaauau 11880
ggggacaugu aguccggauu cuuaaagaua ccucccacgc aguuuuaaaa gucuuaucua 11940
augcucuauc ucaucccaaa aucuucaaac gauucuggaa ugcagguguc guggaaccug 12000
uguaugggcc uaaccucuca aaucaggaua agauacucuu ggcccucucu gucugugaau 12060
auucugugga ucuauucaug cacgauuggc aagggggugu accgcuugag aucuuuaucu 12120
gugacaauga cccagaugug gccgacauga ggagguccuc uuucuuggca agacaucuug 12180
cauaccuaug cagcuuggca gagauaucua gggaugggcc aagauuagaa ucaaugaacu 12240
cucuagagag gcucgaguca cuaaagaguu accuggaacu cacauuucuu gaugacccgg 12300
uacugaggua cagucaguug acuggccuag ucaucaaagu auucccaucu acuuugaccu 12360
auauccggaa gucaucuaua aaaguguuaa ggacaagagg uauaggaguc ccugaagucu 12420
uagaagauug ggaucccgag gcagauaaug cacuguuaga ugguaucgcg gcagaaauac 12480
aacagaauau uccuuuggga caucagacua gagccccuuu uuggggguug agaguaucca 12540
agucacaggu acugcgucuc cggggguaca aggagaucac aagaggugag auaggcagau 12600
cagguguugg ucugacguua ccauucgaug gaagauaucu aucucaccag cugaggcucu 12660
uuggcaucaa caguacuagc ugcuugaaag cacuugaacu uaccuaccua uugagccccu 12720

uaguugacaa ggauaaagau aggcuauauu uaggggaagg agcuggggcc augcuuuccu 12780
guuaugacgc uacucuuggc ccaugcauca acuauuauaa cucaggggua uacucuugug 12840
augucaaugg gcagagagag uuaaauauau auccugcuga gguggcacua gugggaaaga 12900
aauuaaacaa uguuacuagu cugggucaaa gaguuaaagu guuauucaac gggaauccug 12960
gcucgacaug gauugggaau gaugagugug aggcuuugau uuggaaugaa uuacagaaua 13020
gcucgauagg ccuaguccac ugugacaugg agggaggaga ucauaaggau gaucaaguug 13080
uacugcauga gcauuacagu guaauccgga ucgcguaucu ggugggggau cgagacguug 13140
ugcuuauaag caagauugcu cccaggcugg gcacggauug gaccaggcag cucagccuau 13200
aucugagaua cugggacgag guuaaccuaa uagugcuuaa aacaucuaac ccugcuucca 13260
cagagaugua ucuccuaucg aggcacccca aaucugacau uauagaggac agcaagacag 13320
uguuagcuag ucuccucccu uugucaaaag aagauagcau caagauagaa aaguggaucu 13380
uaauagagaa ggcaaaggcu cacgaauggg uuacucggga auugagagaa ggaagcucuu 13440
caucagggau gcuuagaccu uaccaucaag cacugcagac guuuggcuuu gaaccaaacu 13500
uguauaaauu gagcagagau uucuugucca ccaugaacau agcugauaca cacaacugca 13560
ugauagcuuu caacaggguu uugaaggaua caaucuucga augggcuaga auaacugagu 13620
cagauaaaag gcuuaaacua acugguaagu augaccugua uccugugaga gauucaggca 13680
aguugaagac aauuucuaga agacuugugc uaucuuggau aucuuuaucu auguccacaa 13740
gauugguaac ugggucauuc ccugaccaga aguuugaagc aagacuucaa uugggaauag 13800
uuucauuauc aucccgugaa aucaggaacc ugaggguuau cacaaaaacu uuauuagaca 13860
gguuugagga uauuauacau aguauaacgu auagauuccu caccaaagaa auaaagauuu 13920
ugaugaagau uuuaggggca gucaagaugu ucggggccag gcaaaaugaa uacacgaccg 13980
ugauugauga uggaucacua ggugauaucg agccauauga cagcucguaa uaauuagucc 14040
cuaucgugca gaacgaucga agcuccgcgg uaccuggaag ucuuggacuu guccauauga 14100
caauaguaag aaaaacuuac aagaagacaa gaaaauuuaa aaggauacau aucucuuaaa 14160
cucuugucug gu 14172

<210> 18
<211> 14172

<212>  RNA
<213>  Artificial

<220>
<223>  SeV18+mIL-10/TS-dF (genomic RNA)


<220>
<221>  CDS
<222>  (146)..(6829)

<220>
<221>  CDS
<222>  (6968)..(8692)

<220>
<221>  CDS
<222>  (8855)..(9898)

<220>
<221>  CDS
<222>  (10020)..(11723)

<220>
<221>  CDS
<222>  (11876)..(13447)

<220>
<221>  CDS
<222>  (13504)..(14037)

<400>  18
accagacaag aguuuaagag auauguaucc uuuuaaauuu ucuugucuuc uuguaaguuu      60

uucuuacuau ugucauaugg acaaguccaa gacuuccagg uaccgcggag cuucgaucgu     120

ucugcacgau agggacuaau uauuacgagc ugucauaugg cucgauauca ccuagugauc     180

caucaucaau cacggucgug uauucauuuu gccuggcccc gaacaucuug acugccccua     240

aaaucuucau caaaaucuuu auuucuuugg ugaggaaucu auacguuaua cuauguauaa     300

uauccucaaa ccugucuaau aaaguuuuug ugauaacccu cagguuccug auuucacggg     360

augauaauga aacuauuccc aauugaaguc uugcuucaaa cuucugguca gggaaugacc     420

caguuaccaa ucuuguggac auagauaaag auauccaaga uagcacaagu cuucuagaaa     480

uugucuucaa cuugccugaa ucucucacag gauacagguc auacuuacca guuaguuuaa     540

gccuuuuauc ugacucaguu auucuagccc auucgaagau uguauccuuc aaaacccugu     600

ugaaagcuau caugcaguug uguguaucag cuauguucau gguggacaag aaaucucugc     660

ucaauuuaua caaguuuggu ucaaagccaa acgucugcag ugcuugaugg uaaggucuaa 720
gcaucccuga ugaagagcuu ccuucucuca auucccgagu aacccauucg ugagccuuug 780
ccuucucuau uaagauccac uuuucuaucu ugaugcuauc uucuuuugac aaagggagga 840
gacuagcuaa cacugucuug cuguccucua uaaugucaga uuuggggugc cucgauagga 900
gauacaucuc uguggaagca ggguuagaug uuuuaagcac uauuagguua accucguccc 960
aguaucucag auauaggcug agcugccugg uccaauccgu gcccagccug ggagcaaucu 1020
ugcuuauaag cacaacgucu cgauccccca ccagauacgc gauccggauu acacuguaau 1080
gcucaugcag uacaacuuga ucauccuuau gaucuccucc cuccauguca caguggacua 1140
ggccuaucga gcuauucugu aauucauucc aaaucaaagc cucacacuca ucauucccaa 1200
uccaugucga gccaggauuc ccguugaaua acacuuuaac ucuuugaccc agacuaguaa 1260
cauuguuuaa uuucuuuccc acuagugcca ccucagcagg auauauauuu aacucucucu 1320
gcccauugac aucacaagag uauaccccug aguuauaaua guugaugcau gggccaagag 1380
uagcgucaua acaggaaagc auggccccag cuccuucccc uaaauauagc cuaucuuuau 1440
cuucgucaac uaaggggcuc aauagguagg uaaguucaag ugcuuucaag cagcuaguac 1500
uguugaugcc aaagagccuc agcuggugag auagauaucu uccaucgaau gguaacguca 1560
gaccaacacc ugaucugccu aucucaccuc uugugaucuc cuuguacccc cggagacgca 1620
guaccuguga cuuggauacu cucaaccccc aaaaaggggc ucuagucuga ugucccaaag 1680
gaauauucug uuguauuucu gccgcgauac caucuaacag ugcauuaucu gccucgggau 1740
cccaaucuuc uaagacuuca gggacuccua uaccucuugu ccuuaacacu uuuauagaug 1800
acuuccggau auaggucaaa guagauggga auacuuugau gacuaggcca gucaacugac 1860
uguaccucag uaccggguca ucaagaaaug ugaguuccag guaacucuuu agugacucga 1920
gccucucuag agaguucauu gauucuaauc uuggcccauc ccuagauauc ucugccaagc 1980
ugcauaggua ugcaagaugu cuugccaaga aagaggaccu ccucaugucg gccacaucug 2040
ggucauuguc acagauaaag aucucaagcg guacaccccc uugccaaucg ugcaugaaua 2100
gauccacaga auauucacag acagagaggg ccaagaguau cuuauccuga uuugagaggu 2160
uaggcccaua cacagguucc acgacaccug cauuccagaa ucguuugaag auuuuuggau 2220

gagauagagc auuagauaag acuuuuaaaa cugcguggga gguaucuuua agaauccgga 2280

cuacaugucc ccauauuucu ucccuuccuc ugauguuuaa gccguagagu gaguaugcaa 2340

acugauugac uagaauaccc ccgaacguug agcaaaauaa aggaacauca aucaccauaa 2400

acucaguaau caagcuguug acaucaucgu cauuuacuag ugcgaucauc ucuuuuaagu 2460

ugucucuauc uaauugagac auuguaucag cuaucgucau ugcaguacag auacugguug 2520

cucugauaac uucaucaucu gaccaauaag ucaugucaac uguguguaca acaucucuga 2580

ccuugcuaaa uagcucaagg uccacauccu ugaguggauc aggaucauag aucaauuuau 2640

uguucucuug uguaaucucu aaaucuaaug uggaccuugg ggggauauuc gccuccugug 2700

gggacuccau uauacagcac ccguuauuaa gauguaagug caauaucagu ggcuucccua 2760

aggaaccuuu cuuauaucuc auauugaacu cgaacaagcu uagcccaguu agcauaaucu 2820

gcugauacac gagauuagua uccuucgacu ccccugcuuc uuugagugcc auguuaucau 2880

uugauauugu uaugaaccga cuugcacgga cuaguguugc acuagagaac uucaucuggg 2940

uugccguauc uuucaaccua ugagauagau uaguggaggu ugaaacagga gucagcagcu 3000

uuagauucuc uaagcucaga uuagcucuug uuugggcuau aagagcggcu uccauccacg 3060

auaucucauc agucccguag gcccacguau acaccauagc uauccggaug gccgccuuug 3120

cggguuugcu uagauuucuu acauacccga guugggcuuc cgaccuuuca ucaguggcug 3180

auccaaaaua ggggauucuu auagccggac auccguuugu aagcgugucc aggucuauag 3240

agucaggaag auagaaccau guauagaugg ggucugcucc uucagaccug caaagcuugc 3300

acaccucuga accuucgaua aauauucccc ucaagagcuc uaaaggucu gguguuucua 3360

gcccauguau gggucucccg uaagucaggu ggauccacau uuucugccuu agaccgacag 3420

cuagcucaac ugaacacaua uacucauauu cgauguuguc uuucaccggu uuccugagag 3480

uucuagucag ugucucguac ugcaauagau cauaauugac aagccuccuc aauaucccau 3540

augauaaucc uccuuuccua acgcuggcuc ucacuagaga cuuggucgua ucaagcaucc 3600

cugcaaucgc cucccuaacu ccaguuaagg aauuacccag gaucucauga gccacucucg 3660

gcaggaugac uuuccggucc auaaggaacg aggccagguu gagauccucu ucuccacuag 3720

ucucggugaa gagaccagac aguagaggau ucgggauuuc cugcagcaca gaucuagcag 3780

ugauauucuu uauaaucgua guuauacucu gagaaugcgg gagguuacac gaauaagggu 3840

cugaagccca aucuagaaaa cuagagucac cggguucuug auucaugacc cuguauaaua 3900
ccugcuuguc uaacagaucc gcucugauga aucuuuugag aucagcuagg gcugcuacug 3960
cggggucucc aauauuucua acaaagcauc uagauguaga cauguaguug aauccuccaa 4020
cauuugcugg aaucaacacu gcacaucuca gccaauucuu acccuuaaag uauugaucuc 4080
uuacggucgg gcugauaguu ggauuuauag ucaucccuag ugauaugcac accugcugac 4140
aggucuuaua caacgcaaug caguagccua guauaggaga auacccauuu ucgauagcuu 4200
uugcuaugga uguugagaug uucgaacaag cagaucuguu uucaucuacc agugucucgg 4260
accagaauac acacuugguc aaggcuuuca ggcacugugg uaaaaucuuc ccaucauagu 4320
auauccuuuu acuauagaca aacaucuugc uacuaaugau ggucucguuc aauuuuagcu 4380
cgugcccuac aucaaacaug acgugucuua gagcaccgaa auauuuggug aucuccucau 4440
agacaugauu uuucuucugc uuguaagucu gagcuacagg uacucuugau gucacggcua 4500
uagcuugauu gucacccuga accauugcag agacccugac acccacucuc acagcugcua 4560
gguggauugc acugauugag auuaaggucc acagcuucug gcaguaaccu ucuaugcccc 4620
cccuaggauu auguaugaaa augccagagu cugcaugauc cuggaguugu cgaugcaucc 4680
ggucggcgac uggacaguaa ggaucuccaa cauauauugu acaccuuuca aggacuggau 4740
gcauccaguu aaagaagguc uugaagccaa auaucucguu gcaucucuga ccaaacaaug 4800
caguacucuc aaaucuccag uuuaagcagu auuucuugag gucuguugug aggaagcaac 4860
uuaacguuuc auagccgucu guugaugaau cuguugccuu gaauucaugu cuggaccucu 4920
ucuuuucguc ccaguacccc ccagaguucu uauuuuccau gccuucguuu cucuucucug 4980
augauuuaga guuauuguac acugaaucag uccuggggac gccugagaca gaaagaguag 5040
ucaaucuuuu aaguaggucu aucucuccuu uaaccauccc auuuucgcug aauagcucuc 5100
cuauuccuuu agccaguagu gucucugcca gcaccuguac ggcucgcauc uuauaaguca 5160
uuuuugcgaa uagacgaccc ucuugcuuga ucucuuucuc uuugagacug uacgagaugu 5220
ugaacuccuc gucuuucaac caaucuccug acuccacaua auugauaauu ucuucugggu 5280
ugaaauucuc aucauuuaug aacacuucaa uaagccgccg ggucucuuca gacucugggg 5340
cuuuauagua cagauuacua uccggguaua cagaguccca ugccuccuuc cugggggaua 5400

gugcuuuguc uuucauauau auugugagau cuucaucuag uugugguucu auaaacuucc 5460
gaaacuugaa gccuaugaaa cuuguauagu ugucuacagc acauucauaa gagauugccg 5520
uauuggaccc uugagcguuc cuuaguucua gacacacgug aucagggaag ucacaggggg 5580
gccacugucc gccaugccuc ucucuauacc cauuuaugau gauagugcaa aaaacugcau 5640
gacacucgua uagggucuua agcuuuauug ccuuuugugc auacauaugg gcccuuaccu 5700
ugucggcggc agugacagcc ucuaagcugg gguggccaaa uguccuaaag aaggaaaaga 5760
ucucugcuuu cucaucaaua gagguuccau ggaaaauggc gaguaacgac uccacaauag 5820
ugucugcuuc agcaucugug uacacgucuc uacuuguuaa aacagucugu agcucuguca 5880
acacaugccu cauaaaugcc ccacguagag guauaacagg aucauuuagu uguaugagag 5940
caagugauag gggcuccaau agugcgauga cauuguauau uuccucucca agacuugaga 6000
agagggaauc cacuaguucc cauaauuccu caccuuugcu uguuauccca auggacuucu 6060
uaucuagaug cccugcagca gacauauucc accuuccuuc uacaacauca caauacauca 6120
agaccagcuc agggguuagg auauacccug ucaaugucaa cuuguucagu aucaugacaa 6180
gaucuccgua uguuacuaga guguaugauu ugcauucuag gagguuaugu gaauuagagg 6240
uaucgagggg uccccccggu cuggucuucu gcauccaccg caugucauau uugaugcuga 6300
accaaguuag gaauggccua uaccaucuau uccugcugua cuuaucaguu aucuccggga 6360
uggugccgau auccugcaac ggaucguacc ccucucuucc uucuauauug ccuaguugcu 6420
ugaagauauu caaccaaaga uccuggaacc cacuagauaa cucccugguc agccgauccg 6480
agaccgcgaa gacggaucgg auuuugucac auaucucugg uauaucaagc cuaaguaauu 6540
ccugagagua gguuggguac gguucaaacg uguaucgguc uaaauccuuu auugugcguu 6600
gaagagccuu acccagagac cugaucuuaa uuugacgggg ggacaauccu ccguuccuaa 6660
uuuugugcuu uguaauauuu auuaugcugu cguccuucag ucuguagggc ugguucacau 6720
cuaacaagac gugcaacugu gcuaucuucc cccugacuau gggagaguuc agguggcauu 6780
cuggauagag uaugucagaa ggguuuuggg aggacuccug cccauccaug accuauggca 6840
agcuucccau ucacccuggg uuuuucuuaa uacugugaga gacguaagag acugagagau 6900
auuguggaug uucggagaug acucuagugu cagcaaggcc gacaagccug cuagucaguu 6960
aaauuuaaga cucggccuug cauaauuuag ggaugcuagu cuuaaagagc aucggcugua 7020

agguauucag gcucuucuga uugaucucga ugaugugaaa gcaguagccu uuaccaaaau 7080
gcgugauaca cgaugucgug guauaugcag ccucuaauug aacauccuuu auccuuaaca 7140
uauuuauaau guuaguagug uuagaauaca ugauuguugg guugacacgc gauguauugg 7200
cauauagcgu gacgguagcg acguuagcug caucagggga caauggauaa gcaucagugu 7260
auacgccuga uaugcauucc uucggacacu uauuguacca auugcacucu ucauuuccug 7320
gucuagacaa ggcuucauga gguguccagu ugauagucaa aggguggcug acaucaagua 7380
cuccuaucug caguugagag ugccagccug augaucuugu auagauguac acccgaucac 7440
ccaauuuuaa uaaucuaccu uccgccccga gauaguuuug agugauugga augguuguga 7500
cucuuaucuu uggccucucu gagagauagu cauugaccug gaugaucacg cugaccaccu 7560
guuucccucc uagccaugua auuuucagag ccucauugca ugugucuugc gacaccuguu 7620
ggcauccuug gguccuacau uuuguaucac ccugcagagg ggugguuagu ccaccauacc 7680
caagaaauau caaugagccu ucuguugcaa ugccguugcc uacacugggg uauagugcag 7740
agaacgggug aucaagaucu accucgcugu ugcgauaccg gugagacuua guucucccuu 7800
ugagauccag gacaucaagg accagauccu caauaccauc acuagaguag ucgguucuuu 7860
cgucuacagu cggcauggag caaagcugau aaccccuagu ccggguuguc accacagagc 7920
augauuuccg auugucguug augucauaag ugugggacac uacgggguua agaucaggga 7980
acauaucuga auugagugau auguacccua gcugcaggac cugauaugau uucccuaugu 8040
cagcacaacc uuguguaaug agauuugaug aauaggcaua gauugccucg ccaauugaga 8100
gugaagggag ccuaacacau ccagagaucg uuguagaacc agauaacaag cucggaccag 8160
gcagcaauga gauuucagga ucugagcuaa gauacgguuc uccgacaggg caucuccaga 8220
aacuaugugg cucaaguggg gcaauuccau cggcauggug gacugcgauc guacucucac 8280
agugcugagu gagcucuugu cugcugcacg acuuaucaau caucuggaug acaucccugc 8340
uguuuuuguu caacaagacu gggauuccgg uuugcacaga gcucugaaug uugacagccc 8400
uugcuauaac cucuugccuu auuagacugg uaagugacuc uuucaccucc cugcugcuca 8460
uguucaaugc cucuacaguc auugaguacu cuuucauacu auacccuugu cuagcagaaa 8520
uuaugauaca gaugaucacu guggcaauug acaaagccca cugggugaau gagagaauca 8580

gcaaccaugu gucggcuuua cuugaccucu cccaaccuga ugcugguuuu guggugcuac 8640
cacuaggaga aguagaccag uacgagucac guuugccccu aucaccaucc augaucugug 8700
cuuguuugag gugaaagcuu aauuaaccaa gcacucacaa gggacuuuau cccuaaguuu 8760
uucuuauuua agacaaggag ugacccggug gcuccgacug ccagguggug ucugcuuggg 8820
gcauugucgc aggucucuga ugggugcaca uuuacagcuu ucugauccuu ccgauguucu 8880
uagccacaac auuaggguag uagcggaaau cacgagggau ggccgguugg aacaccgcau 8940
cgacgccugu gauuucuaca gaugccgccc aaaucaccau guucauaugg ggauucacau 9000
ccauuaaugg gaagcagacu gcccucuucc augcgagcug acucaugaau gucuuagaua 9060
gugucccauu aaccugaaca uggaagcuua uaccgccgau uaacccaagu gagaaaauca 9120
gccgcauucu cucaaucuug cucuugcagu acucaacaga guauaucuuc ccgaccuuuc 9180
uccugaucaa cccgaggugc accauaaaau ugagcuuuuu cuccccuuga ucaucaagua 9240
cugggaguac ccccuuuugu ucuguggaga ucccggucuu gagugucacc aguaaauuaa 9300
cagauauaga guugggcaau gcaaggucug caaggucuuu ugggaucuug gauaugguga 9360
uugccccuag agauguccca uugacaaaca ccacucugag ucuuaugucc uuguccacag 9420
ggaggcauug gggagcuagu gcgaccuugu uugcauuaaa uaucauuccc ugucucagcc 9480
ugccugacca ugguaggagu ggagcaccaa ucgaauccac cauguauacg aucaucucuc 9540
cugcucgaac agcccuccuc accguaauuc ugagaucggu gcaggccuuu aagaguuccu 9600
gaucaguccc guaguauuug gccacaccua uggguaacga cccggagccg cauauugagu 9660
agcuggucgg cucugucaag ucagauacgc ucucuagauu gguuguuugu uucggugucu 9720
caaagaaacc caagagcaau aaaucuaggu aucucacucc auguuuagga gggucuccua 9780
ccuugacaau ccugaugugg gggauggcuu ucuuauccgg accaguucuc agaggcaggg 9840
gcuccacagu accguuaucc ucauaugaga acuuagggaa ucuauagaua ucugccauug 9900
cgccguguua ggugaaauuu cuuucacccu aaguuuuucu uaaucuuuac uggcugaugc 9960
ugaugguaga uuggaucucu cuaugacuga ggaugguagg augccucacc cgggaucuag 10020
uuggucagug acucuauguc cucuucuacg aguuccauga cugccuuaac cucuuggucu 10080
gucuugcacu uggauaauga uuucacauau gcuacuuucu cagcucugcu uaggggacug 10140
cucucuauga cgagccugag agagugcauu gugggcuucu cuuuggaggg aaagagacgu 10200

gaugcguuug aggcccuggg uucugugucc cucucuuggu acaccggguu gcggaucuca 10260
ucucuaaauu cauccucucg gauuagguccc gguuuguacu ucauaucuuc uaggucuccc 10320
auagaugggu caaaccuggu agccuuaguc uuguucucuu uugauuuugc aaaaacggag 10380
ggggaccuug uaagggaguc uguguuguca gucuugccac cucuaucugu gaugauauga 10440
aguguagaua gguuggacau cagcaaugag uucuguucuu ucugauacuc agagaaucuc 10500
uuguaaauau cccggaauga uuccacgcuc ucuuggaucu guuugagcag uuguuuguuc 10560
ucaucuaccu uacgagcgga agauuucucg gcagaaagga ucaggccgca uacauugaau 10620
gucaucucug cauaguuugc agacuuuagg gcacgucuug caaacacaua acucgcgucu 10680
cgggaugauu cgaauucuug agcagacugg auuacaccaa gacucgucaa caauguagcc 10740
aucucuuuca uagaugaugu guucucuccu augcccuuuu uuguugaguc ggucucuaga 10800
cccgggugga uugggcggcc guuggcugga caaucugaga cagagcgggu cccuauuggu 10860
gguuuccggu cuuugacccu gacggcgggg guguccccag aguugaugug cucauccugu 10920
guagaugggg guuuuccugg uggugacccu gugcuguugu aacgauucag cgguggggac 10980
cgggugccag gcacgguugc uggaguaaga gguuuggacc cacuguuggu aggucuucuu 11040
uuguuccucc guagcacagc cucuucaagu ucggggcuag gaaucaccag gaccccaguu 11100
acucuugcac uaugugagcu gccaggcucc augcuucuuc cauuauuacu ugcuccaccu 11160
ucuccuucau cagguaggga uguacuuccu cguaccucuu cuggaagucc uucagcuugg 11220
ucuucucccc ucuuaucagg gugcgcagcc aucucucugu uuucaucuuc aauaccugau 11280
cucggauauc cucucucauu uggaggauuu ucaaggauuc cggagucucc uccaucgccc 11340
agauccugag auacagaguu uguaccaguu cuucccccaa aggcccggug uauauuuugu 11400
uuaucaaggu uuccagcaug ugcuucugcc ucuggcuugc uuguucuccc agagacucua 11460
cucuccucac cugaucgauu aucuuggguc gacgguguug agacuucucc uucgcccuca 11520
cuuuuggcuc uaugagcaga gccugguccu uggggagugu ugaugguguu guggagccag 11580
cuucuguccc cuccgauguc aguugguuca cucgacagga cagcaucgag gaauccgaua 11640
acauccgaga gcgacucucg uccuccuggc gccucccucu caacuucaga aucuucuuua 11700
agaaugaagg caucuugauc caugcgguaa guguagccga agccguggcu gucucgacug 11760

cugggugugg ucgguggggu uggguguggc cuugccugag ccgaucggug gaugaacuuu 11820
cacccuaagu uuuucuuacu acggaucaag uaccuugaag ccucguauga uccuagauuc 11880
cuccuauccc agcuacugcu gcggcaucgu caucuucguc augaucgaca ccguuauugc 11940
ggccuucauc uccauggguu gcagaauccu cuugccgucu cucugcgagu cucauggcua 12000
uucuucucuc uaugucugau acauccucau cauugguuuc cuccucuaac cguucagccc 12060
cauguagugu gacaaagugg ccaccacuca ccugacgugc ccaucuuuca ccacuaucuc 12120
caccccaacc ccuagcgucc ugguccgcau gggcuuuugu uucuaggucg augucggcau 12180
ugucuagagc uaccucaauu gcaccaccgc cuguugguuu gugguaagca ccauccccac 12240
cggacaaguu ugccagauga ugucugagcc uccccuuggc uguauccguc acuccuaacu 12300
caucuuccaa ggcacugcug aucuucgauu cagcauccuu ugccacggcu uguccuagua 12360
agaacauuuc cauaucaagg uauguccucc cugugacgua cugcugcauu gccuuguucu 12420
guacgacggc gacucccaug gcguaacucc auagugcagg auaauugccu ggagcaaauu 12480
caccaugaac aggguccuug aggauacaga uaaagggagc ucuggggccu uuugacaggu 12540
aggugucuau gaggcuucua agcuuauuaa uaucgggccu cagguuugac aacguuagag 12600
cugccaucuu uguuuccacc ccauauuuaa uaguguucau gaaggaagcc agcccugcau 12660
cucggaugua guucccaacg aucuggaugu ucuucucuaa uguggugaga ucagaucuug 12720
caguauucau agucacaagg gucucaacca ugagagauac aaggcuuugc ugagaucuca 12780
uaaccgagcc uauccccuca acugucuccc cagugaaaac uaaggcaccu uucacggugc 12840
cgucuugucu gaacgccucu aaccuguuga agaacccuuu ccuuaagccg gcgcugcuug 12900
ugauggccuu caccagcaca auccagacuu ggacaauuau ugcuccuagg caugcaggau 12960
acccauagau uugaaggagu ggugucaggu cugcagcauc ccguugaccc uggaagagug 13020
ggcucuuguu gaccauaggu ccaaacagcc auucuguggu ccucucauau uccauaucuc 13080
ucgucuucac aaugaauccg ucugucuucg uccucuuagg gucuuucucu auguuguaga 13140
ucacauauuu gacaucggcg uuuacuccgu uuguugucaa guacaauucu ggacuacugu 13200
aagccauggc aagcagagag acgaggaacc ccccucucug agagugcugc uuaucugugu 13260
ccaaugagug agcuaggaag guaguugcaa ugaauaacuu gucugcauca ucagucacac 13320
uugggccuag uacgaacacu gagacugugc uccucuggcc ggggauaaca gcaccuccuc 13380

ccgacuuauu aauacuuucg cuccuccuag agcuaaaugu aucgaaggug cucaacaacc 13440

cggccaucgu gaagaucugc ggccgcgaug aacuuucacc cuaaguuuuu cuuacuacgg 13500

uuagcuuuuc auuuugauca ucauguaugc uucuaugcag uugaugaaga ugucaaauuc 13560

auucauggcc uuguagacac cuuggucuug gagcuuauua aaaucacucu ucaccugcuc 13620

cacugccuug cucuuauuuu cacaggggag aaaucgauga cagcgccuca gccgcauccu 13680

gagggucuuc agcuucucac ccagggaauu caaaugcucc uugauuucug ggccaugcuu 13740

cucugccugg ggcaucacuu cuaccaggua aaacuggauc auuuccgaua aggcuuggca 13800

acccaaguaa cccuuaaagu ccugcauuaa ggagucgguu agcaguaugu uguccagcug 13860

guccuuuguu ugaaagaaag ucuucaccug gcugaaggca guccgcagcu cuaggagcau 13920

guggcucugg ccgacuggga agugggugca guuauugucu ucccggcugu acuggccccu 13980

gcugauccuc augccaguca guaagagcag gcagcauagc agugcugagc caggcauuga 14040

acuuuggcgg ccgcgugaac uuuggcagca aagcaaaggg ucuggaaccu gcuccucagg 14100

guggauacuu ugacccuaaa auccuguaua acuucauuac auaucccaua cauguuuuuu 14160

cucuuguuug gu 14172

<210> 19
<211> 14172
<212> RNA
<213> Artificial

<220>
<223> SeV18+mIL-10/TS-dF (antigenomic RNA)


<220>
<221> CDS
<222> (136)..(669)

<220>
<221> CDS
<222> (726)..(2297)

<220>
<221> CDS
<222> (2450)..(4153)

<220>
<221> CDS

<222>  (4275)..(5318)

<220>
<221>  CDS
<222>  (5481)..(7205)

<220>
<221>  CDS
<222>  (7344)..(14027)

<400>  19
accaaacaag agaaaaaaca uguaugggau auguaaugaa guuauacagg auuuuagggu      60

caaaguaucc acccugagga gcagguucca gacccuuugc uuugcugcca aaguucacgc     120

ggccgccaaa guucaaugcc uggcucagca cugcuaugcu gccugcucuu acugacuggc     180

augaggauca gcaggggcca guacagccgg gaagacaaua acugcaccca cuucccaguc     240

ggccagagcc acaugcuccu agagcugcgg acugccuuca gccaggugaa gacuuucuuu     300

caaacaaagg accagcugga caacauacug cuaaccgacu ccuuaaugca ggacuuuaag     360

gguuacuugg guugccaagc cuuaucggaa augauccagu uuuaccuggu agaagugaug     420

ccccaggcag agaagcaugg cccagaaauc aaggagcauu ugaauucccu gggugagaag     480

cugaagaccc ucaggaugcg gcugaggcgc ugucaucgau uucuccccug ugaaaauaag     540

agcaaggcag uggagcaggu gaagagugau uuuaauaagc uccaagacca aggugucuac     600

aaggccauga augaauuuga caucuucauc aacugcauag aagcauacau gaugaucaaa     660

augaaaagcu aaccguagua agaaaaacuu agggugaaag uucaucgcgg ccgcagaucu     720

ucacgauggc cggguuguug agcaccuucg auacauuuag cucuaggagg agcgaaagua     780

uuaauaaguc gggaggaggu gcuguuaucc ccggccagag gagcacaguc ucaguguucg     840

uacuaggccc aagugugacu gaugaugcag acaaguuauu cauugcaacu accuuccuag     900

cucacucauu ggacacagau aagcagcacu cucagagagg gggguuccuc gucucucugc     960

uugccauggc uuacaguagu ccagaauugu acuugacaac aaacggagua aacgccgaug    1020

ucaaauaugu gaucuacaac auagagaaag acccuaagag gacgaagaca gacggauuca    1080

uugugaagac gagagauaug gaauaugaga ggaccacaga auggcuguuu ggaccuaugg    1140

ucaacaagag cccacucuuc cagggucaac gggaugcugc agacccugac acacuccuuc    1200

aaaucuaugg guauccugca ugccuaggag caauaauugu ccaagucugg auugugcugg    1260

ugaaggccau cacaagcagc gccggcuuaa ggaaaggguu cuucaacagg uuagaggcgu 1320
ucagacaaga cggcaccgug aaaggugccu uaguuuucac uggggagaca guugagggga 1380
uaggcucggu uaugagaucu cagcaaagcc uuguaucucu caugguugag acccuuguga 1440
cuaugaauac ugcaagaucu gaucucacca cauuagagaa gaacauccag aucguuggga 1500
acuacauccg agaugcaggg cuggcuuccu ucaugaacac uauuaaauau gggguggaaa 1560
caaagauggc agcucuaacg uugucaaacc ugaggcccga uauuaauaag cuuagaagcc 1620
ucauagacac cuaccuguca aaggccccca gagcucccuu uaucuguauc cucaaggacc 1680
cuguucaugg ugaauuugcu ccaggcaauu auccugcacu auggaguuac gccaugggag 1740
ucgccgucgu acagaacaag gcaaugcagc aguacgucac agggaggaca uaccuugaua 1800
uggaaauguu cuuacuagga caagccgugg caaaggaugc ugaaucgaag aucagcagug 1860
ccuuggaaga ugaguuagga gugacggaua cagccaaggg gaggcucaga caucaucugg 1920
caaacuuguc cgguggggau ggugcuuacc acaaaccaac aggcgguggu gcaauugagg 1980
uagcucuaga caaugccgac aucgaccuag aaacaaaagc ccaugcggac caggacgcua 2040
gggguugggg uggagauagu ggugaaagau gggcacguca ggugaguggu ggccacuuug 2100
ucacacuaca uggggcugaa cgguuagagg aggaaaccaa ugaugaggau guaucagaca 2160
uagagagaag aauagccaug agacucgcag agagacggca agaggauucu gcaacccaug 2220
gagaugaagg ccgcaauaac ggugucgauc augacgaaga ugacgaugcc gcagcaguag 2280
cugggauagg aggaaucuag gaucauacga ggcuucaagg uacuugaucc guaguaagaa 2340
aaacuuaggg ugaaaguuca uccaccgauc ggcucaggca aggccacacc caaccccacc 2400
gaccacaccc agcagucgag acagccacgg cuucggcuac acuuaccgca uggaucaaga 2460
ugccuucauu cuuaaagaag auucugaagu ugagagggag gcgccaggag gacgagaguc 2520
gcucucggau guuaucggau uccucgaugc uguccugucg agugaaccaa cugacaucgg 2580
aggggacaga agcuggcucc acaacaccau caacacuccc caaggaccag gcucugcuca 2640
uagagccaaa agugagggcg aaggagaagu cucaacaccg ucgacccaag auaaucgauc 2700
aggugaggag aguagagucu cugggagaac aagcaagcca gaggcagaag cacaugcugg 2760
aaaccuugau aaacaaaaua uacaccgggc cuuuggggga agaacuggua caaacucugu 2820
aucucaggau cugggcgaug gaggagacuc cggaauccuu gaaaauccuc caaaugagag 2880

aggauauccg agaucaggua uugaagauga aaacagagag auggcugcgc acccugauaa 2940
gaggggagaa gaccaagcug aaggacuucc agaagaggua cgaggaagua caucccuacc 3000
ugaugaagga gaagguggag caaguaauaa uggaagaagc auggagccug gcagcucaca 3060
uagugcaaga guaacugggg uccuggugau uccuagcccc gaacuugaag aggcugugcu 3120
acggaggaac aaaagaagac cuaccaacag uggguccaaa ccucuuacuc cagcaaccgu 3180
gccuggcacc cgguccccac cgcugaaucg uuacaacagc acagggucac caccaggaaa 3240
acccccaucu acacaggaug agcacaucaa cucuggggac acccccgccg ucaggucaaa 3300
agaccggaaa ccaccaauag ggacccgcuc ugucucagau uguccagcca acggccgccc 3360
aauccacccg ggucuagaga ccgacucaac aaaaaagggc auaggagaga acacaucauc 3420
uaugaaagag auggcuacau uguugacgag ucuuggugua auccagucug cucaagaauu 3480
cgaaucaucc cgagacgcga guuauguguu ugcaagacgu gcccuaaagu cugcaaacua 3540
ugcagagaug acauucaaug uaugcggccu gauccuuucu gccgagaaau cuuccgcucg 3600
uaagguagau gagaacaaac aacugcucaa acagauccaa gagagcgugg aaucauuccg 3660
ggauauuuac aagagauucu cugaguauca gaaagaacag aacucauugc ugauguccaa 3720
ccuaucuaca cuucauauca ucacagauag agguggcaag acugacaaca cagacucccu 3780
uacaaggucc cccuccguuu uugcaaaauc aaaagagaac aagacuaagg cuaccagguu 3840
ugacccaucu auggagaccc uagaagauau gaaguacaaa ccggaccuaa uccgagagga 3900
ugaauuuaga gaugagaucc gcaacccggu guaccaagag agggacacag aacccagggc 3960
cucaaacgca ucacgucucu uucccuccaa agagaagccc acaaugcacu cucucaggcu 4020
cgucauagag agcagucccc uaagcagagc ugagaaagua gcauauguga aaucauuauc 4080
caagugcaag acagaccaag agguuaaggc agucauggaa cucguagaag aggacauaga 4140
gucacugacc aacuagaucc cgggugaggc auccuaccau ccucagucau agagagaucc 4200
aaucuaccau cagcaucagc caguaaagau uaagaaaaac uuagggugaa agaaauuuca 4260
ccuaacacgg cgcaauggca gauaucuaua gauucccuaa guucucauau gaggauaacg 4320
guacugugga gccccugccu cugagaacug guccggauaa gaaagccauc ccccacauca 4380
ggauugucaa gguaggagac ccuccuaaac auggagugag auaccuagau uuauugcucu 4440

uggguuucuu ugagacaccg aaacaaacaa ccaaucuaga gagcguaucu gacuugacag 4500
agccgaccag cuacucaaua ugcggcuccg ggucguuacc cauaggugug gccaaauacu 4560
acgggacuga ucaggaacuc uuaaaggccu gcaccgaucu cagaauuacg gugaggaggg 4620
cuguucgagc aggagagaug aucguauaca ugguggauuc gauuggugcu ccacuccuac 4680
cauggucagg caggcugaga cagggaauga uauuuaaugc aaacaagguc gcacuagcuc 4740
cccaaugccu cccuguggac aaggacauaa gacucagagu gguguuuguc aaugggacau 4800
cucuaggggc aaucaccaua uccaagaucc caaagacccu ugcagaccuu gcauugccca 4860
acucuauauc uguuaauuua cuggugacac ucaagaccgg gaucuccaca gaacaaaagg 4920
ggguacuccc aguacuugau gaucaagggg agaaaaagcu caauuuuaug gugcaccucg 4980
gguugaucag gagaaagguc gggaagauau acucuguuga guacugcaag agcaagauug 5040
agagaaugcg gcugauuuuc ucacuugggu uaaucggcgg uauaagcuuc cauguucagg 5100
uuaaugggac acuaucuaag acauucauga gucagcucgc auggaagagg gcagucugcu 5160
ucccauuaau ggaugugaau ccccauauga acauggugau uugggcggca ucuguagaaa 5220
ucacaggcgu cgaugcggug uuccaaccgg ccaucccucg ugauuuccgc uacuacccua 5280
auguuguggc uaagaacauc ggaaggauca gaaagcugua aaugugcacc caucagagac 5340
cugcgacaau gccccaagca gacaccaccu ggcagucgga gccaccgggu cacuccuugu 5400
cuuaaauaag aaaaacuuag ggauaaaguc ccuugugagu gcuugguuaa uuaagcuuuc 5460
accucaaaca agcacagauc auggauggug auaggggcaa acgugacucg uacuggucua 5520
cuucuccuag ugguagcacc acaaaaccag caucagguug ggagagguca aguaaagccg 5580
acacaugguu gcugauucuc ucauucaccc agugggcuuu gucaauugcc acagugauca 5640
ucuguaucau aauuucugcu agacaagggu auaguaugaa agaguacuca augacuguag 5700
aggcauugaa caugagcagc agggagguga aagagucacu uaccagucua auaaggcaag 5760
agguuauagc aagggcuguc aacauucaga gcucugugca aaccggaauc ccagucuugu 5820
ugaacaaaaa cagcagggau gucauccaga ugauugauaa gucgugcagc agacaagagc 5880
ucacucagca cugugagagu acgaucgcag uccaccaugc cgauggaauu gccccacuug 5940
agccacauag uuucuggaga ugcccugucg gagaaccgua ucuuagcuca gauccugaaa 6000
ucucauugcu gccugguccg agcuuguuau cugguucuac aacgaucucu ggauguguua 6060

ggcucccuuc acucucaauu ggcgaggcaa ucuaugccua uucaucaaau cucauuacac 6120
aagguugugc ugacauaggg aaaucauauc agguccugca gcuaggguac auaucacuca 6180
auucagauau guucccugau cuuaaccccg uaguguccca cacuuaugac aucaacgaca 6240
aucggaaauc augcucugug gugacaaccc ggacuagggg uuaucagcuu ugcuccaugc 6300
cgacuguaga cgaaagaacc gacuacucua gugaugguau ugaggaucug guccuugaug 6360
uccuggaucu caaagggaga acuaagucuc accgguaucg caacagcgag guagaucuug 6420
aucacccguu cucugcacua uaccccagug uaggcaacgg cauugcaaca gaaggcucau 6480
ugauauuucu uggguauggu ggacuaacca ccccucugca gggugauaca aaauguagga 6540
cccaaggaug ccaacaggug ucgcaagaca caugcaauga ggcucugaaa auuacauggc 6600
uaggagggaa acaggugguc agcgugauca uccaggucaa ugacuaucuc ucagagaggc 6660
caaagauaag agucacaacc auuccaauca cucaaaacua ucucggggcg gaagguagau 6720
uauuaaaauu gggugaucgg guguacaucu auacaagauc aucaggcugg cacucucaac 6780
ugcagauagg aguacuugau gucagccacc cuuugacuau caacuggaca ccucaugaag 6840
ccuugucuag accaggaaau gaagagugca auugguacaa uaaguguccg aaggaaugca 6900
uaucaggcgu auacacugau gcuuauccau uguccccuga ugcagcuaac gucgcuaccg 6960
ucacgcuaua ugccaauaca ucgcguguca acccaacaau cauguauucu aacacuacua 7020
acauuauaaa uauguuaagg auaaaggaug uucaauuaga ggcugcauau accacgacau 7080
cguguaucac gcauuuuggu aaaggcuacu gcuuucacau caucgagauc aaucagaaga 7140
gccugaauac cuuacagccg augcucuuua agacuagcau cccuaaauua ugcaaggccg 7200
agucuuaaau uuaacugacu agcaggcuug ucggccuugc ugacacuaga gucaucuccg 7260
aacauccaca auaucucuca gucucuuacg ucucucacag uauuaagaaa aacccagggu 7320
gaaugggaag cuugccauag gucauggaug ggcaggaguc cucccaaaac ccuucugaca 7380
uacucuaucc agaaugccac cugaacucuc ccauagucag gggaagauaa gcacaguugc 7440
acgucuuguu agaugugaac cagcccuaca gacugaagga cgacagcaua auaaauauua 7500
caaagcacaa aauuaggaac ggaggauugu ccccccguca aauuaagauc aggucucugg 7560
guaaggcucu ucaacgcaca auaaaggauu uagaccgaua cacguuugaa ccguacccaa 7620

ccuacucuca ggaauuacuu aggcuugaua uaccagagau augugacaaa auccgauccg 7680
ucuucgcggu cucggaucgg cugaccaggg aguuaucuag uggguuccag gaucuuuggu 7740
ugaauaucuu caagcaacua ggcaauauag aaggaagaga gggguacgau ccguugcagg 7800
auaucggcac caucccggag auaacugaua aguacagcag gaauagaugg uauaggccau 7860
uccuaacuug guucagcauc aaauaugaca ugcgguggau gcagaagacc agaccggggg 7920
gaccccucga uaccucuaau ucacauaacc uccuagaaug caaaucauac acucuaguaa 7980
cauacggaga ucuugucaug auacugaaca aguugacauu gacaggguau auccuaaccc 8040
cugagcuggu cuugauguau ugugauguug uagaaggaag guggaauaug ucugcugcag 8100
ggcaucuaga uaagaagucc auugggauaa caagcaaagg ugaggaauua ugggaacuag 8160
uggauucccu cuucucaagu cuuggagagg aaauauacaa ugucaucgca cuauuggagc 8220
cccuaucacu ugcucucaua caacuaaaug auccuguuau accucuacgu ggggcauuua 8280
ugaggcaugu guugacagag cuacagacug uuuuaacaag uagagacgug uacacagaug 8340
cugaagcaga cacuauugug gagucguuac ucgccauuuu ccauggaacc ucuauugaug 8400
agaaagcaga gaucuuuucc uucuuuagga cauuggccca ccccagcuua gaggcuguca 8460
cugccgccga caagguaagg gcccauaugu augcacaaaa ggcaauaaag cuuaagaccc 8520
uauacgagug ucaugcaguu uuuugcacua ucaucauaaa uggguauaga gagaggcaug 8580
gcggacagug gccccccugu gacuucccug aucacgugug ucuagaacua aggaacgcuc 8640
aaggguccaa uacggcaauc ucuuaugaau gugcuguaga caacuauaca aguuucauag 8700
gcuucaaguu ucggaaguuu auagaaccac aacuagauga agaucucaca auauauauga 8760
aagacaaagc acuauccccc aggaaggagg caugggacuc uguauacccg gauaguaauc 8820
uguacuauaa agccccagag ucugaagaga cccggcggcu uauugaagug uucauaaaug 8880
augagaauuu caacccagaa gaaauuauca auuaugugga gucaggagau ugguugaaag 8940
acgaggaguu caacaucucg uacagucuca aagagaaaga gaucaagcaa gagggucguc 9000
uauucgcaaa aaugacuuau aagaugcgag ccguacaggu gcuggcagag acacuacugg 9060
cuaaaggaau aggagagcua uucagcgaaa augggauggu uaaaggagag auagaccuac 9120
uuaaaagauu gacuacucuu ucugucucag gcguccccag gacugauuca guguacaaua 9180
acucuaaauc aucagagaag agaaacgaag gcauggaaaa uaagaacucu gggggguacu 9240

gggacgaaaa gaagaggucc agacaugaau ucaaggcaac agauucauca acagacggcu 9300
augaaacguu aaguugcuuc cucacaacag accucaagaa auacugcuua aacuggagau 9360
uugagaguac ugcauuguuu ggucagagau gcaacgagau auuuggcuuc aagaccuucu 9420
uuaacuggau gcauccaguc cuugaaaggu guacaauaua uguuggagau ccuuacuguc 9480
cagucgccga ccggaugcau cgacaacucc aggaucaugc agacucuggc auuuucauac 9540
auaauccuag ggggggcaua gaagguuacu gccagaagcu guggaccuua aucucaauca 9600
gugcaaucca ccuagcagcu gugagagugg gugucagggu cucugcaaug guucagggug 9660
acaaucaagc uauagccgug acaucaagag uaccuguagc ucagacuuac aagcagaaga 9720
aaaaucaugu cuaugaggag aucaccaaau auuucggugc ucuaagacac gucauguuug 9780
auguagggca cgagcuaaaa uugaacgaga ccaucauuag uagcaagaug uuugucuaua 9840
guaaaaggau auacuaugau gggaagauuu uaccacagug ccugaaagcc uugaccaagu 9900
guguauucug guccgagaca cugguagaug aaaacagauc ugcuuguucg aacaucucaa 9960
cauccauagc aaaagcuauc gaaaaugggu auucuccuau acuaggcuac ugcauugcgu 10020
uguauaagac cugucagcag gugugcauau cacuaggaau gacuauaaau ccaacuauca 10080
gcccgaccgu aagagaucaa uacuuuaagg guaagaauug gcugagaugu gcaguguuga 10140
uuccagcaaa uguuggagga uucaacuaca ugucuacauc uagaugcuuu guuagaaaua 10200
uuggagaccc cgcaguagca gcccuagcug aucucaaaag auucaucaga gcggaucugu 10260
uagacaagca gguauuauac agggucauga aucaagaacc cggugacucu aguuuucuag 10320
auugggcuuc agacccuuau ucguguaacc ucccgcauuc ucagaguaua acuacgauua 10380
uaaagaauau cacugcuaga ucugugcugc aggaaucccc gaauccucua cugucugguc 10440
ucuucaccga gacuagugga gaagaggauc ucaaccuggc cucguuccuu auggaccgga 10500
aagucauccu gccgagagug gcucaugaga uccuggguaa uuccuuaacu ggaguuaggg 10560
aggcgauugc agggaugcuu gauacgacca agucucuagu gagagccagc guuaggaaag 10620
gaggauuauc auaugggaua uugaggaggc uugucaauua ugaucuauug caguacgaga 10680
cacugacuag aacucucagg aaaccgguga aagacaacau cgaauaugag uauauguguu 10740
caguugagcu agcugucggu cuaaggcaga aaauguggau ccaccugacu uacgggagac 10800

ccauacaugg gcuagaaaca ccagacccuu uagagcucuu gaggggaaua uuuaucgaag 10860
guucagaggu gugcaagcuu ugcaggucug aaggagcaga ccccaucuau acaugguucu 10920
aucuuccuga cucuauagac cuggacacgc uuacaaacgg auguccggcu auaagaaucc 10980
ccuauuuugg aucagccacu gaugaaaggu cggaagccca acucgguaug guaagaaauc 11040
uaagcaaacc cgcaaaggcg gccauccgga uagcuauggu guauacgugg gccuacggga 11100
cugaugagau aucguggaug gaagccgcuc uuauagccca aacaagagcu aaucugagcu 11160
uagagaaucu aaagcugcug acuccuguuu caaccuccac uaaucuaucu cauagguuga 11220
aagauacggc aacccagaug aaguucucua gugcaacacu aguccgugca agucgguuca 11280
uaacaauauc aaaugauaac auggcacuca aagaagcagg ggagucgaag gauacuaauc 11340
ucguguauca gcagauuaug cuaacugggc uaagcuuguu cgaguucaau augagauaua 11400
agaaagguuc cuuagggaag ccacugauau ugcacuuaca ucuuaauaac gggugcugua 11460
uaauggaguc cccacaggag gcgaauaucc ccccaagguc cacauuagau uuagagauua 11520
cacaagagaa caauaaauug aucuaugauc cugauccacu caaggaugug gaccuugagc 11580
uauuuagcaa ggucagagau guuguacaca caguugacau gacuuauugg ucagaugaug 11640
aaguuaucag agcaaccagu aucuguacug caaugacgau agcugauaca augucucaau 11700
uagauagaga caacuuaaaa gagaugaucg cacuaguaaa ugacgaugau gucaacagcu 11760
ugauuacuga guuuauggug auugauguuc cuuuauuuug cucaacguuc ggggguauuc 11820
uagucaauca guuugcauac ucacucuacg gcuuaaacau cagaggaagg gaagaaauau 11880
ggggacaugu aguccggauu cuuaaagaua ccucccacgc aguuuuaaaa gucuuaucua 11940
augcucuauc ucaucccaaa aucuucaaac gauucuggaa ugcagguguc guggaaccug 12000
uguaugggcc uaaccucuca aaucaggaua agauacucuu ggcccucucu gucugugaau 12060
auucugugga ucuauucaug cacgauuggc aagggggugu accgcuugag aucuuuaucu 12120
gugacaauga cccagaugug gccgacauga ggagguccuc uuucuuggca agacaucuug 12180
cauaccuaug cagcuuggca gagauaucua gggaugggcc aagauuagaa ucaaugaacu 12240
cucuagagag gcucgaguca cuaaagaguu accuggaacu cacauuucuu gaugacccgg 12300
uacugaggua cagucaguug acuggccuag ucaucaaagu auucccaucu acuuugaccu 12360
auauccggaa gucaucuaua aaaguguuaa ggacaagagg uauaggaguc ccugaagucu 12420

uagaagauug ggaucccgag gcagauaaug cacuguuaga ugguaucgcg gcagaaauac 12480
aacagaauau uccuuuggga caucagacua gagccccuuu uuggggguug agaguaucca 12540
agucacaggu acugcgucuc cggggguaca aggagaucac aagaggugag auaggcagau 12600
cagguguugg ucugacguua ccauucgaug gaagauaucu aucucaccag cugaggcucu 12660
uuggcaucaa caguacuagc ugcuugaaag cacuugaacu uaccuaccua uugagccccu 12720
uaguugacga agauaaagau aggcuauauu uaggggaagg agcuggggcc augcuuuccu 12780
guuaugacgc uacucuuggc ccaugcauca acuauuauaa cucaggggua uacucuugug 12840
augucaaugg gcagagagag uuaaauauau auccugcuga gguggcacua gugggaaaga 12900
aauuaaacaa uguuacuagu cugggucaaa gaguuaaagu guuauucaac gggaauccug 12960
gcucgacaug gauugggaau gaugagugug aggcuuugau uuggaaugaa uuacagaaua 13020
gcucgauagg ccuaguccac ugugacaugg agggaggaga ucauaaggau gaucaaguug 13080
uacugcauga gcauuacagu guaauccgga ucgcguaucu ggugggggau cgagacguug 13140
ugcuuauaag caagauugcu cccaggcugg gcacggauug gaccaggcag cucagccuau 13200
aucugagaua cugggacgag guuaaccuaa uagugcuuaa aacaucuaac ccugcuucca 13260
cagagaugua ucuccuaucg aggcacccca aaucugacau uauagaggac agcaagacag 13320
uguuagcuag ucuccucccu uugucaaaag aagauagcau caagauagaa aaguggaucu 13380
uaauagagaa ggcaaaggcu cacgaauggg uuacucggga auugagagaa ggaagcucuu 13440
caucagggau gcuuagaccu uaccaucaag cacugcagac guuuggcuuu gaaccaaacu 13500
uguauaaauu gagcagagau uucuugucca ccaugaacau agcugauaca cacaacugca 13560
ugauagcuuu caacaggguu uugaaggaua caaucuucga augggcuaga auaacugagu 13620
cagauaaaag gcuuaaacua acugguaagu augaccugua uccugugaga gauucaggca 13680
aguugaagac aauuucuaga agacuugugc uaucuuggau aucuuuaucu auguccacaa 13740
gauugguaac ugggucauuc ccugaccaga aguuugaagc aagacuucaa uugggaauag 13800
uuucauuauc aucccgugaa aucaggaacc ugaggguuau cacaaaaacu uuauuagaca 13860
gguuugagga uauuauacau aguauaacgu auagauuccu caccaaagaa auaaagauuu 13920
ugaugaagau uuuaggggca gucaagaugu ucggggccag gcaaaaugaa uacacgaccg 13980

ugauugauga uggaucacua ggugauaucg agccauauga cagcucguaa uaauuagucc 14040

cuaucgugca gaacgaucga agcuccgcgg uaccuggaag ucuuggacuu guccauauga 14100

caauaguaag aaaaacuuac aagaagacaa gaaaauuuaa aaggauacau aucucuuaaa 14160

cucuugucug gu 14172